# EUROPEAN PATENT APPLICATION

(11) **EP 4 818 190 A1**
(43) Date of publication of application: **07.10.2026**
(21) Application number: 24896739.0
(22) Date of filing: 29.11.2024
(51) Int. Cl.: C07K 7/06, C07K 5/103, A61K 31/165, A61K 31/475, A61K 31/537, A61K 38/07, A61K 45/00, A61K 47/65, A61P 35/00, A61P 37/02

(54) **TRISACCHARIDE LINKER, LINKER-PAYLOAD COMPRISING TRISACCHARIDE LINKER, AND GLYCAN CHAIN-REMODELED ANTIBODY-DRUG CONJUGATE, AND PREPARATION METHODS THEREFOR AND USES THEREOF**

(30) Priority: 01.12.2023 CN 202311634308
(71) Applicant: Genequantum Healthcare (Suzhou) Co., Ltd., Suzhou, Jiangsu 215123 (CN)
(72) Inventor: XIONG, Meijun, Suzhou, Jiangsu 215123 (CN); QIN, Gang, Suzhou, Jiangsu 215123 (CN); AO, Jiaming, Suzhou, Jiangsu 215123 (CN)
(74) Representative: Zwicker, Jörk
(86) International application number: PCT/CN2024/135760
(87) International publication number: WO 2025/113659

(57) **Abstract**

The present disclosure relates to a linker-payload compound comprising a trisaccharide group, wherein the trisaccharide group is linked to the rest of the compound by an amide bond. The present disclosure also relates to an antibody-conjugated drug comprising the linker-payload compound, wherein a glycan chain in the antibody is remodeled with the trisaccharide group in the linker-payload compound. The present disclosure also relates to a method for preparing the compounds above and use thereof.

## Description

### TECHNICAL FIELD

The present disclosure relates to a trisaccharide linker. The present disclosure also relates to a linker-payload compound comprising a trisaccharide group, wherein the trisaccharide group is linked to the rest of the compound by an amide bond. The present disclosure also relates to an antibody-conjugated drug comprising the linker-payload compound, wherein a glycan chain in the antibody is remodeled with the trisaccharide group in the linker-payload compound. The present disclosure also relates to a method for preparing the compounds above and use thereof.

### BACKGROUND

The antibody-conjugated drug is a novel drug form formed by linking an antibody to an effector molecule payload with physiological activity. It fully utilizes the targeting capacity of the antibody and the efficient physiological activity of the effector molecule to achieve the targeted delivery of the effector molecule, reduces the off-target toxicity due to the drug distribution in non-target organs and tissues, and further improves the defect of limited efficacy of conventional antibody drugs on solid tumors. Currently, more than ten antibody-conjugated drugs of small-molecule toxins have been approved worldwide, bringing better survival benefits to cancer patients (Dumontet C, Reichert JM, Senter PD, etal. Antibody-drug conjugates come of age in oncology. Nat. Rev. Drug. Discov. 2023, 22, 641.). For example, traditional means for treating breast cancer such as surgery, chemotherapy, radiotherapy and endocrine therapy have problems of permanent damage, serious adverse effects of chemotherapy and radiotherapy, limited prognostic effect, and the like. The advent of targeted monoclonal antibody therapies represented by trastuzumab has brought about a revolution in the treatment of breast cancer and even other cancers. Such therapies significantly reduce the mortality of breast cancer patients and have benefited millions of breast cancer patients. However, trastuzumab also has its own limitations. It is only effective in HER2-positive patients that account for about 20% among all breast cancer patients, whereas breast cancer patients with low HER2 expression, who account for a higher proportion, can hardly benefit from the trastuzumab treatment. Antibody-conjugated drug Enhertu based on trastuzumab and the topoisomerase I inhibitor DXd has brought another revolution in targeted therapy: for patients with HER2-positive breast cancer who have received at least two or more targeted HER2 therapies and exhibited drug resistance, Enhertu achieved an overall response rate of 60.3%; for patients with metastatic breast cancer with low HER2 expression with no targeted therapies available, Enhertu can reduce the risk of death or tumor progression by 50% as compared to standard chemotherapies. In summary, antibody-conjugated drugs give full play to antibodies and effector molecules through the organic linkage of the two, improve the targeting effect, safety, and efficacy of treatment, and bring about changes in cancer treatment. Most antibody-conjugated drugs currently approved or in clinical development are based on traditional conjugation modes such as lysine or cysteine on the antibodies, which often results in random conjugation sites and high product heterogeneity. In addition, due to the limitation of conjugation mode, the antibody backbone may be affected after an unstable linker or interchain disulfide bond is subjected to a reduction procedure. Therefore, traditional conjugation modes face severe challenges in process stability, quality control, antibody-conjugated drug stability, metabolic consistency, safety, and the like. In order to overcome the above problems, in recent years, a new generation of site-specific conjugation techniques, such as site-specific conjugation via antibody engineering to introduce specific amino acids, site-specific conjugation via transpeptidase catalysis, affinity-mediated site-specific conjugation, and enzymatic site-specific conjugation on antibody glycan chain have been developed in the industry, which solve the limitations of heterogeneity in traditional conjugations to a certain extent. However, these techniques still have certain limitations. For example, some of the conjugation techniques require additional antibody modification or engineering, the screening and synthesis of affinity ligands are complicated, the conjugation conditions are neither mild nor environmentally friendly, the process flow is complex, and the applicable range for substrates is limited (Walsh SJ, Bargh JD, Dannheim FM, et al. Site-selective modification strategies in antibody-drug conjugates. Chem. Soc. Rev. 2021, 50, 1305).

The enzymatic site-specific conjugation technique on antibody glycan chain uses glycosyltransferases or endoglycosidases to modify antibody glycan chains and introduce effector molecules without additional antibody engineering. However, previous studies often fail to completely solve problems such as complex conjugation processes (involving multiple enzymes and multiple reaction procedures), great substrate limitations, etc. (Manabe S, Yamaguchi Y, Antibody Glycoengineering and Homogeneous Antibody-Drug Conjugate Preparation. Chem. Rec. 2021, 21, 1). In 2022, Shi W. et al reported a one-step site-specific conjugation method for antibody glycan chains, catalyzed by endoglycosidase Endo S2 and based on the structure of 6'-formyl-*N*-acetyllactosamine disaccharide derivatives. The method requires no antibody engineering, and uses the same enzyme to catalyze both the deglycosylation reaction of the glycosyl at position N-297 of the antibody Fc region and the transglycosylation reaction of disaccharide linker-effector molecule to complete the site-specific modification of the antibody in one pot, so as to yield an antibody-conjugated drug (Shi W., Li W.,Zhang J., et. al. One-step synthesis of site-specific antibody-drug conjugates by reprograming IgG glycoengineering with LacNAc-based substrates, Acta Pharmaceutica Sinica B 2022,12,2417.). Similarly, Zhang X. et al. reported a one-step site-specific conjugation method for antibody glycan chains, catalyzed by endoglycosidase Endo S2 and based on the structure of disaccharide derivatives functionalized with alkylamine at position 6' in the same year (Zhang X., Ou C., Liu H., et. al. Synthesis and Evaluation of Three Azide-Modified disaccharide Oxazolines as Enzyme Substrates for Single-Step Fc Glycan Mediated Antibody-Drug Conjugation, Bioconjugate Chem. 2022, 33, 1179.).

### SUMMARY

The present disclosure provides a novel trisaccharide linker, a preparation method, and use thereof. The present disclosure also provides a linker-payload compound comprising a trisaccharide group, wherein the trisaccharide group is linked to the rest of the compound by an amide bond. In particular, the present disclosure provides a linker-payload compound of formula (I):

D-L-(P)ₜ formula (I)

wherein
P is the payload;
L is a linker, and L is directly linked to D via the terminal -NH-, wherein when L is an unbranched linker, it is linked to one P, and t is 1, and when L is a branched linker, each branch can be linked to one P, and t is an integer greater than 1 (e.g., 2, 3, 4, 5, 6, 7, 8, 9, or 10);
D is a trisaccharide unit structure comprising a first hexose unit or a derivative moiety thereof, a second hexose unit or a derivative moiety thereof, and a third hexose unit or a derivative moiety thereof;
the -OH at position 6 of the first hexose unit or the derivative moiety thereof is oxidized to - C(O)- to link to the terminal -NH- of L;
the first hexose unit or the derivative moiety thereof is linked to the second hexose unit or the derivative moiety thereof via an α-(1→6) glycosidic bond;
the second hexose unit or the derivative moiety thereof is linked to the third hexose unit or the derivative moiety thereof via a β-(1→4) glycosidic bond;
the third hexose unit or the derivative moiety thereof has the following structure: 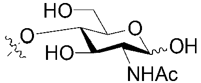 (β-D-*N-*acetylglucosamine moiety) or 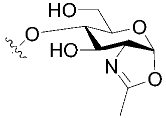 (β-D-glucose oxazoline moiety).

In one embodiment, L can be chemically (e.g., hydrolytically) or biologically (e.g., enzymatically) detached from P to release P.

The present disclosure also relates to an antibody-conjugated drug comprising the linker-payload compound, wherein a glycan chain in the antibody is remodeled with the trisaccharide group in the linker-payload compound. Specifically, the present disclosure provides an antibody-conjugated drug based on site-specific conjugation at an N-glycosylation site in an antibody Fc region having the structure of formula (II): 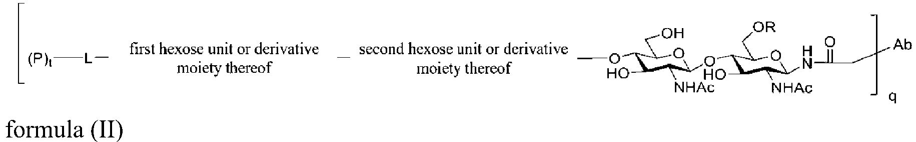 wherein
P is the payload;
R is hydrogen or α-L-fucosyl;
q is 1 or 2;
Ab is an antibody or an antigen-binding fragment;
the first hexose unit or the derivative moiety thereof is linked to the second hexose unit or the derivative moiety thereof via an α-(1→6) glycosidic bond;
the second hexose unit or the derivative moiety thereof is linked to a β-D-*N*-acetylglucosamine moiety via a β-(1→4) glycosidic bond;
the -OH at position 6 of the first hexose unit or the derivative moiety thereof is oxidized to - C(O)-;
L is a linker, and L is directly linked to -C(O)- in the first hexose unit or the derivative moiety thereof via the terminal -NH-, wherein when L is an unbranched linker, it is linked to one P, and t is 1, and when L is a branched linker, each branch can be linked to one P, and t is an integer greater than 1 (e.g., 2, 3, 4, 5, 6, 7, 8, 9, or 10).
In one embodiment, the first hexose unit or the derivative moiety thereof is selected from glucosyl, mannosyl, galactosyl, fructosyl, gulosyl, idosyl, and derivatives thereof.
In one embodiment, the second hexose unit or the derivative moiety thereof is selected from glucosyl, mannosyl, galactosyl, fructosyl, and derivatives thereof.
In one embodiment, the -NHC(O)CH₂- linked to Ab in formula (II) is derived from asparagine at position 297 of the antibody Fc region.

The present disclosure also relates to a method for preparing the compounds above and use thereof.

### BRIEF DESCRIPTION OF THE DRAWINGS

FIGs. 1 and 2 show the results of hydrophobic interaction chromatography-high performance liquid chromatography (HIC-HPLC) and size exclusion chromatography-high performance liquid chromatography (SEC-HPLC) for antibody-conjugated drug ADC-1, respectively.
FIGs. 3 and 4 show the results of hydrophobic interaction chromatography-high performance liquid chromatography (HIC-HPLC) and size exclusion chromatography-high performance liquid chromatography (SEC-HPLC) for antibody-conjugated drug ADC-2, respectively.
FIGs. 5 and 6 show the results of hydrophobic interaction chromatography-high performance liquid chromatography (HIC-HPLC) and size exclusion chromatography-high performance liquid chromatography (SEC-HPLC) for antibody-conjugated drug ADC-3, respectively.
FIGs. 7 and 8 show the results of hydrophobic interaction chromatography-high performance liquid chromatography (HIC-HPLC) and size exclusion chromatography-high performance liquid chromatography (SEC-HPLC) for antibody-conjugated drug ADC-4, respectively.
FIGs. 9-11 show the *in vitro* activity of antibody-conjugated drugs ADC-1 to ADC-4 on cell lines SKBR-3, NCI-N87, and MDA-MB-468, respectively.
FIGs. 12-14 show the *in vitro* activity of antibody-conjugated drugs ADC-5 to ADC-8 on cell lines SKBR-3, NCI-N87, and MDA-MB-468, respectively.
FIGs. 15-17 show the *in vitro* activity of antibody-conjugated drugs ADC-9 to ADC-11 on cell lines SKBR-3, NCI-N87, and MDA-MB-468, respectively.
FIGs. 18-20 show the *in vitro* activity of antibody-conjugated drugs ADC-12 to ADC-14 on cell lines SKBR-3, NCI-N87, and MDA-MB-468, respectively.
FIG. 21 shows an affinity assay of ADC-5 to ADC-8 for HER2 on the cell surface.
FIG. 22 shows an *in vivo* efficacy evaluation of ADC-6 to ADC-8.
FIG. 23 shows the ADCC effect of ADC-5 to ADC-8.

### DETAILED DESCRIPTION

### General Definitions

Unless otherwise defined hereinafter, all technical and scientific terms used herein have the same meaning as commonly understood by those skilled in the art. The techniques used herein are those commonly understood in the art, including variants and equivalent substitutions that are apparent to those skilled in the art. While the following terms are believed to be readily understood by those skilled in the art, the following definitions are set forth to better illustrate the present disclosure. When referring to a trade name herein, it is intended to refer to its corresponding commercial product or its active ingredient. All patents, published patent applications, and publications cited herein are incorporated herein by reference.

When a certain amount, concentration, or another value or parameter is set forth in the form of a range, a preferred range, or a preferred upper limit or a preferred lower limit, it will be appreciated that it is equivalent to specifically indicating any range formed by combining any upper limit or preferred value with any lower limit or preferred value, regardless of whether the range is explicitly recited. Unless otherwise indicated, the numerical ranges set forth herein are intended to include the endpoints of the ranges, as well as all integers and fractions (decimals) within the ranges. For example, the expression "i is an integer of 1-20" means that i is any integer from 1 to 20. For example, i may be 1, 2, 3, 4, 5, 6, 7, 8, 9, 10, 11, 12, 13, 14, 15, 16, 17, 18, 19, or 20. Other similar expressions such as j, g, and k should also be interpreted in a similar manner.

Unless the context clearly dictates otherwise, the singular forms "a", "an", and "the" encompass the plural forms. The expression "one or more" or "at least one" may denote 1, 2, 3, 4, 5, 6, 7, 8, 9, or more.

The terms "about" and "approximately", when used together with a numerical variable, generally mean that the value of the variable and all values of the variable are within experimental error (e.g., within a 95% confidence interval of the mean) or within ±10% or more of a specified value.

The term "optional" or "optionally" means that the subsequently described event may, but not necessarily, occur, and that the description includes instances where the event or circumstance occurs or does not occur.

The expressions "comprise", "include", "contain" and "have" are open-ended and do not exclude additional unrecited elements, steps, or ingredients. The expression "consisting of" excludes any element, step, or ingredient not designated. The expression "consisting essentially of" means that the scope is limited to the designated elements, steps, or ingredients, as well as elements, steps, or ingredients that are optionally present and do not substantially affect the essential and novel characteristics of the claimed subject matter. It will be appreciated that the expression "comprise" encompasses the expressions "consisting essentially of" and "consisting of".

The term "targeting molecule" refers to a molecule that has affinity for a particular target (e.g., a receptor, a cell surface protein, a cytokine, a tumor-specific antigen, or the like). The targeting molecule can deliver the payload to a specific site *in vivo* by targeted delivery. The targeting molecule can recognize one or more targets. The specific target sites are defined by the target it recognizes. For example, a targeting molecule that targets a receptor can deliver the cytotoxin to sites containing a large number of receptors. Examples of targeting molecules include, but are not limited to, antibodies, binding proteins for a given antigen, antibody mimetics, scaffold proteins having affinity for a given target, ligands, and the like. Targets recognized by targeting molecules include, but are not limited to, CD19, CD22, CD25, CD30/TNFRSF8, CD33, CD37, CD44v6, CD56, CD70, CD71, CD74, CD79b, CD117/KIT, CD123, CD138, CD142, CD174, CD227/MUC1, CD352, CLDN18.2, DLL3, ErbB2/HER2, ErbB3/HER3, CN33, GPNMB, ENPP3, Nectin-4, EGFRvIII, SLC44A4/AGS-5, CEACAM5, PSMA, TIM1, LY6E, LIV1, Nectin4, SLITRK6, HGFR/cMet, SLAMF7/CS1, EGFR, BCMA, AXL, NaPi2B, GCC, STEAP1, MUC16, mesothelin, ETBR, EphA2, 5T4, FOLR1, LAMP1, Cadherin 6, FGFR2, FGFR3, CA6, CanAg, Integrin αV, TDGF1, Ephrin A4, TROP2, PTK7, NOTCH3, C4.4A, FLT3, B7H3/4, TF (tissue factor), and ROR1/2, and combinations of the above targets. The antibody of the present disclosure includes, but is not limited to, antibodies and antibody fragments based on the targets described above, and bispecific antibodies and multispecific antibodies based on combinations of the targets described above.

As used herein, the term "antibody" is used in a broad manner encompassing conventional antibodies, recombinant antibodies/genetically engineered antibodies, and specifically includes intact monoclonal antibodies, polyclonal antibodies, monospecific antibodies, multispecific antibodies (e.g., bispecific antibodies), and antibody fragments so long as they possess the desired biological activity. An antibody may be of any subtype (e.g., IgG, IgE, IgM, IgD, and IgA) or subclass and may be derived from any suitable species. In some embodiments, the antibody is of human or murine origin. The antibody may also be a fully human antibody, a humanized antibody, or a chimeric antibody prepared by recombinant methods.

The monoclonal antibody used herein refers to an antibody obtained from a population of substantially homogeneous antibodies, i.e., the individual antibodies composing the population are identical except for a few possible natural mutations. Monoclonal antibodies are highly specific for a single antigenic site. The "monoclonal" means that the characteristics of the antibodies are derived from a population of substantially homogeneous antibodies, and it should not be construed as requiring some particular methods to produce the antibodies.

An intact or full-length antibody essentially comprises an antigen-binding variable region, a light chain constant region (C_{L}), and a heavy chain constant region (C_{H}), which may include C_{H}1, C_{H}2, C_{H}3, and C_{H}4, depending on the subtype of the antibody. The antigen-binding variable region (also referred to as fragment variable region or Fv fragment) typically comprises a light chain variable region (V_{L}) and a heavy chain variable region (V_{H}). The constant region may be a constant region having a natural sequence (e.g., a constant region having a human natural sequence) or an amino acid sequence variant thereof. The variable region recognizes and interacts with the target antigen. The constant region can be recognized by and interact with the immune system.

An antibody fragment may comprise a portion of an intact antibody, preferably the antigen-binding region or variable region thereof. Examples of antibody fragments include Fab, Fab', F(ab')₂, an Fd fragment consisting of the V_{H} and C_{H}1 domains, an Fv fragment, a single-domain antibody (dAb) fragment, and an isolated complementarity determining region (CDR). The Fab fragment is an antibody fragment obtained by digesting a full-length immunoglobulin with papain or a fragment of the same structure produced, for example, by recombinant expression. The Fab fragment comprises a light chain (comprising a V_{L} and a C_{L}) and another chain comprising the variable region of the heavy chain (V_{H}) and one constant region of the heavy chain (C_{H}1). The F(ab')₂ fragment is an antibody fragment obtained by digesting an immunoglobulin with pepsin at pH 4.0-4.5 or a fragment of the same structure produced, for example, by recombinant expression. The F(ab')₂ fragment substantially comprises two Fab fragments, wherein each heavy chain moiety comprises a few additional amino acids, including cysteines that form a disulfide bond linking the two fragments. The Fab' fragment is a fragment that comprises half of an F(ab')₂ fragment (one heavy chain and one light chain). The antibody fragment may comprise multiple chains linked together, for example, by a disulfide bond and/or by a peptide linker. Examples of antibody fragments also include single-chain Fv (scFv), Fv, dsFv, diabody, Fd, and Fd' fragments, as well as other fragments, including modified fragments. An antibody fragment typically comprises at least or about 50 amino acids, typically at least or about 200 amino acids. The antigen-binding fragment may include any antibody fragment that, when inserted into an antibody framework (e.g., by replacing the corresponding region), can result in an antibody that immunospecifically binds to an antigen.

In particular, the antibody-conjugated drug of the present application is subjected to site-specific conjugation on the basis of any natural N-glycosylation modification site containing the antibody Fc region, and molecules containing the antibody Fc region comprising a glycan chain (including but not limited to antibodies/diabodies/Fc fusion proteins/single-chain antibodies/nanobodies, etc.) can be prepared in one step using the linker-payload containing a trisaccharide of the present application. Therefore, the antibody of the present application is not particularly limited, as long as its Fc region contains a glycan chain. The antibody may be a natural antibody. The drug is not limited to a small molecule therapeutic drug, as long as the molecular entity possesses therapeutic, diagnostic, or prophylactic effects. The drug includes, but is not limited to, oligonucleotides (e.g., ASOs, siRNAs, etc.), polypeptides, cytokines, etc. Additionally, the antibody of the present disclosure may also be prepared using techniques well known in the art, such as the following techniques or a combination thereof: recombinant techniques/genetic engineering techniques, phage display techniques, synthetic techniques, or other techniques known in the art. For example, a genetically engineered recombinant antibody can be expressed by a suitable culture system (e.g., *E. coli* or mammalian cells). The genetic engineering may refer to, for example, the introduction of a ligase-specific recognition sequence at its termini.

As used herein, the term "targeting molecule-drug conjugate" is referred to as a "conjugate". Examples of conjugates include, but are not limited to, antibody-drug conjugates.

A small molecule compound refers to a molecule that is comparable in size to organic molecules commonly used in pharmaceuticals. The term does not encompass biological macromolecules (e.g., proteins, nucleic acids, etc.), but encompasses low molecular weight peptides or derivatives thereof, such as dipeptides, tripeptides, tetrapeptides, pentapeptides, etc. Typically, the small molecule compound may have a molecular weight of, for example, about 100 to about 2000 Da, about 200 to about 1000 Da, about 200 to about 900 Da, about 200 to about 800 Da, about 200 to about 700 Da, about 200 to about 600 Da, or about 200 to about 500 Da.

The cytotoxin refers to a substance that inhibits or prevents the expression activity of a cell and the function of a cell and/or causes the destruction of a cell. Cytotoxins that are commonly used in antibody-conjugated drugs at present are more toxic than chemotherapeutic drugs. Examples of cytotoxins include, but are not limited to, drugs that target the following targets: microtubule cytoskeleton, DNA, RNA, kinesin-mediated protein transport, and the regulation of apoptosis. The drug targeting the microtubule cytoskeleton may be, e.g., a microtubule stabilizer or a tubulin polymerization inhibitor. Examples of microtubule stabilizers include, but are not limited to, taxanes. Examples of tubulin polymerization inhibitors include, but are not limited to, maytansinoids, auristatins, vinca alkaloids, colchicines, and dolastatins. The drug targeting DNA may be, e.g., a drug that directly destroys the DNA structure or a topoisomerase inhibitor. Examples of drugs that directly destroy the structure of DNA include, but are not limited to, a DNA double strand breaker, a DNA alkylator, and a DNA intercalator. The DNA double strand breaker may be, e.g., an enediyne antibiotic, including but not limited to dynemicin, esperamicin, neocarzinostatin, uncialamycin, and the like. The DNA alkylator may be, e.g., a DNA bis-alkylator (i.e., DNA crosslinker) or a DNA mono-alkylator. Examples of DNA alkylators include, but are not limited to, pyrrolo[2,1-c][1,4]benzodiazepine (PBD) dimer, 1-(chloromethyl)-2,3-dihydro-1H-benzo[e]indole (CBI) dimer, CBI-PBD heterodimer, indolino-benzodiazepine (IGN) dimer, a duocarmycin-like compound, and the like. Examples of topoisomerase inhibitors include, but are not limited to, exatecan and derivatives thereof (e.g., DX8951f, DXd-(1), and DXd-(2), the structures of which are described below), camptothecins, and anthracyclines. The drug targeting RNA may be, e.g., a drug that inhibits splicing, and examples thereof include, but are not limited to, pladienolide. The drug targeting kinesin-mediated protein transport may be, e.g., mitotic kinesin inhibitors, including but not limited to kinesin spindle protein (KSP) inhibitors.

Small molecule compounds may also include, for example, small molecule drugs with various mechanisms of action, including various conventional small molecule drugs, photoacoustic kinetic therapeutic drugs, photothermal therapeutic drugs, etc., such as chemotherapeutic drugs, small molecule targeted drugs, immune agonists, etc., for example, conventional cytotoxic drugs such as cisplatin, paclitaxel, 5-fluorouracil, cyclophosphamide, bendamustine, etc.; small molecule targeted drugs such as imatinib mesylate, gefitinib, anlotinib, etc.; immune agonists such as STING agonists, TLR agonists, etc. The payload of the present disclosure may also be nucleic acids and nucleic acid analogs, and tracer molecules including fluorescent molecules, biotin, fluorophores, chromophores, spin resonance probes, radiolabels and the like, or may be short peptides, polypeptides, peptidomimetics, or proteins.

The "spacer" refers to a structure that is located between different structural modules and can spatially separate the structural modules. The definition of the spacer does not limit whether it has a certain function or whether it can be cut off or degraded *in vivo.* Examples of spacers include, but are not limited to, amino acids and non-amino acid structures, wherein the non-amino acid structures may be, but are not limited to, amino acid derivatives or analogs. The "spacer sequence" refers to an amino acid sequence as a spacer, and examples thereof include, but are not limited to, a single amino acid and sequences containing a plurality of amino acids, e.g., a sequence containing two amino acids, such as GA, or, e.g., GGGGS (SEQ ID No. 3), GGGGSGGGGS (SEQ ID No. 4), GGGGSGGGGSGGGGS (SEQ ID No. 5). The self-immolative spacer (e.g., self-immolative spacer Sp1) is a covalent assembly that enables the sequential cleavage of two chemical bonds after the activation of the protective moiety in the precursor: the protective moiety (e.g., cleavable sequence) is removed upon activation, triggering a cascade of decomposition reactions and thereby resulting in the sequential release of relatively smaller molecules. Examples of self-immolative spacers include, but are not limited to, PABC (p-aminobenzyloxycarbonyl), acetal, heteroacetal, and combinations thereof. As used herein, the term "amino acid" includes "natural amino acids" and "non-natural amino acids".

The term "natural amino acid" refers to amino acids that are protein constituent amino acids, including the twenty common amino acids (alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, and valine), and the less common amino acids selenocysteine and pyrrolysine.

As used herein, the term "non-natural amino acid" refers to an amino acid that is not a protein constituent amino acid. Specifically, the term refers to an amino acid that is not a natural amino acid as defined above.

The term "alkyl" refers to a linear or branched saturated aliphatic hydrocarbon group consisting of carbon atoms and hydrogen atoms, which is linked to the rest of the molecule by a single bond. The alkyl may have 1-20 carbon atoms and is referred to as "C₁-C₂₀ alkyl", such as C₁-C₄ alkyl, C₁-C₃ alkyl, C₁-C₂ alkyl, C₃ alkyl, C₄ alkyl, or C₃-C₆ alkyl. Non-limiting examples of the alkyl include, but are not limited to, methyl, ethyl, propyl, butyl, pentyl, hexyl, isopropyl, isobutyl, sec-butyl, tert-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, or 1,2-dimethylbutyl, or isomers thereof. A divalent radical refers to a group obtained by removing one hydrogen atom from a carbon atom having a free valence electron of a corresponding monovalent radical. Divalent radicals have two linking sites for linking to the rest of the molecule. For example, the "alkylene" or "alkylidene" refers to a saturated linear or branched divalent hydrocarbon group. Examples of the "alkylene" include, but are not limited to, methylene (-CH₂-), ethylene (-C₂H₄-), propylene (-C₃H₆-), butylene (-C₄H₈-), pentylene (-C₃H₁₀-), hexylene (-C₆H₁₂-), 1-methylethylene (-CH(CH₃)CH₂-), 2-methylethylene (-CH₂CH(CH₃)-), methylpropylene, ethylpropylene, or the like.

The term "cycloalkyl" refers to a cyclic saturated aliphatic group consisting of carbon atoms and hydrogen atoms, which is linked to the rest of the molecule by a single bond. The cycloalkyl may have 3-10 carbon atoms, i.e., "C₃-C₁₀ cycloalkyl", e.g., cyclopropyl, cyclobutyl, cyclopentyl, cyclohexyl, cycloheptyl, cyclooctyl, cyclononyl, or cyclodecyl. The "cycloalkylene" refers to a divalent cycloalkyl group.

The term "heterocyclyl" refers to that one or more carbon atoms in the cycloalkyl described above are substituted by a heteroatom selected from nitrogen, oxygen, and sulfur, e.g., aza-, oxa-, or thiacyclopropyl, aza-, oxa-, or thiacyclobutyl, pyrrolidinyl, pyrazolidinyl, imidazolidinyl, tetrahydrofuranyl, tetrahydrothienyl, piperidinyl, piperazinyl, tetrahydropyranyl, or tetrahydrothiopyranyl. The "heterocyclylene" refers to a divalent cycloalkyl group.

When "substitute" is mentioned herein, unless otherwise stated, the relevant substituent is selected from alkyl, halogen, amino, monoalkylamino, dialkylamino, nitro, cyano, formyl, alkylcarbonyl, carboxyl, alkyloxycarbonyl, alkylcarbonyloxy, aminocarbonyl, monoalkylaminocarbonyl, dialkylaminocarbonyl, formylamino, alkylcarbonylamino, formyl(monoalkyl)amino, and alkylcarbonyl(monoalkyl)amino.

As used herein, when a group is combined with another group, the linkage between the groups may be linear or branched, provided that a chemically stable structure is formed. The structure formed by such a combination may be linked to the rest of the molecule through any suitable atom in the structure, preferably through a designated chemical bond. For example, when two or more divalent groups selected from -CR¹R²-, C₁₋₁₀ alkylene, C₄₋₁₀ cycloalkylene, C₄₋₁₀ heterocyclylene, and -(CO)- are combined together to form a combination, the two or more divalent groups may form a linear linkage with each other, such as -CR¹R²-C₁₋₁₀ alkylene-(CO)-, -CR¹R²-C₄₋₁₀ cycloalkylene-(CO)-, -CR¹R²-C₄₋₁₀ cycloalkylene-C₁₋₁₀ alkylene-(CO)-, -CR¹R²-CR¹R^{2'}-(CO)-, or -CR¹R²-CR^{'}R^{2'}-CR^{1"}R^{2"}-(CO)-. The resulting divalent structure may be further linked to other portions of the molecule.

When a plurality of identical letters denoting chemical groups are present in the same chemical structural formula, they are independently selected and are not necessarily identical. For example, the plurality of M in formula I-2 are each independently selected from LKa-L²-L¹-B-P; the plurality of L² and the like are also independent of each other and are not necessarily identical. As another example, two a or i in the same structural formula are present, they are also independently selected, and are not necessarily identical.

As used herein, the expressions "antibody-conjugated drug" and "antibody-drug conjugate" have the same meaning.

### Linker-Payload Compound

In a first aspect, the present disclosure provides a linker-payload compound of formula (I):

D-L-(P)ₜ formula (I)

wherein
P is the payload;
L is a linker, and L is directly linked to D via the terminal -NH-, wherein when L is an unbranched linker, it is linked to one P, and t is 1, and when L is a branched linker, each branch can be linked to one P, and t is an integer greater than 1 (e.g., 2, 3, 4, 5, 6, 7, 8, 9, or 10);
D is a trisaccharide unit structure comprising a first hexose unit or a derivative moiety thereof, a second hexose unit or a derivative moiety thereof, and a third hexose unit or a derivative moiety thereof;
the -OH at position 6 of the first hexose unit or the derivative moiety thereof is oxidized to - C(O)- to link to the terminal -NH- of L;
the first hexose unit or the derivative moiety thereof is linked to the second hexose unit or the derivative moiety thereof via an α-(1→6) glycosidic bond;
the second hexose unit or the derivative moiety thereof is linked to the third hexose unit or the derivative moiety thereof via a β-(1→4) glycosidic bond;
the third hexose unit or the derivative moiety thereof has the following structure:

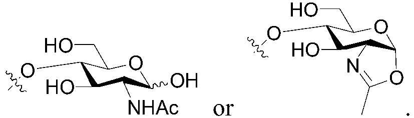

In one embodiment, the structure of formula (I) has the following structural formula (III):
linker L, with the -NH- at the terminus directly linked to the carbonyl group 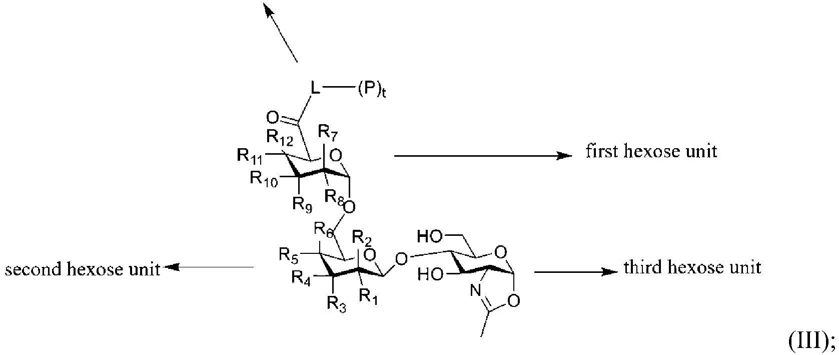
wherein, R₁-R₁₂ in formula (III) are selected from -OH and H;
preferably, two substituents on the same carbon atom are different;
more preferably, two substituents on the same carbon atom are different, R₃ is H, and R₄ is - OH;
even more preferably, two substituents on the same carbon atom are different, R₃ is H, R₄ is - OH, R₉ is H, and R₁₀ is -OH;
still more preferably, two substituents on the same carbon atom are different, R₃ is H, R₄ is - OH, R₉ is H, R₁₀ is -OH, R₈ is H, R₇ is -OH, R₁₂ is H, and R₁₁ is -OH;
L and P are as defined for formula (I).

In one embodiment, the structure of formula (III) is shown in formula (III-1):
linker L, with the -NH- at the terminus directly linked to the carbonyl group 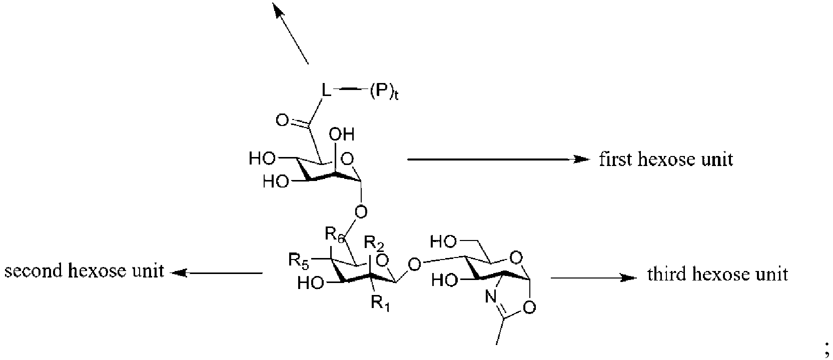 wherein, R₁, R₂, R₅, and R₆ are selected from -OH and H, and the two substituents on the same carbon atom are different.

In one embodiment, the first hexose unit or the derivative moiety thereof is selected from glucosyl, mannosyl, galactosyl, fructosyl, gulosyl, idosyl, and derivatives thereof, wherein the hydroxyl at position 6 of the first hexose unit or the derivative moiety thereof is oxidized to - C(O)- to link to the terminal -NH- of L.

In one embodiment, the first hexose unit or the derivative moiety thereof is selected from 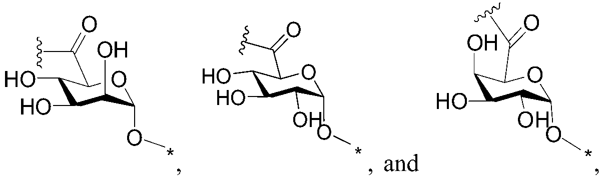 wherein, 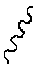 denotes the site for linking to L; * denotes the site for linking to the second hexose unit or the derivative moiety thereof.

In one embodiment, the second hexose unit or the derivative moiety thereof is selected from glucosyl, mannosyl, galactosyl, fructosyl, and derivatives thereof, wherein the first hexose unit or the derivative moiety thereof is linked to the second hexose unit or the derivative moiety thereof via an α-(1→6) glycosidic bond, and the second hexose unit or the derivative moiety thereof is linked to the third hexose unit or the derivative moiety thereof via a β-(1→4) glycosidic bond.

In one embodiment, the second hexose unit or the derivative moiety thereof is selected from 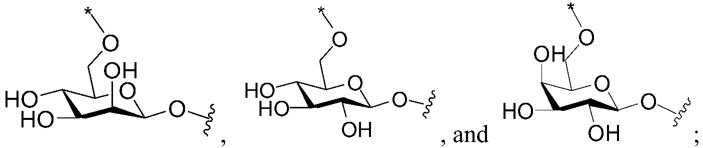 wherein 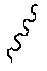 denotes the site for linking to the third hexose unit or the derivative moiety thereof; * denotes the site for linking to the first hexose unit or the derivative moiety thereof.

In one embodiment, the hexose derivative moiety is independently selected from a uronic acid and a monosaccharide with hydroxyl being substituted by an acylamino (e.g., alkanoylamino, e.g., formylamino, acetylamino, propionylamino, and especially acetylamino).

In one embodiment, the trisaccharide unit structure has the following structure:

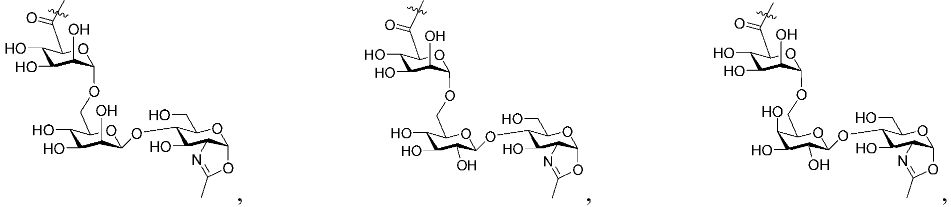

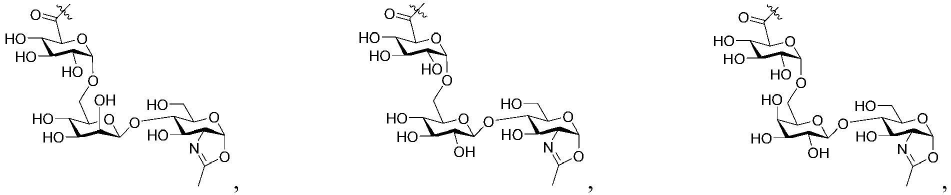

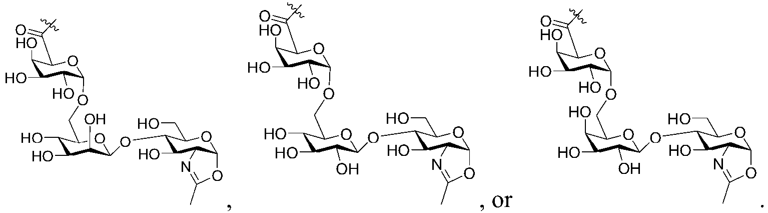

In one embodiment, -L-(P)t is -L²-L¹-B-P; that is, formula (I) is:

D-L²-L¹-B-P formula (I-1)

wherein
each B is independently absent, or is 1), 2), or the combination of 1) and 2) below: 1) a self-immolative spacer Sp1; and 2) one divalent group, or a combination of two or more divalent groups, wherein the divalent group is selected from: -CR¹R²-, C₁₋₁₀ alkylene, C₄₋₁₀ cycloalkylene, C₄₋₁₀ heterocyclylene, and -(CO)-;
each L¹ is independently absent, or is a non-cleavable sequence, or is a cleavable sequence comprising an enzymatically cleavable amino acid sequence, wherein the enzymatically cleavable amino acid sequence comprises 1, 2, 3, 4, 5, 6, 7, 8, 9, or 10 amino acids;
each L² is independently absent, or is 1), 2), or the combination of 1) and 2) below:
   1) -NH-C₂₋₂₀ alkylene, wherein one or more -CH₂- structures in the alkylene are optionally substituted by the following groups: -CR³R⁴-, -O-, -(CO)-, -S-, -S(=O)₂-, -NR⁵-, -N^{⊕}R⁶R⁷-, C₄₋₁₀ cycloalkylene, C₄₋₁₀ heterocyclylene, and phenylene, wherein the cycloalkylene, heterocyclylene, and phenylene are each independently unsubstituted or substituted with at least one substituent selected from halogen, -C₁₋₁₀ alkyl, -C₁₋₁₀ haloalkyl, -C₁₋₁₀ alkylene-NH-R⁸, and -C₁₋₁₀ alkylene-O-R⁹; and
   2) an amino acid residue sequence, i.e., -*(AA)ₙ**-, wherein n is an integer of 1-100, AA is each independently an amino acid residue, * denotes the N-terminus of a corresponding amino acid, ** denotes the C-terminus of a corresponding amino acid, and -(C₂H₄-O)ₘ-(CH₂)ₚ- is optionally present between the amino group and the α-carbon of one amino acid, wherein m is 1, 2, 3, 4, 5, 6, 7, 8, 9, or 10; p is 0, 1, 2, or 3, and the * terminus forms an amide bond with the carbonyl group in the trisaccharide structure,
wherein, B, L¹, and L² are not simultaneously absent;
R¹, R², R³, R⁴, R⁵, R⁶, R⁷, R⁸, and R⁹ are each independently selected from hydrogen, halogen, substituted or unsubstituted -C₁₋₁₀ alkyl, and C₄₋₁₀ cycloalkylene; or R¹ and R², together with the carbon atom to which they are attached, form a 3-6 membered cycloalkylene, and/or R³ and R⁴, together with the carbon atom to which they are attached, form a 3-6 membered cycloalkylene;
P is the payload linked to B moiety, L¹ moiety, or L² moiety;
D is as defined herein.

In another embodiment, -L-(P)ₜ is 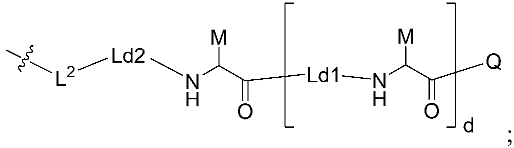 that is, formula (I) is: 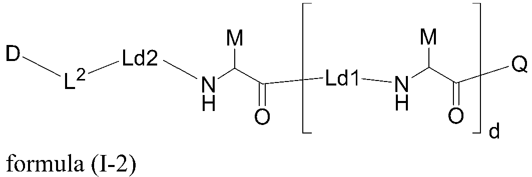 wherein,
Ld2 and each Ld1 are independently a bond, or are selected from -NH-C₁₋₂₀ alkylene-(CO)- and -NH-(PEG)ᵢ-(CO)-, or are natural amino acids each independently unsubstituted or substituted with -CO-(PEG)ⱼ-R¹¹ on the side chain or oligomeric natural amino acids with a degree of polymerization of 2-10 (i.e., 2, 3, 4, 5, 6, 7, 8, 9, or 10);
the -(PEG)ᵢ- and -(PEG)ⱼ- are each a PEG fragment comprising a specified number of consecutive -(O-C₂H₄)- structural units or consecutive -(C₂H₄-O)- structural units, optionally with an additional C₁₋₁₀ alkylene at one terminus;
M is hydrogen or LKa-L²-L¹-B-P;
Q is NH₂ or L²-L¹-B-P;
provided that the following condition is excluded: M is hydrogen and Q is NH₂;
each LKa is independently selected from 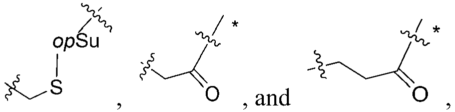
opSu is 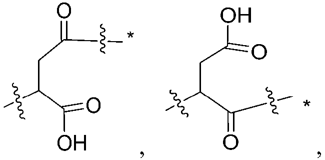 or a mixture thereof, wherein * denotes the linker moiety to L²;
each L² is independently absent, or is 1), 2), or the combination of 1) and 2) below:
   1) -NH-C₂₋₂₀ alkylene, wherein one or more -CH₂- structures in the alkylene are optionally substituted by the following groups: -CR³R⁴-, -O-, -(CO)-, -S-, -S(=O)₂-, -NR⁵-, -N^{⊕}R⁶R⁷-, C₄₋₁₀ cycloalkylene, C₄₋₁₀ heterocyclylene, and phenylene, wherein the cycloalkylene, heterocyclylene, and phenylene are each independently unsubstituted or substituted with at least one substituent selected from halogen, -C₁₋₁₀ alkyl, -C₁₋₁₀ haloalkyl, -C₁₋₁₀ alkylene-NH-R⁸, and -C₁₋₁₀ alkylene-O-R⁹, preferably, -NH-(C₂H₄O)₂-(C₂H₄)CO-; and
   2) an amino acid residue sequence, i.e., -*(AA)ₙ**-, wherein n is an integer of 1-100, AA is each independently an amino acid residue, * denotes the N-terminus of a corresponding amino acid, ** denotes the C-terminus of a corresponding amino acid, and -(C₂H₄-O)ₘ-(CH₂)ₚ- is optionally present between the amino group and the α-carbon of one amino acid, wherein m is 1, 2, 3, 4, 5, 6, 7, 8, 9, or 10; p is 0, 1, 2, or 3, and the * terminus forms an amide bond with the carbonyl group in the trisaccharide structure, preferably, -Gly-Gly-Gly-, wherein
each L¹ is independently absent, or is a non-cleavable sequence (for example, the payload and the antibody are conjugated via a thioether bond), or is a cleavable sequence comprising an enzymatically cleavable amino acid sequence, wherein the enzymatically cleavable amino acid sequence comprises 1, 2, 3, 4, 5, 6, 7, 8, 9, or 10 amino acids;
each B is independently absent, or is 1), 2), or the combination of 1) and 2) below: 1) a self-immolative spacer Sp1; and 2) one divalent group, or a combination of two or more divalent groups, wherein the divalent group is selected from: -CR¹R²-, C₁₋₁₀ alkylene, C₄₋₁₀ cycloalkylene, C₄₋₁₀ heterocyclylene, and -(CO)-;
wherein, B, L¹, and L² are not simultaneously absent;
P is the payload linked to B moiety, L¹ moiety, or L² moiety;
R¹, R², R³, R⁴, R⁵, R⁶, R⁷, R⁸, and R⁹ are each independently selected from hydrogen, halogen, substituted or unsubstituted -C₁₋₁₀ alkyl, and C₄₋₁₀ cycloalkylene; or R¹ and R², together with the carbon atom to which they are attached, form a 3-6 membered cycloalkylene, and/or R³ and R⁴, together with the carbon atom to which they are attached, form a 3-6 membered cycloalkylene;
R¹¹ is C₁₋₁₀ alkyl;
d is 0, 1, 2, 3, 4, 5, or 6;
each i is independently an integer of 1-100 (e.g., 1, 2, 3, 4, 5, 6, 7, 8, 9, or 10), preferably 1-20;
preferably, each i is independently an integer of 1-12, more preferably 2-8, particularly 4;
each j is independently an integer of 1-100 (e.g., 1, 2, 3, 4, 5, 6, 7, 8, 9, or 10), preferably 1-20;
preferably, each j is independently an integer of 1-12, more preferably 8-12, particularly 8, 9, 12, or 13.

In one embodiment, at least one of B, L¹, and L² is not "absent".

In one embodiment, L² is an amino acid residue sequence, i.e., -*(AA)ₙ**-, wherein n is an integer of 1-100, AA is each independently an amino acid residue, * denotes the N-terminus of a corresponding amino acid, ** denotes the C-terminus of a corresponding amino acid, and - (C₂H₄-O)ₘ-(CH₂)ₚ- is optionally present between the amino group and the α-carbon of one amino acid, wherein m is 1, 2, 3, 4, 5, 6, 7, 8, 9, or 10; p is 0, 1, 2, or 3, and the * terminus forms an amide bond with the carbonyl group in the trisaccharide structure. In one embodiment, AA is each independently any one of Phe, Lys, Gly, Ala, Leu, Asn, Val, Ile, Pro, Trp, Ser, Tyr, Cys, Met, Asp, Gln, Glu, Thr, Arg, and His, or any combination thereof. In one embodiment, n is an integer of 1-50, preferably an integer of 1-30, preferably an integer of 1-20, preferably an integer of 1-10, and preferably 1, 2, 3, 4, 5, 6, 7, 8, 9, or 10.

In one embodiment, L¹ is a cleavable sequence comprising an enzymatically cleavable amino acid sequence, wherein the enzymatically cleavable amino acid sequence comprises 1, 2, 3, 4, 5, 6, 7, 8, 9, or 10 amino acids. In one embodiment, the enzymatically cleavable amino acid sequence is selected from -Val-Ala-, -Gly-Gly-Phe-Gly-, -Phe-Lys-, -Val-Cit-, -Val-Lys-, -Gly-Phe-Leu-Gly-, -Ala-Leu-Ala-Leu-, -Ala-Ala-Ala-, and a combination thereof; preferably, the enzymatically cleavable amino acid sequence is -Gly-Gly-Phe-Gly-. In one embodiment, L¹ is any one of Val, Cit, Phe, Lys, Gly, Ala, Leu, and Asn, or any combination thereof, preferably, -Val-Ala-, -Gly-Gly-Phe-Gly-, -Phe-Lys-, -Val-Cit-, -Val-Lys-, -Gly-Phe-Leu-Gly-, -Ala-Leu-Ala-Leu-, -Ala-Ala-Ala-, or a combination thereof. In one embodiment, L¹ denotes -Val-Cit-. In one embodiment, Sp1 is selected from PABC (p-aminobenzyloxycarbonyl), acetal, heteroacetal, and a combination thereof; preferably, Sp1 is acetal, heteroacetal, or PABC; more preferably, the heteroacetal is selected from N,O-heteroacetal; even more preferably, Sp1 is - O-CH₂-U- or -NH-CH₂-U-, wherein -O- or -NH- is linked to the enzymatically cleavable amino acid sequence, and U is absent or is CH ₂, O, S, or NH, preferably O or S; still more preferably, Sp1 is PABC.

In one embodiment, B is absent. In one embodiment, B is 1), 2), or the combination of 1) and 2) below: 1) a self-immolative spacer Sp1; and 2) one divalent group, or a combination of two or more divalent groups, wherein the divalent group is selected from: -CR¹R²-, C₁₋₁₀ alkylene, and -(CO)-. In one embodiment, B is linked to the payload via an amide bond, an ester bond, or an ether bond. In one embodiment, B is selected from: 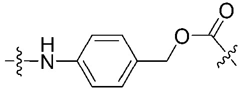 (-PABC-), -NH-CH₂-U-, and -NH-CH₂-U-(CH₂)_{g}-(CO)-, wherein g is 1, 2, 3, 4, 5, or 6; U is absent, or is CH₂, O, S, or NH, preferably O or S.

In one embodiment, -L¹-B- denotes -Val-Cit-PABC-.

In one embodiment, -L¹-B- denotes -Gly-Gly-Phe-Gly-.

In one embodiment, -L²-L¹-B- denotes -Gly-Gly-Gly-Val-Cit-PABC-.

In one embodiment, -L²-L¹-B- denotes -HN-(C₂H₄-O)ₘ-(CH₂)ₚ-Gly-Gly-Phe-Gly-.

In one embodiment, Ld2 and each Ld1 are independently selected from a bond, 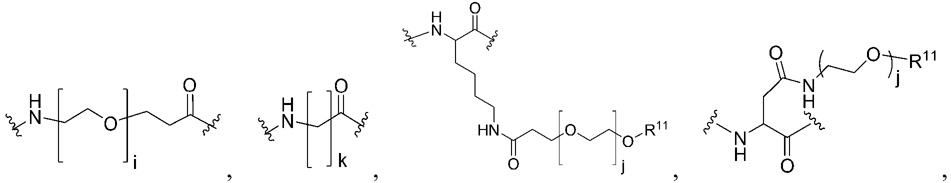 and 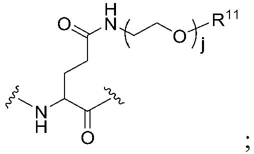 each i, j, and k is independently selected from integers of 1-100.

In one embodiment, each i, j, and k is independently selected from integers of 1-20. In one embodiment, each i, j, and k is independently selected from integers of 1-12.

In one embodiment, each i is independently selected from integers of 2-8, particularly 4.

In one embodiment, each j is independently selected from integers of 8-12, particularly 8 or 12. In one embodiment, each k is independently selected from integers of 1-7, particularly 1, 3, or 5.

In one embodiment, Ld2 and each Ld1 are independently selected from a bond, C₁₋₂₀ alkylene with amino and carbonyl separately at the two termini, a PEG fragment of a certain length (denoted as -(PEG)ᵢ-) with amino and carbonyl separately at the two termini, and one or more natural amino acids each independently unsubstituted or substituted with a PEG fragment of a certain length (denoted as -CO-(PEG)ⱼ-) on the side chain.

In one embodiment, -(PEG)ᵢ- comprises -(O-C₂H₄)ᵢ- or -(C₂H₄-O)ᵢ-, optionally with additional C₁₋₁₀ alkylene at one terminus; the -(PEG)ⱼ- comprises -(O-C₂H₄)ⱼ- or -(C₂H₄-O)ⱼ-, optionally with additional C₁₋₁₀ alkylene at one terminus. In one specific embodiment, the -(PEG)ᵢ-comprises -C₂H₄-(O-C₂H₄)ᵢ- or -(C₂H₄-O)ᵢ-C₂H₄-.

In one embodiment, the payload can be selected from the group consisting of small molecule compounds (for example, small molecule drugs with various mechanisms of action, including various conventional small molecule drugs, photoacoustic kinetic therapeutic drugs, photothermal therapeutic drugs, etc., such as chemotherapeutic drugs, small molecule targeted drugs, immune agonists, etc., for example, conventional cytotoxic drugs such as cisplatin, paclitaxel, 5-fluorouracil, cyclophosphamide, bendamustine, etc.; small molecule targeted drugs such as imatinib mesylate, gefitinib, anlotinib, etc.; immune agonists such as STING agonists, TLR agonists, etc.), nucleic acids and nucleic acid analogs, tracer molecules (including fluorescent molecules, biotin, fluorophores, chromophores, spin resonance probes, radiolabels and the like), short peptides, polypeptides, peptidomimetics, and proteins. In one embodiment, the payload is selected from the group consisting of small molecule compounds and nucleic acid molecules. In one preferred embodiment, the payload is selected from small molecule compounds. In one more preferred embodiment, the payload is selected from the group consisting of cytotoxins and fragments thereof.

In one embodiment, the payload is a cytotoxin or a fragment thereof, with optional derivatization to link to the L portion in formula (1), or the B, L², or L¹ portion in the compound of formula (I-1) or formula (I-2).

In one embodiment, the cytotoxin is selected from the group consisting of drugs targeting the microtubule cytoskeleton. In one preferred embodiment, the cytotoxin is selected from the group consisting of taxanes, maytansinoids, auristatins, epothilones, combretastatin A-4 phosphate, combretastatin A-4 and derivatives thereof, indole-sulfonamides, vinca alkaloids such as vinblastine, vincristine, vindesine, vinorelbine, vinflunine, vinglycinate and anhydrovinblastine, dolastatin 10 and analogs thereof, halichondrin B, indole-3-oxoacetamides, podophyllotoxins, 7-diethylamino-3-(2'-benzoxazolyl)-coumarin (DBC), discodermolide, and laulimalide. In another embodiment, the cytotoxin is selected from the group consisting of DNA topoisomerase inhibitors (e.g., camptothecins and derivatives thereof), mitoxantrone, and mitoguazone. In one preferred embodiment, the cytotoxin is selected from the group consisting of nitrogen mustards (e.g., chlorambucil, chlornaphazine), cholophosphamide, estramustine, ifosfamide, mechlorethamine, mechlorethamine oxide hydrochloride, melphalan, novembichin, phenamet, phenesterine, prednimustine, trofosfamide, and uramustine. In yet another preferred embodiment, the cytotoxin is selected from the group consisting of nitrosoureas (e.g., carmustine), flubenzuron, formoterol, lomustine, nimustine, and ranimustine. In one embodiment, the cytotoxin is selected from the group consisting of aziridines. In one preferred embodiment, the cytotoxin is selected from the group consisting of benzodopa, carboquone, meturedepa, and uredepa. In one embodiment, the cytotoxin is selected from the group consisting of anti-tumor antibiotics. In one preferred embodiment, the cytotoxin is selected from the group consisting of enediyne antibiotics. In one more preferred embodiment, the cytotoxin is selected from the group consisting of dynemicin, esperamicin, neocarzinostatin, and aclacinomycin. In another preferred embodiment, the cytotoxin is selected from the group consisting of actinomycin, anthramycin, bleomycins, actinomycin C, carabicin, carminomycin, cardinophyllin, carminomycin, actinomycin D, daunorubicin, detorubicin, adriamycin, epirubicin, esorubicin, idarubicin, marcellomycin, mitomycins, nogalamycin, olivomycin, peplomycin, porfiromycin, puromycin, ferric adriamycin, rodorubicin, rufocromomycin, streptozocin, zinostatin, and zorubicin. In yet another preferred embodiment, the cytotoxin is selected from the group consisting of trichothecenes. In one more preferred embodiment, the cytotoxin is selected from the group consisting of T-2 toxin, verracurin A, bacillocporin A, and anguidine. In one embodiment, the cytotoxin is selected from the group consisting of anti-tumor amino acid derivatives. In one preferred embodiment, the cytotoxin is selected from the group consisting of ubenimex, azaserine, and 6-diazo-5-oxo-L-norleucine. In another embodiment, the cytotoxin is selected from the group consisting of folic acid analogs. In one preferred embodiment, the cytotoxin is selected from the group consisting of denopterin, methotrexate, pteropterin, trimetrexate, and edatrexate. In one embodiment, the cytotoxin is selected from the group consisting of purine analogs. In one preferred embodiment, the cytotoxin is selected from the group consisting of fludarabine, 6-mercaptopurine, thiamiprine, and thioguanine. In yet another embodiment, the cytotoxin is selected from the group consisting of pyrimidine analogs. In one preferred embodiment, the cytotoxin is selected from the group consisting of ancitabine, gemcitabine, enocitabine, azacitidine, 6-azauridine, carmofur, cytarabine, dideoxyuridine, doxifluridine, and floxuridine. In one embodiment, the cytotoxin is selected from the group consisting of androgens. In one preferred embodiment, the cytotoxin is selected from the group consisting of calusterone, dromostanolone propionate, epitiostanol, mepitiostane, and testolactone. In another embodiment, the cytotoxin is selected from the group consisting of anti-adrenals. In one preferred embodiment, the cytotoxin is selected from the group consisting of aminoglutethimide, mitotane, and trilostane. In one embodiment, the cytotoxin is selected from the group consisting of anti-androgens. In one preferred embodiment, the cytotoxin is selected from the group consisting of flutamide, nilutamide, bicalutamide, leuprorelin acetate, and goserelin. In yet another embodiment, the cytotoxin is selected from the group consisting of protein kinase inhibitors and proteasome inhibitors. In another embodiment, the cytotoxin is selected from the group consisting of vinca alkaloids, colchicines, taxanes, auristatins, maytansinoids, calicheamicin, doxorubicin, duocarmycin, SN-38, cryptophycin analogs, deruxtecan, duocarmazine, calicheamicin, centanamycin, dolastansine, pyrrolobenzodiazepine (PBD), and exatecan. In one embodiment, the cytotoxin is selected from the group consisting of vinca alkaloids, colchicines, taxanes, auristatins, and maytansinoids.

In one embodiment, the cytotoxin is exatecan or a derivative thereof, such as DX8951f.

In one embodiment, the cytotoxin is a maytansinoid, such as DM1. It should be noted that when a cytotoxin comprising a sulfhydryl moiety is used, the sulfhydryl moiety is capable of reacting with a maleimide moiety to form thiosuccinimide, e.g., a maytansinoid, such as DM1, and the cytotoxin can be directly linked through the thiosuccinimide. In this case, it will be appreciated that in some embodiments, the payload and the sulfhydryl moiety together constitute the cytotoxin, and thus in this case, the payload denotes the rest of the cytotoxin molecule except for the sulfhydryl moiety.

In one embodiment, the cytotoxin is an auristatin, such as MMAE (monomethyl auristatin E), MMAF (monomethyl auristatin F), or MMAD (monomethyl auristatin D). In one embodiment, the cytotoxin is MMAE. The synthesis and structure of auristatin compounds are described in US20060229253, the entire disclosure of which is incorporated herein by reference.

The payload contains a reactive group that can react with the reactive group in the compound of formula (I) and thereby covalently conjugate the payload to the compound of formula (I). Compounds that do not contain a reactive group require appropriate derivatization to obtain a payload.

In one embodiment, the cytotoxin is a compound of formula (i) below: 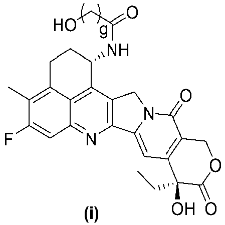
wherein, g is any integer of 1-6;
in one embodiment, g is any integer of 1-3, preferably 1.

In one embodiment, the cytotoxin is selected from the group consisting of compounds 1-16 below, wherein the wavy bond ( 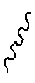 ) denotes the linking site for linking to the compound of formula (I).

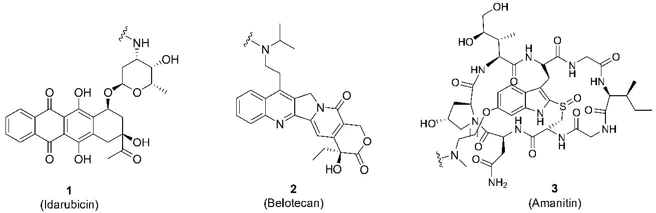

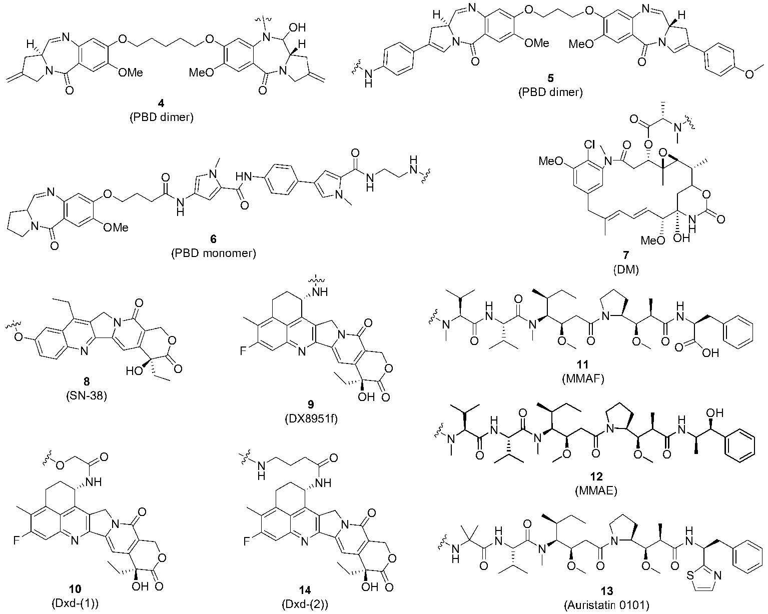

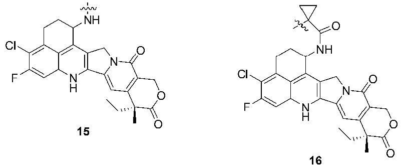

In one embodiment, the linker-payload compound of formula (I) is: 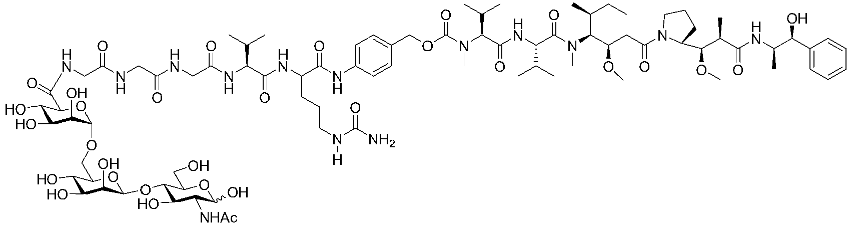 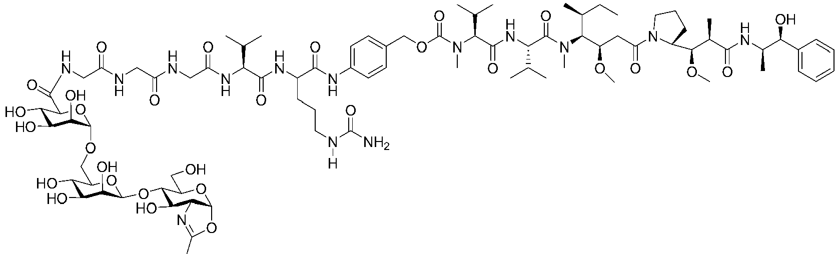 for example,

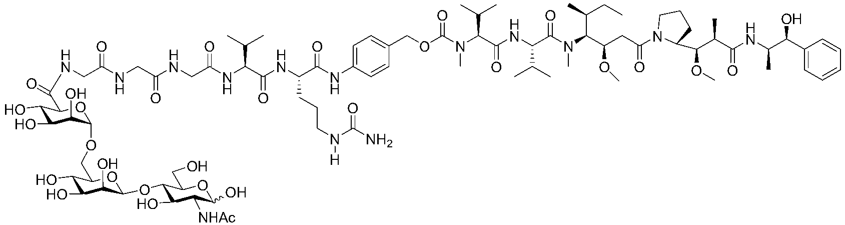

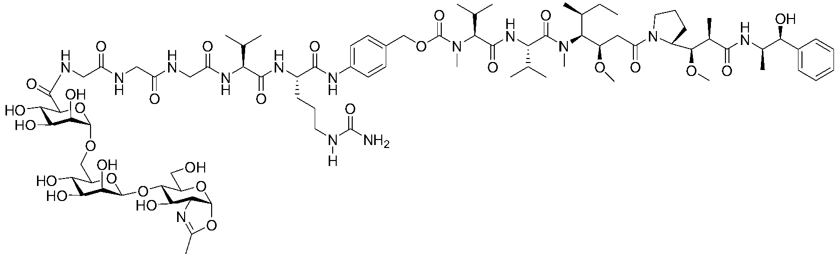

In another aspect, the present disclosure provides a linker-payload compound obtained by a method comprising:
(i) oxidizing a primary alcohol at position 6 of a terminal first hexose unit in a trisaccharide comprising the terminal first hexose unit and a terminal N-acetylglucosamine (GlcNAc) unit to a carboxyl group, to obtain an intermediate compound (a) having a carboxyl group, wherein a second hexose unit or a derivative moiety thereof is present between the terminal first hexose unit and the terminal N-acetylglucosamine (GlcNAc) unit; and
(ii) reacting the carboxyl group in the intermediate compound (a) obtained in step (i) with the reactive group in the linker-payload compound (b) having a reactive group at the terminus to obtain the linker-payload compound.

In one embodiment of this another aspect, the first hexose unit is selected from glucosyl, mannosyl, galactosyl, fructosyl, gulosyl, and idosyl; and/or
the second hexose unit is selected from glucosyl, mannosyl, galactosyl, and fructosyl; and/or
the first hexose unit or the derivative moiety thereof in the trisaccharide structure is linked to the second hexose unit or the derivative moiety thereof via an α-(1→6) glycosidic bond; and/or the second hexose unit or the derivative moiety thereof in the trisaccharide structure is linked to a β-D-*N*-acetylglucosamine moiety or a β-D-glucose oxazoline moiety via a β-(1→4) glycosidic bond; and/or
the derivative is independently selected from derivatives of a monosaccharide with a hydroxyl being substituted by an acylamino (e.g., alkanoylamino, e.g., formylamino, acetylamino, propionylamino, and especially acetylamino).

In one embodiment of this another aspect,
the first hexose unit is selected from mannosyl, glucosyl, and galactosyl; and/or
the second hexose unit is selected from mannosyl, glucosyl, and galactosyl.

In one embodiment of this another aspect, the trisaccharide in step (i) has the following structure:

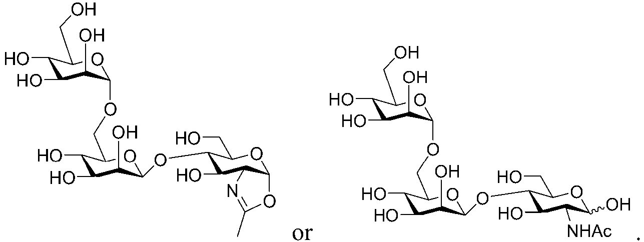

In one embodiment of this another aspect, the trisaccharide in step (i) has the following structure:

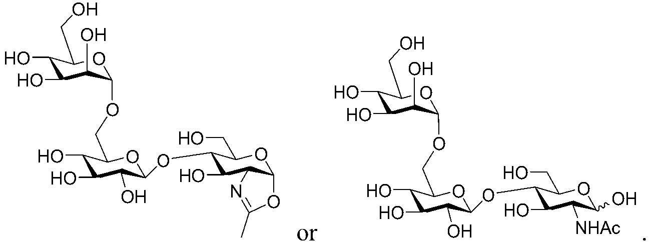

In one embodiment of this another aspect, the trisaccharide in step (i) has the following structure:

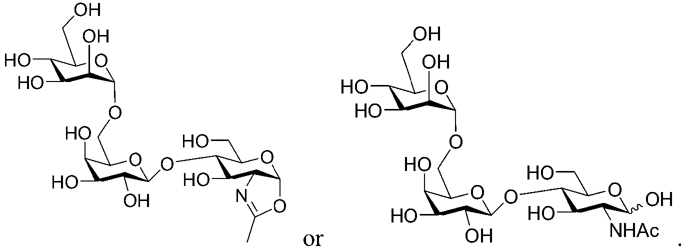

In one embodiment of this another aspect, the trisaccharide in step (i) has the following structure:

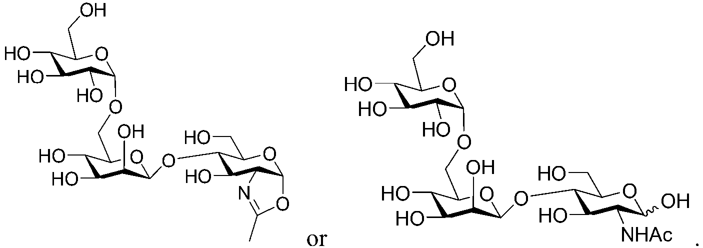

In one embodiment of this another aspect, the trisaccharide in step (i) has the following structure:

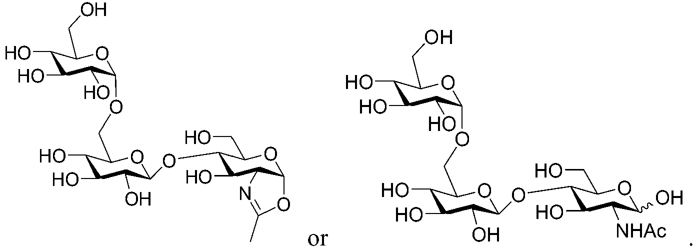

In one embodiment of this another aspect, the trisaccharide in step (i) has the following structure:

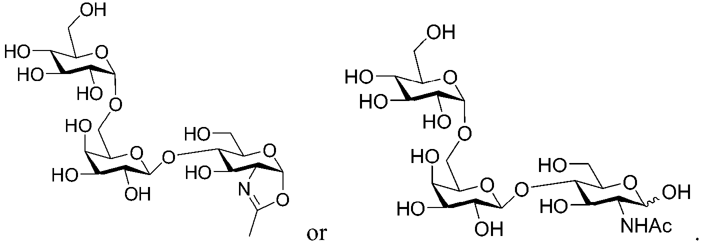

In one embodiment of this another aspect, the trisaccharide in step (i) has the following structure:

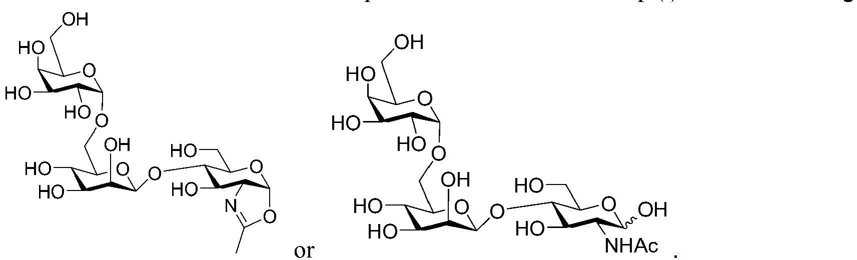

In one embodiment of this another aspect, the trisaccharide in step (i) has the following structure:

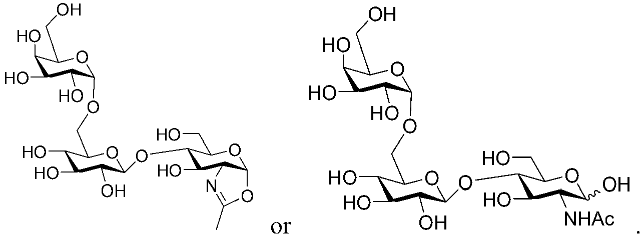

In one embodiment of this another aspect, the trisaccharide in step (i) has the following structure:

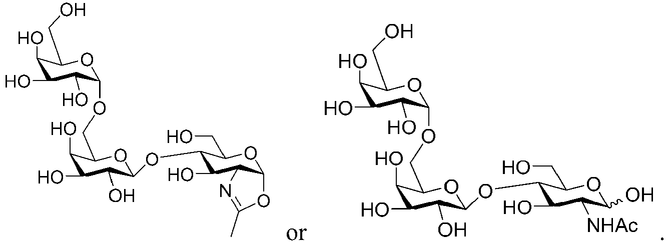

In one embodiment of this another aspect, the reactive group of the linker-payload compound (b) having a reactive group at the terminus in step (ii) is amino.

In one embodiment of the other aspect, the method further comprises: converting the intermediate compound (a) having a carboxyl group into an acyl halide, and further reacting the acyl halide with the linker-payload compound (b) having a reactive group at the terminus to obtain the linker-payload compound.

In one embodiment of this another aspect, the linker-payload compound is the linker-payload compound as defined in the first aspect or in the embodiments thereof.

### Method for Preparing Linker-Payload Compound

In a second aspect, the present disclosure provides a method for preparing a linker-payload compound of formula (I), wherein unless otherwise stated, the variables are as defined in the first aspect or the embodiments thereof.

In one embodiment, the method comprises: subjecting D and L'-(P)ₜ to an amide formation reaction to form D-L-(P)ₜ, wherein L' is identical to L defined in the first aspect or the embodiments thereof, except that the moiety that is -NH- (linked to D in L) is H₂N- in L'.

In one embodiment, when D in the prepared linker-payload compound of formula (I) is 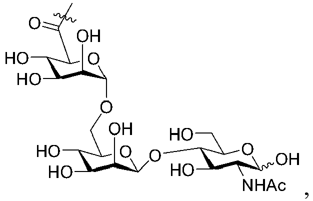 the method further comprises: condensing 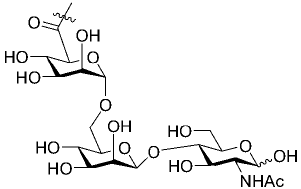 into 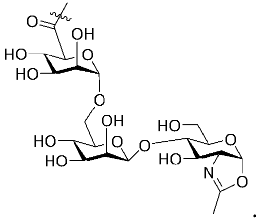 In one embodiment, the condensation is conducted in the presence of water, a base, and 2-chloro-1,3-dimethylimidazolinium chloride (DMC, CAS: 37091-73-9). In one embodiment, the base is an inorganic base or an organic base. In one embodiment, the inorganic base is potassium carbonate, potassium phosphate, etc. In one embodiment, the organic base is an amine, e.g., a tertiary amine, such as triethylamine (Et₃N).

In one embodiment, the amide formation reaction is conducted in the presence of an organic solvent, an organic base, and a condensation reagent. In one embodiment, the organic solvent is selected from N,N-dimethylformamide (DMF) and N,N-dimethylacetamide (DMA). In one embodiment, the organic base is selected from diisopropylethylamine (DIPEA) and N-methylmorpholine (NMM). In one embodiment, the condensing agent is selected from 2-(7-azabenzotriazol-1-yl)-N,N,N',N'-tetramethyluronium hexafluorophosphate (HATU), benzotriazole-N,N,N',N'-tetramethyluronium hexafluorophosphate (HBTU), 2-(1H-benzotriazol-1-yl)-1,1,3,3-tetramethyluronium tetrafluoroborate (TBTU), 1H-benzotriazole-1-yloxytripyrrolidinyl hexafluorophosphate (PyBOP), N,N'-diisopropylcarbodiimide (DIC), and 1-(3-dimethylaminopropyl)-3-ethylcarbodiimide (EDCI).

In one embodiment, D-OH is

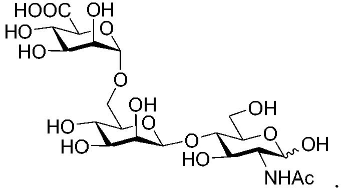

In one embodiment, 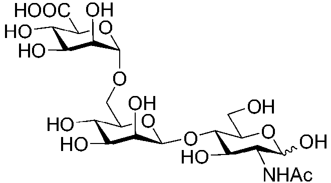 is prepared from 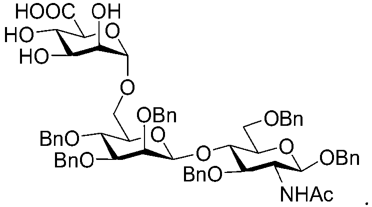 In one embodiment, the preparation is conducted in a condition of debenzylation protection of the substrate. In one embodiment, the deprotection is conducted in a hydrogen atmosphere and the presence of a palladium-based catalyst. In one embodiment, the palladium-based catalyst is selected from palladium on carbon and palladium hydroxide on carbon. In one embodiment, the palladium-based catalyst is palladium on carbon.

In one embodiment, 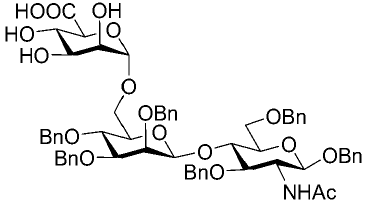 is prepared from 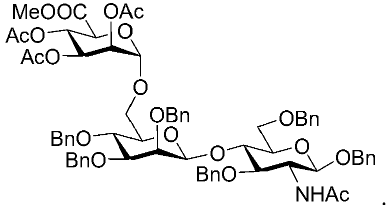 In one embodiment, the preparation is conducted in a basic condition, and the reaction system is then adjusted to an acidic condition for purification. In one embodiment, the basic condition is a pH of about 11-12 in the reaction system, and the acidic condition is a pH of about 6-7 in the reaction system.

In one embodiment, 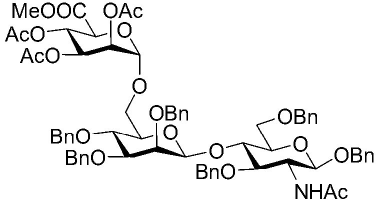 is prepared from 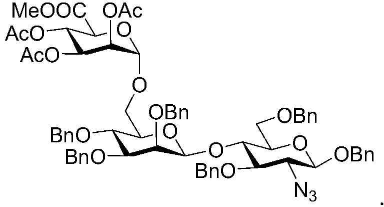 In one embodiment, the preparation is conducted in the presence of thioacetic acid (AcSH) and an organic solvent. In one embodiment, the organic solvent is selected from chloroform and pyridine or a mixture thereof.

In one embodiment, 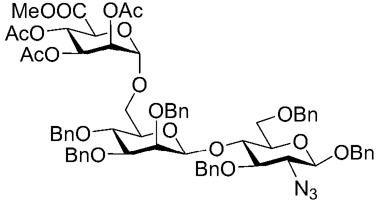 is prepared from 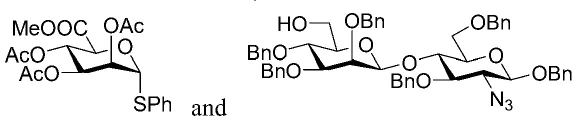 by forming an α-(1→6) glycosidic bond. In one embodiment, the preparation is conducted in a glycosidic bond formation condition. In one embodiment, the glycosidic bond formation condition comprises the use of N-iodosuccinimide and silver triflate in the absence of water.

In one embodiment, 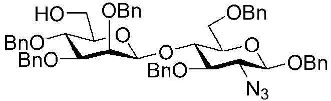 is prepared from 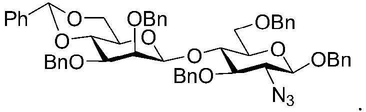 In one embodiment, the preparation comprises the use of dibutylboron triflate and borane-tetrahydrofuran.

In one embodiment, 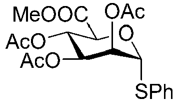 is prepared from 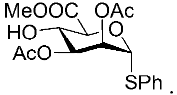 In one embodiment, the preparation comprises the use of acetic anhydride.

In one embodiment, 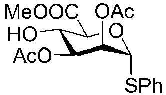 is prepared from 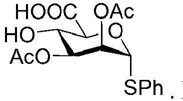 In one embodiment, the preparation comprises the use of iodomethane.

In one embodiment, 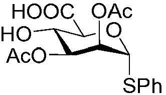 is prepared from 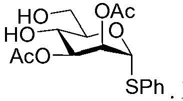 In one embodiment, the preparation comprises the use of 2,2,6,6-tetramethylpiperidine oxide (TEMPO) and iodobenzenediacetic acid.

In one embodiment, 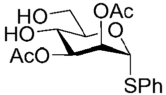 is prepared from 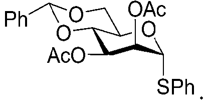 In one embodiment, the preparation is conducted in the presence of an acid. In one embodiment, the acid is p-toluenesulfonic acid.

In one embodiment, 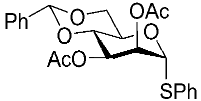 is prepared from 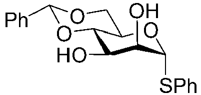 . In one embodiment, the preparation comprises the use of a catalyst and acetic anhydride. In one embodiment, the catalyst is 4-dimethylaminopyridine (DMAP).

### Antibody-Conjugated Drug

In a third aspect, the present disclosure provides an antibody-conjugated drug based on site-specific linkage at an N-glycosylation site in an antibody Fc region having the structure of formula (II): 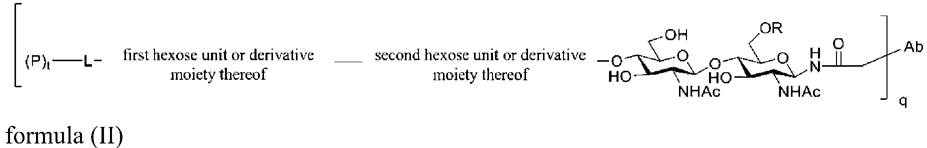 wherein
P is the payload;
R is hydrogen or α-L-fucosyl;
q is 1 or 2;
Ab is an antibody or an antigen-binding fragment (e.g., the -NHC(O)CH₂- linked to Ab in formula (II) is derived from asparagine at position 297 of the antibody Fc region);
the first hexose unit or the derivative moiety thereof is linked to the second hexose unit or the derivative moiety thereof via an α-(1→6) glycosidic bond;
the second hexose unit or the derivative moiety thereof is linked to a β-D-*N*-acetylglucosamine moiety via a β-(1→4) glycosidic bond;
the -OH at position 6 of the first hexose unit or the derivative moiety thereof is oxidized to - C(O)-;
L is a linker, and L is directly linked to -C(O)- in the first hexose unit or the derivative moiety thereof via the terminal -NH-, wherein when L is an unbranched linker, it is linked to one P, and t is 1, and when L is a branched linker, each branch can be linked to one P, and t is an integer greater than 1 (e.g., 2, 3, 4, 5, 6, 7, 8, 9, or 10).

In one embodiment, the first hexose unit or the derivative moiety thereof is selected from glucosyl, mannosyl, galactosyl, fructosyl, gulosyl, idosyl, and derivatives thereof.

In one embodiment, the first hexose unit or the derivative moiety thereof is selected from 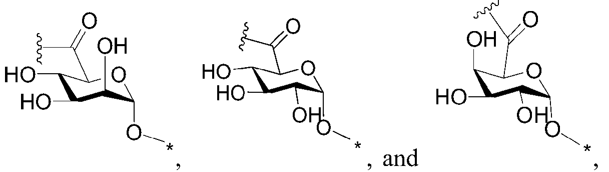 wherein, 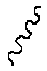 denotes the site for linking to L; * denotes the site for linking to the second hexose unit or the derivative moiety thereof.

In one embodiment, the second hexose unit or the derivative moiety thereof is selected from glucosyl, mannosyl, galactosyl, fructosyl, and derivatives thereof. In one embodiment, the second hexose unit or the derivative moiety thereof is selected from 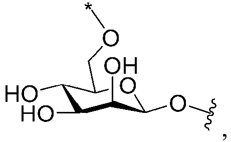 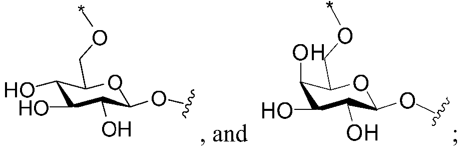 wherein 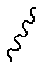 denotes the site for linking to the β-D-*N-*acetylglucosamine moiety; * denotes the site for linking to the first hexose unit or the derivative moiety thereof.

In one embodiment, the hexose derivative moiety is independently selected from a uronic acid and a monosaccharide with hydroxyl being substituted by an acylamino (e.g., alkanoylamino, e.g., formylamino, acetylamino, propionylamino, and especially acetylamino).

In one embodiment, the -NHC(O)CH₂- linked to Ab in formula (II) is derived from asparagine at position 297 of the antibody Fc region.

In some embodiments, Ab is a monoclonal antibody. In some embodiments, Ab is an anti-human HER2 antibody or an antigen-binding fragment thereof. Examples of the anti-human HER2 antibody include, but are not limited to, pertuzumab and trastuzumab. In one embodiment, Ab is trastuzumab.

In one embodiment, formula (II) is formula (II-1):

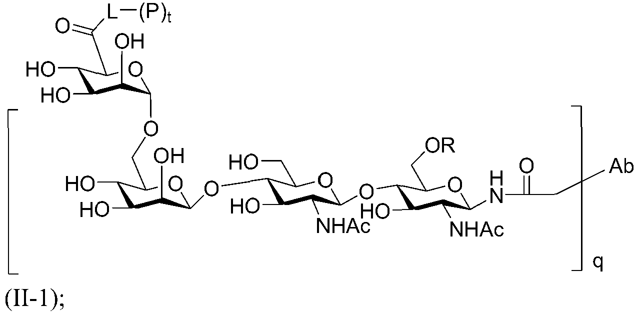

In one embodiment, formula (II) is formula (II-2):

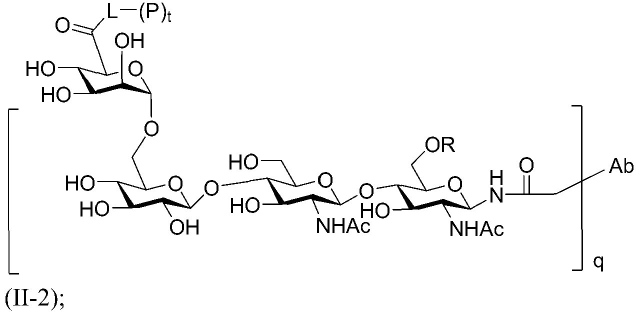

In one embodiment, formula (II) is formula (II-3):

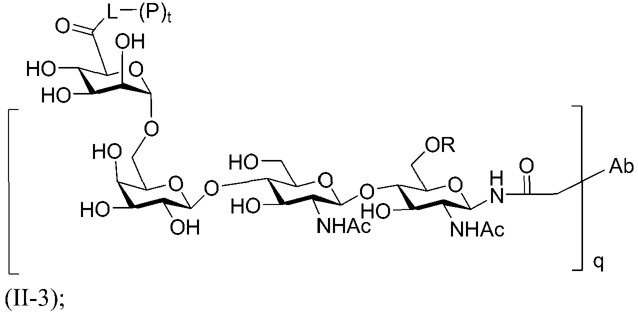

In one embodiment, formula (II) is formula (II-4):

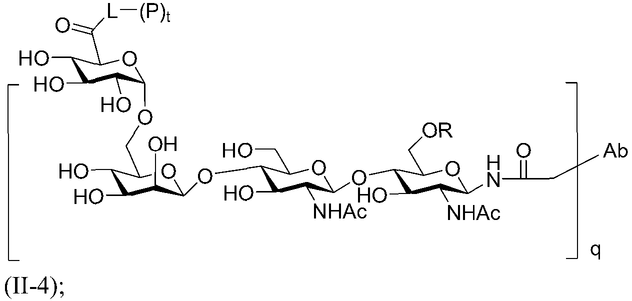

In one embodiment, formula (II) is formula (II-5):

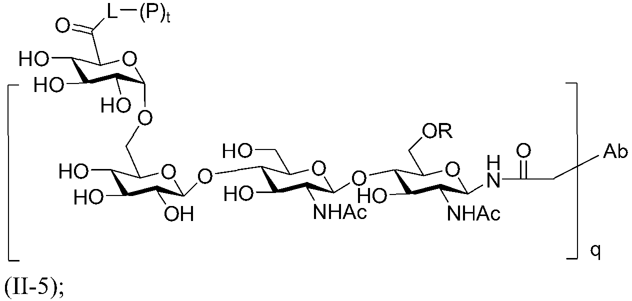

In one embodiment, formula (II) is formula (II-6):

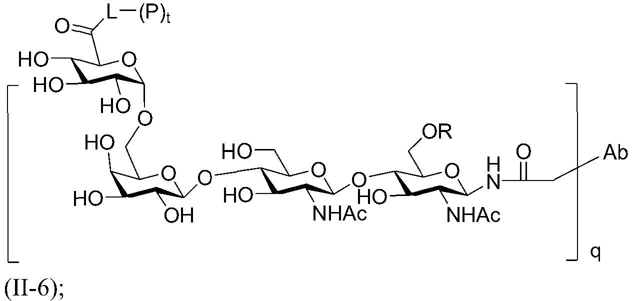

In one embodiment, formula (II) is formula (II-7):

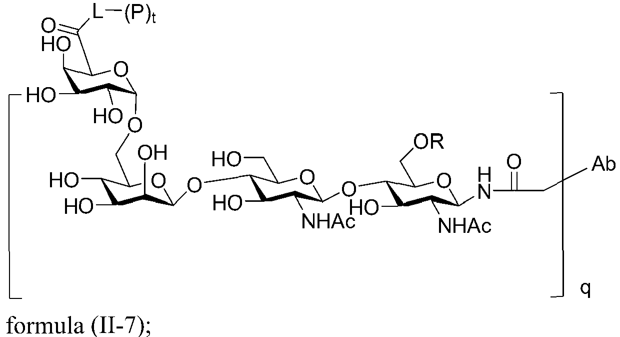

In one embodiment, formula (II) is formula (II-8):

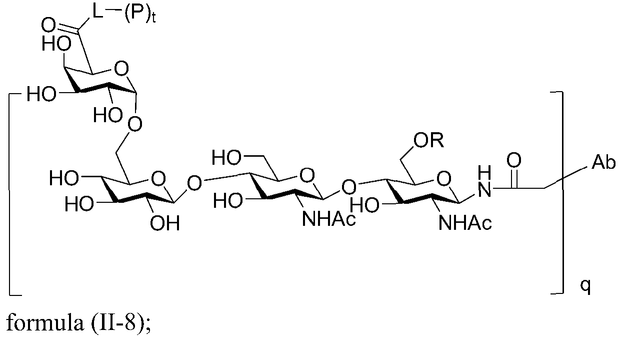

In one embodiment, formula (II) is formula (II-9): 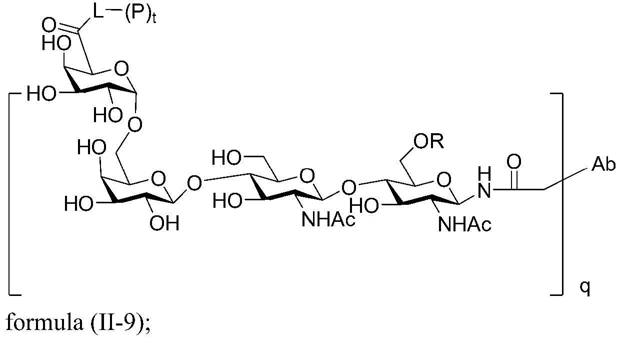 wherein
R is hydrogen or α-L-fucosyl;
q is 1 or 2;
Ab is an antibody or an antigen-binding fragment (e.g., the -NHC(O)CH₂- linked to Ab in formula (II-1) is derived from asparagine at position 297 of the antibody Fc region);
the other variables are as defined in the first aspect or the embodiments thereof.

In one preferred embodiment, Ab is an anti-ErbB2/HER2 antibody (e.g., trastuzumab); (P)ₜ-L- is:

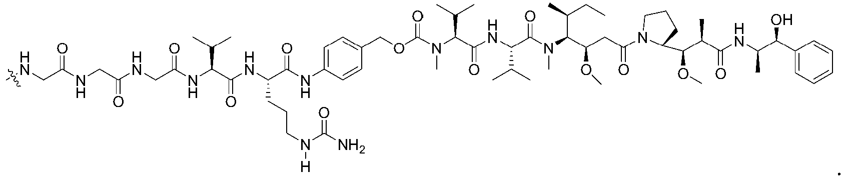

In one embodiment, R in formula (II) is α-L-fucosyl;
q is 2;
Ab is trastuzumab;
(P)ₜ-L- is 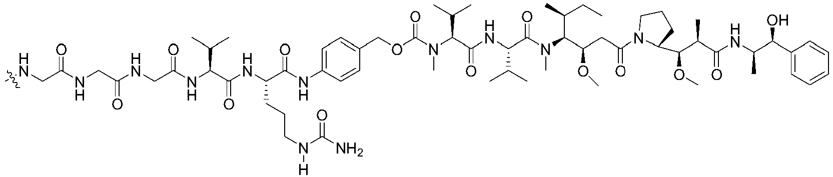 for example, 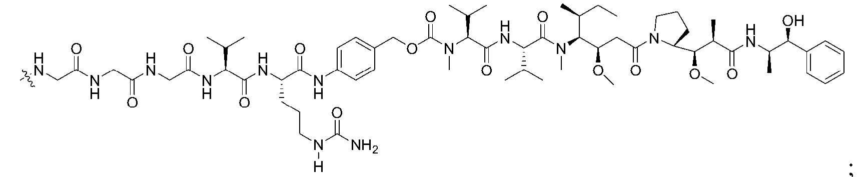
the -NHC(O)CH₂- linked to Ab in formula (II) is derived from asparagine at position 297 of the antibody Fc region.

### Method for Preparing Antibody-Conjugated Drug

In a fourth aspect, the present disclosure provides a method for preparing an antibody-conjugated drug of formula (II), comprising: conjugating a linker-payload compound of formula (I) to an antibody Ab, wherein unless otherwise stated, the variables are as defined in the third aspect or the embodiments thereof.

In one embodiment, the method comprises:
a) cleaving an N-glycan chain of the antibody Ab under the catalysis of a glycosidase or a mutant thereof, to obtain an antibody with a *N*-acetylglucosamine or fucosyl-α-1,6-*N-*acetylglucosamine modification at an N-glycosylation site in the Fc region thereof; and
b) conjugating the modified antibody obtained in step a) to the linker-payload compound described above under the catalysis of a glycosidase or a mutant thereof;
wherein the glycosidase or the mutant thereof used in step a) and step b) may be identical or different.

In one embodiment, the glycosidase or the mutant thereof used in steps a) and b) is a fucose hydrolase, a N-acetylglucosamine endohydrolase, or a mutant thereof. In one embodiment, the N-acetylglucosamine endohydrolase comprises at least one selected from Endo-S (*Streptococcus pyogenes* endoglycosidase-S), Endo-F3 (*Elizabethkingia miricola* endoglycosidase-F3), Endo-S2 (endoglycosidase-S2, or *Streptococcus pyogenes* endoglycosidase-S2), Endo-Sd (endoglycosidase-Sd, or *Streptococcus pyogenes* endoglycosidase-Sd), and Endo-CC (endoglycosidase-CC, or *Streptococcus pyogenes* endoglycosidase-CC); preferably, the endoglycosidase is Endo H, Endo D, Endo F2, Endo F3, Endo M, Endo CC1, Endo CC2, Endo Om, Endo S, or Endo S2. In one embodiment, the enzyme is Endo S2.

In one embodiment, steps a) and b) are conducted via one-pot enzymatic catalysis.

In one embodiment, steps a) and b) are conducted via one-pot enzymatic catalysis, and the enzyme is Endo S2.

### Trisaccharide Linker

In one embodiment, a trisaccharide structure of the following structural formula (IV) is disclosed: 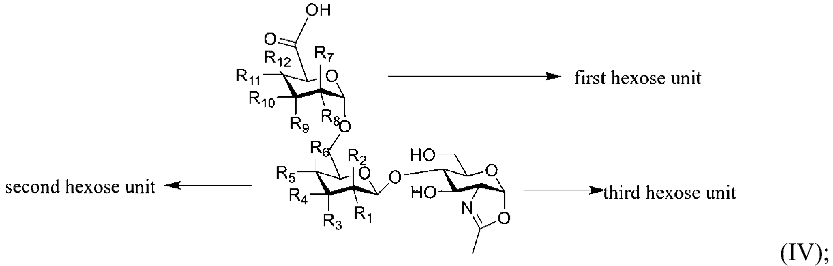
wherein, R₁-R₁₂ in formula (IV) are selected from -OH and H;
preferably, two substituents on the same carbon atom are different;
more preferably, two substituents on the same carbon atom are different, R₃ is H, and R₄ is - OH;
even more preferably, two substituents on the same carbon atom are different, R₃ is H, R₄ is - OH, R₉ is H, and R₁₀ is -OH;
still more preferably, two substituents on the same carbon atom are different, R₃ is H, R₄ is - OH, R₉ is H, R₁₀ is -OH, R₈ is H, R₇ is -OH, R₁₂ is H, and R₁₁ is -OH;
L and P are as defined for formula (I).

In one embodiment, the structure of formula (IV) is shown in formula (IV-1): 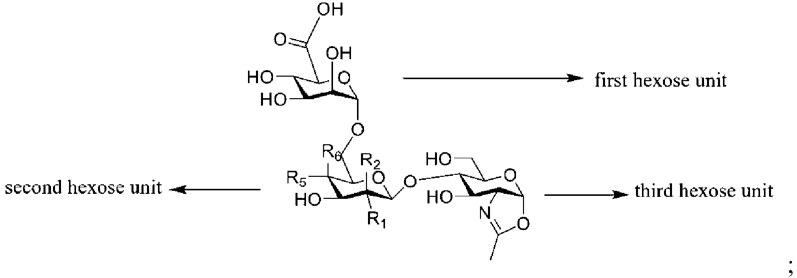 wherein, R₁, R₂, R₅, and R₆ are selected from -OH and H, and the two substituents on the same carbon atom are different.

In one embodiment, the trisaccharide structure of formula (IV) or (IV-1) attachs to an amino-containing fragment to link a drug fragment via formation of an amide bond, or is linked to a linker bearing a bioorthogonal functional group via formaton of an amide bond and is then reacted with a drug fragment bearing the aforementioned bioorthogonal functional group to form an antibody-drug conjugate.

### Pharmaceutical Composition and Pharmaceutical Formulation

Another objective of the present disclosure is to provide a pharmaceutical composition comprising a prophylactically or therapeutically effective amount of the antibody-conjugated drug of the present disclosure, and at least one pharmaceutically acceptable carrier.

The pharmaceutical composition of the present disclosure can be administered in any manner as long as it achieves the effect of preventing, alleviating, preventing, or treating a symptom in a human or an animal. For example, various suitable dosage forms can be prepared according to the administration route, particularly injections such as a lyophilized powder for injection, an injectable solution, or sterile injection powder.

The term "pharmaceutically acceptable" means that a substance, when in contact with tissues of the patient within the scope of sound medical judgment, results in no undue toxicity, irritation, allergic reaction, or the like, has a proper advantage-disadvantage ratio, and is effective for the intended use.

The term pharmaceutically acceptable carrier refers to those carrier materials that are pharmaceutically acceptable and do not interfere with the biological activity and performance of the antibody-conjugated drug. Examples of aqueous carriers include, but are not limited to, buffered saline and the like. Pharmaceutically acceptable carriers also include carrier substances that allow the composition to approximate physiological conditions, such as pH adjusters and buffering agents, and toxicity adjusters, sodium acetate, sodium chloride, potassium chloride, calcium chloride, sodium lactate, and the like.

In one embodiment, the drug/antibody ratio (DAR) of the pharmaceutical composition of the present disclosure is an integer or non-integer of 1-20, for example about 1 to about 10, about 1 to about 8, about 1 to about 6, about 1 to about 4, about 1 to about 3, about 1 to about 2.5, or about 1 to about 2. In one particular embodiment, the DAR of the antibody-conjugated drug of the present disclosure is about 2, about 4, about 6, or about 8. In another particular embodiment, the DAR of the antibody-conjugated drug of the present disclosure is about 2. In yet another particular embodiment, the DAR of the antibody-conjugated drug of the present disclosure is about 1.65.

### Treatment Method and Use

The antibody-conjugated drug of the present disclosure can be used for treating a tumor and/or an autoimmune disease. Tumors sensitive to antibody-conjugated drug treatment include those characterized by specific tumor-associated antigens or cell surface receptors, and such tumor cells can be recognized by the targeting molecule in the antibody-conjugated drug and thereby can be killed by the payload/cytotoxin in the antibody-conjugated drug.

Therefore, in yet another aspect, the present disclosure further provides use of the antibody-conjugated drug of the present disclosure or the pharmaceutical composition of the present disclosure in preparing a medicament for treating a disease, disorder, or condition selected from a tumor and an autoimmune disease.

In another aspect, the present disclosure provides the antibody-conjugated drug of the present disclosure or the pharmaceutical composition of the present disclosure for use in treating a tumor or an autoimmune disease.

In a further aspect, the present disclosure provides a method for treating a tumor or an autoimmune disease, comprising: administering to an individual in need thereof an effective amount of the antibody-conjugated drug of the present disclosure or the pharmaceutical composition of the present disclosure.

In one embodiment, the antibody-conjugated drug provided herein, formed by linking an anti-human HER2 antibody to a small molecule cytotoxin, can specifically bind to HER2 on the surface of tumor cells and thus selectively kill tumor cells expressing HER2. In another embodiment, the present disclosure provides use of the antibody-conjugated drug of the present disclosure or the pharmaceutical composition of the present disclosure in preparing a medicament for treating a disease, disorder, or condition selected from HER2-positive tumors. In one more preferred embodiment, the disease, disorder, or condition is selected from the group consisting of breast cancer, gastric cancer, lung cancer, ovarian cancer, urothelial cancer, and the like.

The dose of the antibody-conjugated drug administered to a subject can be adjusted to a considerable extent. The dose may vary depending on the particular administration route and the needs of the subject and may be determined as per the judgment of a healthcare provider.

### Beneficial Effects

By adopting the novel trisaccharide structure, which is capable of forming amide bonds, the present disclosure extends the scope of the antibody-conjugated drug acquired by the glycan chain remodeling technology. In the present disclosure, a unique trisaccharide carboxylic acid substrate (e.g., 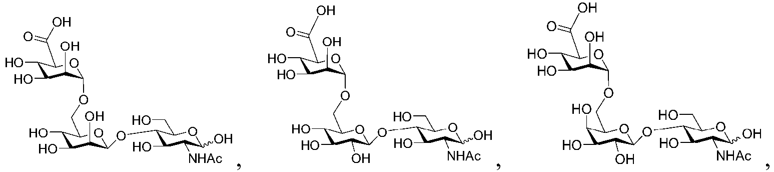 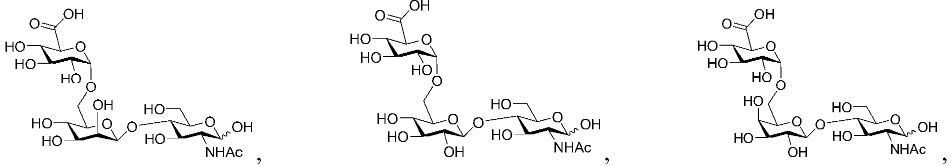 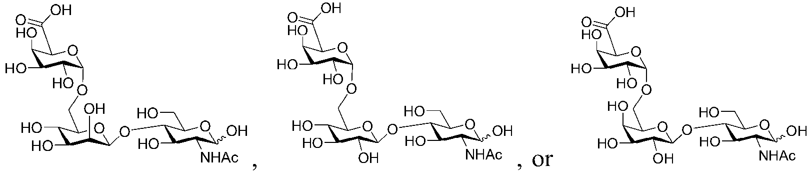 ) can be well linked to a compound containing an amine group in one step via a mild and compatible amide bonding method, thereby simplifying and accelerating the synthesis of linker-payload compounds without the production of undesirable by-products, while also expanding the sources of substrates. Therefore, in predictable circumstances, any compound having NH₂ (e.g., a compound having a structure of L'-(P)t, wherein P and t are as defined herein, except that the moiety that is -NH- linked to D in L is H₂N- in L', and L' is identical to L defined herein) can form a linker-payload D-L-(P)ₜ comprising a trisaccharide linker through a simple amide reaction via the above trisaccharide structure. The present disclosure further verified that the linker-payload can be efficiently and site-specifically conjugated to an antibody via a one-pot enzymatically catalyzed conjugation method to form a highly homogeneous antibody-conjugated drug product.

The present disclosure utilizes modular combinatorial chemistry to efficiently synthesize a series of linker-payloads on the basis of the trisaccharide structures, and verifies that the linker-payloads can achieve efficient conjugation of different payloads. Due to the introduction of the trisaccharide, the water solubility of the antibody-conjugated drug is increased to a certain extent, which provides a significant improvement in the stability of the antibody-conjugated drug product. The above linker-payloads based on trisaccharide structures are a further supplement and improvement to the one-step disaccharide-based site-specific glycan chain conjugation technology, particularly expanding the sources of substrates, improving the linkage mode between disaccharides and effector molecules, expanding the glycan chain modes of the obtained antibody-conjugated drugs, improving the water solubility of the antibody-conjugated drugs, avoiding the limitations in the prior art that a certain amount of organic solvents are still required for conjugation to promote dissolution, and the like, and further improving the conjugating efficiency of some substrates.

In addition, the present disclosure further develops a series of trisaccharide molecular libraries with novel structures and differentiated characteristics, and based on such libraries, constructs a group of antibody-conjugated drug molecular libraries in which the antibody Fc region contains structurally differentiated glycoforms. These molecular libraries exhibit unique antibody-dependent cell-mediated cytotoxicity (ADCC) activity and are significantly different from existing ADC techniques based on disaccharide linkers. The use of disaccharide linkers in conventional technologies generally results in the loss of ADCC functionality of antibody-conjugated drugs such as ADCs, while the technology of the present disclosure enables new molecular libraries to meet the requirements of diverse drug molecule design by means of the introduction of trisaccharide linkers.

In addition, compared with disaccharide linkers, trisaccharide molecules exhibit stronger hydrophilicity and better adaptability to hydrophobic load molecules. Such a feature not only improves the water solubility of ADC molecules, but also expands the application range of ADC molecule design. Therefore, adopting a trisaccharide linker with a diverse structure can conveniently construct ADC molecules with differentiated Fc glycoforms and diverse physicochemical properties, thereby better meeting the requirements of diverse ADC drug development.

### Examples

The solutions of the present disclosure are further described in detail below with reference to specific examples.

It will be appreciated that the following examples are merely examples for clearly illustrating the technical solutions of the present disclosure, and are not intended to limit the present disclosure. For those of ordinary skill in the art, other different forms of variations or modifications can be made based on the description of the present disclosure, and it is unnecessary and impossible to exhaustively list all the embodiments herein. The obvious variations or modifications introduced thereby still fall within the scope of the present disclosure. Unless otherwise indicated, devices, reagents, and materials used herein are commercially available.

### I. Preparation of trisaccharide linker

### Example 1: Preparation of first hexose unit or derivative moiety thereof, compound 1-1

Compound **1-1** was prepared using the following steps, and its structure is as follows:

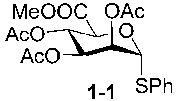

### (1) Preparation of compound 1-1b

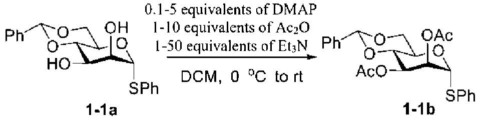

At room temperature, compound **1-la** (500 mg, 1.39 mmol, CAS No.: 159407-19-9), dichloromethane (7.5 mL), and triethylamine were sequentially added to a 25 mL single-necked flask for dissolution. The reaction system was then cooled to 0 °C, and 4-dimethylaminopyridine and acetic anhydride were added. The flask was stoppered, and the mixture was naturally warmed to room temperature and stirred for about 6 h. After TLC monitoring indicated the depletion of the starting materials, a saturated sodium bicarbonate solution (20 mL) was added to quench the reaction, and the reaction system was extracted with dichloromethane (20 mL × 3). The resulting organic phase was washed with saturated brine (50 mL), dried over anhydrous sodium sulfate, concentrated at reduced pressure, and then purified by column chromatography (eluent: EtOAc/PE = 1/100-1/10) to afford compound 1-1b (554 mg, 90% yield, colorless viscous oily liquid). ¹H NMR (400 MHz, Chloroform-d) δ 7.35-7.54 (m, 10H), 5.65 (dd, 1H, J = 1.4, 3.4 Hz), 5.64 (s, 1H), 5.48 (s, 1H), 5.46 (dd, 1H, J = 7.0, 10.4 Hz), 5.52 (td, 1H, J = 4.8, 9.8 Hz), 4.30 (dd, 1H, J = 4.9, 10.4 Hz), 4.17 (t, 1H, J = 9.8 Hz), 3.91 (t, 1H, J = 10.3 Hz), 2.21 (s, 3H), 2.09 (s, 3H). MS (ESI) of C₂₃H₂₄O₇SNa⁺ [M+Na]⁺: calculated: 467.11, measured: 467.22.

### (2) Preparation of compound 1-1c

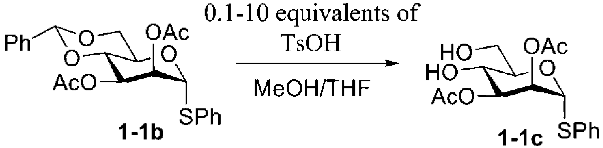

At room temperature, compound **1-1b** (554 mg, 1.25 mmol), methanol (3 mL), tetrahydrofuran (6 mL), and p-toluenesulfonic acid were sequentially added to a 25 mL single-necked flask for dissolution. The flask was stoppered, and the mixture was stirred at room temperature for about 3 h. After TLC monitoring indicated the depletion of the starting materials, a saturated sodium bicarbonate solution (20 mL) was added to quench the reaction, and the reaction system was extracted with ethyl acetate (20 mL × 3). The resulting organic phase was washed with saturated brine (50 mL), dried over anhydrous sodium sulfate, concentrated at reduced pressure, and then purified by column chromatography (eluent: EtOAc/PE = 1/100-1/1) to afford compound **1-1c** (345 mg, 78% yield, colorless viscous oily liquid). ¹H NMR (400 MHz, Chloroform-d) δ 7.30-7.49 (m, 5H), 5.50 (dd, 1H, J = 1.6, 3.4 Hz), 5.44 (d, 1H, J = 1.5 Hz), 5.18 (dd, 1H, J = 3.3, 9.9 Hz), 4.26-4.22 (m, 1H), 4.09 (t, 1H, J = 9.8 Hz), 3.91 (s, 1H), 3.90 (s, 1H), 2.11 (s, 3H), 2.10 (s, 3H). MS (ESI) of C₁₆H₂₀O₇SNa⁺ [M+Na]⁺: calculated: 379.08, measured: 379.15.

### (3) Preparation of compound 1-1e

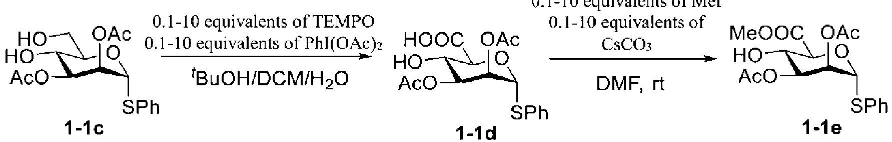

At 0 °C, compound **1-1c** (345 mg, 0.97 mmol), tert-butanol, dichloromethane, water, 2,2,6,6-tetramethylpiperidine oxide (TEMPO), and iodobenzenediacetic acid were sequentially added to a 25 mL single-necked flask for dissolution. The mixture was naturally warmed to room temperature and stirred in air for about 12 h. After TLC monitoring indicated the depletion of the starting materials, a saturated sodium thiosulfate solution (15 mL) was added to quench the reaction, and the reaction system was extracted with dichloromethane (15 mL × 3). The resulting organic phase was washed with saturated brine (30 mL), dried over anhydrous sodium sulfate, concentrated at reduced pressure, and then purified by column chromatography (eluent: EtOAc/PE = 1/100-1/1) to afford compound **1-1d** (201 mg, 56% yield, colorless viscous oily liquid). MS (ESI) of C₁₆H₁₈O₈S⁻ [M-H]⁻: calculated: 369.06, measured: 369.10.

At room temperature, compound **1-1d** (201 mg, 0.54 mmol), DMF (2 mL), cesium carbonate, and iodomethane were sequentially added to a 25 mL single-necked flask for dissolution, and the mixture was stirred at room temperature for about 2 h. After TLC monitoring indicated the depletion of the starting materials, water (15 mL) was added to quench the reaction, and the reaction system was extracted with ethyl acetate (15 mL × 3). The resulting organic phase was washed with water (30 mL × 3) and saturated brine (30 mL), dried over anhydrous sodium sulfate, concentrated at reduced pressure, and then purified by column chromatography (eluent: EtOAc/PE = 1/100-1/2) to afford compound **1-1e** (111 mg, 53% yield, white solid). ¹H NMR (400 MHz, Chloroform-d) δ 7.30-7.52 (m, 5H), 5.66 (d, 1H, *J* = 3.4 Hz), 5.35 (t, 1H, *J =* 8.3 Hz), 5.31 (t, 1H, *J* = 3.4 Hz), 4.74 (d, 1H, *J =* 8.2 Hz), 4.11 (dd, 1H, *J =* 3.4, 8.5 Hz), 3.77 (s, 3H), 2.16 (s, 3H), 2.14 (s, 3H). MS (ESI) of C₁₇H₂₀O₈SNa⁺ [M+Na]⁺: calculated: 407.08, measured: 407.16.

### (4) Preparation of compound 1-1

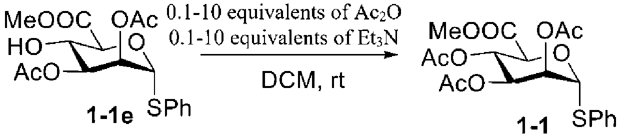

At room temperature, compound **1-1e** (182 mg, 0.47 mmol), dichloromethane (4 mL), triethylamine, and acetic anhydride were sequentially added to a 25 mL single-necked flask for dissolution. The flask was stoppered, and the mixture was stirred at room temperature for about 12 h. After TLC monitoring indicated the depletion of the starting materials, a saturated sodium thiosulfate solution (10 mL) was added to quench the reaction, and the reaction system was extracted with dichloromethane (10 mL × 3). The resulting organic phase was washed with saturated brine (30 mL), dried over anhydrous sodium sulfate, concentrated at reduced pressure, and then purified by column chromatography (eluent: EtOAc/PE = 1/100-1/4) to afford compound **1-1** (119 mg, 59% yield, colorless viscous oily liquid). ¹H NMR (400 MHz, Chloroform-d) δ 7.33-7.57 (m, 5H), 5.63 (d, 1H, *J =* 3.8 Hz), 5.50 (t, 1H, *J =* 8.2 Hz), 5.46 (t, 1H, *J* = 3.4 Hz), 5.37 (dd, 1H, *J =* 3.2, 8.5 Hz), 4.83 (d, 1H, *J* = 7.9 Hz), 3.81 (s, 3H), 2.17 (s, 3H), 2.13 (s, 3H), 2.07 (s, 3H). MS (ESI) of C₁₉H₂₂O₉SNa⁺ [M+Na]⁺: calculated: 449.09, measured: 449.17.

### Example 2: Preparation of first hexose unit or derivative moiety thereof, compound 1-2

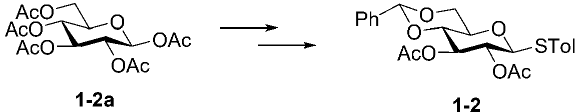

Compound **1-2a** (20 g, 51.2 mmol, CAS No.: 604-68-2) was synthesized according to a previously reported method (Beilstein Journal of Organic Chemistry, 2015, 11, 155-161) in 4 steps to ultimately afford compound **1-2** (7.1 g, 30% total yield, colorless oily liquid). ¹H NMR (400 MHz, DMSO-d) δ 7.36-7.18 (m, 9H), 5.64 (s, 1H), 5.37 (t, 1H, *J* = 9.0 Hz), 5.23 (d, 1H, *J* = 10.0 Hz), 4.83 (t, 1H, *J =* 8.9 Hz), 4.27 (dd, 1H, *J =* 3.8, 9.5 Hz), 3.84-3.73 (m, 3H), 2.30 (s, 3H), 2.04 (s, 3H), 1.97 (s, 3H). MS (ESI) of C₂₄H₂₆O₇SNa⁺ [M+Na]⁺: calculated: 481.13, measured: 481.16.

### Example 3: Preparation of first hexose unit or derivative moiety thereof, compound 1-3

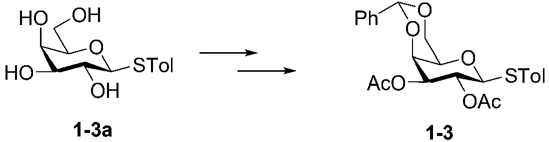

Compound **1-3a** (15 g, 52.3 mmol, CAS No.: 28244-98-6) was synthesized according to a previously reported method (Synlett, 2009, 4, 603-606) in 2 steps to ultimately afford compound **1-3** (11.5 g, 48% total yield, colorless oily liquid). ¹H NMR (400 MHz, DMSO-d) δ 7.40-7.09 (m, 9H), 5.55 (s, 1H), 5.18-5.15 (m, 1H), 5.05-4.98 (m, 2H), 4.36 (d, 1H, *J =* 3.4 Hz), 4.10-4.01 (m, 2H), 3.84 (s, 1H), 2.28 (s, 3H), 1.98 (s, 3H), 1.92 (s, 3H). MS (ESI) of C₂₄H₂₆O₇SNa⁺ [M+Na]⁺: calculated: 481.13, measured: 481.18.

### Example 4: Preparation of first hexose unit or derivative moiety thereof, compound 1-4

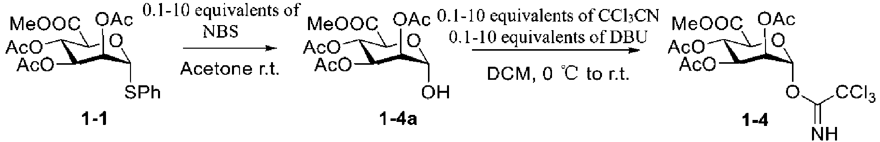

At room temperature, compound **1-1** (6 g, 14.1 mmol), acetone (30 mL), and *N-*bromosuccinimide were sequentially added to a 250 mL single-necked flask, and the mixture was stirred at room temperature for about 1 h. After TLC monitoring indicated the depletion of the starting materials, a saturated sodium thiosulfate solution (10 mL) was added to quench the reaction, and excess acetone was removed by concentration at reduced pressure. The reaction system was extracted with ethyl acetate (50 mL × 3). The resulting organic phase was washed with saturated brine (150 mL), dried over anhydrous sodium sulfate, concentrated at reduced pressure, and then purified by column chromatography (eluent: EtOAc/PE = 1/10-1/2) to afford compound **1-4a** (3.8 g, 80% yield, colorless oily liquid). MS (ESI) of C₁₃H₂₂NO₁₀⁺ [M+NH₄]⁺: calculated: 352.12, measured: 352.18.

At 0 °C, compound **1-4a** (3.8 g, 11.3 mmol), anhydrous dichloromethane (50 mL), and trichloroacetonitrile were added to a 250 mL single-necked flask, and the mixture was stirred at 0 °C for half an hour. Subsequently, 1,8-diazabicyclo[5.4.0]undec-7-ene was added dropwise into the reaction mixture. The resulting mixture was warmed to room temperature, stirred for 2 h, concentrated at reduced pressure, and then purified by column chromatography (eluent: EtOAc/PE = 1/10-1/1) to afford compound **1-4** (2.5 g, 3.4% total yield, colorless oily liquid). ¹H NMR (400 MHz, DMSO-d) δ 8.83 (s, 1H), 6.40 (d, 1H, *J* = 4.0 Hz), 5.50-5.31 (m, 3H), 4.51 (d, 1H, *J* = 8.0 Hz), 3.86 (s, 3H), 2.09 (s, 3H), 2.05 (s, 3H), 2.02 (s, 3H). MS (ESI) of C₁₃H₁₈Cl₃NO₁₀⁺ [M+H]⁺: calculated: 478.01, measured: 478.05.

### Example 5: Preparation of disaccharide substrate, compound 2-1

Compound **2-1** was prepared using the following steps, and its structure is as follows:

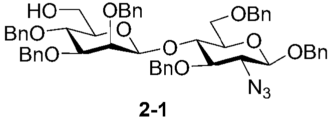

### Preparation of compound 2-1

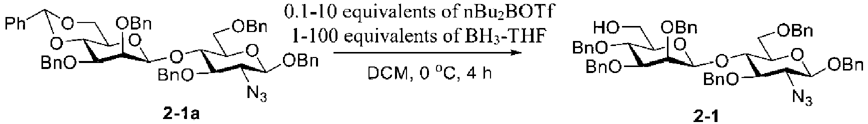

At 0 °C, compound **2-la** (500 mg, 0.55 mmol), anhydrous dichloromethane (10 mL), dibutylboron trifluoromethanesulfonate, and borane-tetrahydrofuran were sequentially added to a 25 mL Schlenk reaction flask for dissolution in a nitrogen atmosphere, and the mixture was stirred at 0 °C for about 4 h. After TLC monitoring indicated the depletion of the starting materials, water was added slowly into the reaction system at 0 °C to quench the reaction until no bubbles were produced. The reaction system was extracted with ethyl acetate (20 mL × 3). The resulting organic phase was washed successively with a saturated sodium bicarbonate solution (50 mL), a saturated ammonium chloride solution (50 mL), a 1 M sodium hydroxide solution (50 mL), and saturated brine (50 mL), dried over anhydrous sodium sulfate, concentrated at reduced pressure, and then purified by column chromatography (eluent: EtOAc/PE = 1/100-1/5) to afford compound **2-1** (335 mg, 67% yield, colorless viscous oily liquid). ¹H NMR (400 MHz, Chloroform-d) δ 7.30-7.45 (m, 30H), 5.10 (d, 1H, *J =* 11.8 Hz), 4.97 (d, 1H, *J* = 12.0 Hz), 4.90 (d, 1H, *J=* 10.9 Hz), 4.88 (d, 1H, *J* = 11.8 Hz), 4.83 (d, 1H, *J =* 11.8 Hz), 4.73 (d, 1H, *J =* 12.0 Hz), 4.69 (d, 1H, *J =* 10.8 Hz), 4.67 (d, 1H, *J =* 12.1 Hz), 4.61 (d, 1H, *J* = 10.9 Hz), 4.55 (s, 2H), 4.50 (d, 1H, *J=* 11.6 Hz), 4.48 (s, 1H), 4.35 (d, 1H, *J* = 8.1 Hz), 3.95 (t, 1H, *J* = 9.3 Hz), 3.83 (t, 1H, *J* = 9.5 Hz), 3.76 (d, 1H, *J* = 2.9 Hz), 3.72 (dd, 1H, *J* = 2.3, 11.2 Hz), 3.64-3.68 (m, 2H), 3.51 (t, 1H, *J=* 9.9 Hz), 3.34-3.40 (m, 4H), 3.13-3.17 (m, 1H). MS (ESI) of C₃₄H₃₇N₃0₁₀Na⁺ [M+Na]⁺: calculated: 930.39, measured: 930.55.

### Example 6: Preparation of disaccharide substrate, compound 2-2

Compound **2-2** was prepared using the following steps, and its structure is as follows:

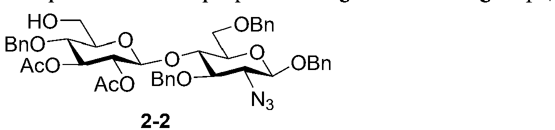

### (1) Synthesis of compound 2-2b

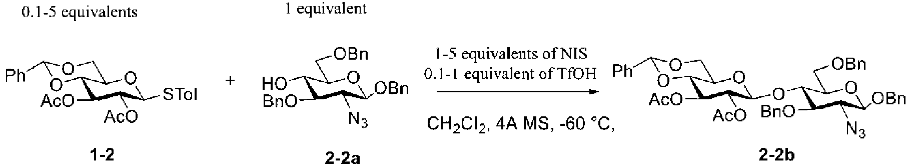

At room temperature, compound **2-2a** (5 g, 10.5 mmol, CAS No.: 342640-42-0), compound **1-2** (equivalent to 0.1-5 equivalents of **2-2a**), 4A molecular sieves, and dichloromethane (100 mL) were sequentially added to a 250 mL three-necked flask, and the mixture was stirred at room temperature for about 3 h. The reaction system was cooled to -60 °C, *N*-iodosuccinimide and trifluoromethanesulfonic acid were added to the reaction system, and the mixture was stirred at the temperature for 2 h. After TLC monitoring indicated the depletion of the starting materials, triethylamine was added to quench the reaction, and the mixture was concentrated at reduced pressure and then purified by column chromatography (eluent: EtOAc/PE = 1/10-1/5) to afford compound **2-2b** (6.6 g, 82% yield, colorless viscous oily liquid). ¹H NMR (400 MHz, DMSO-d) δ 7.43-7.29 (m, 20H), 5.60 (s, 1H), 5.22-5.18 (m, 1H), 4.89 (d, 1H, *J =* 10.8 Hz), 4.84-4.81 (m, 3H), 4.66 (d, 1H, *J =* 12.3 Hz), 4.63 (d, 1H, *J =* 12.0 Hz), 4.62 (d, 1H, *J* = 11.0 Hz), 4.58-4.54 (m, 2H), 4.06-4.02 (m, 1H), 3.84-3.75 (m, 3H), 3.68 (dd, 1H, *J* = 4.5, 11.3 Hz), 3.58-3.43 (m, 5H), 1.98 (s, 3H), 1.97 (s, 3H). MS (ESI) of C₄₄H₃₁N₄O₁₂⁺ [M+NH₄]⁺: calculated: 827.35, measured: 827.27.

### (2) Synthesis of compound 2-2

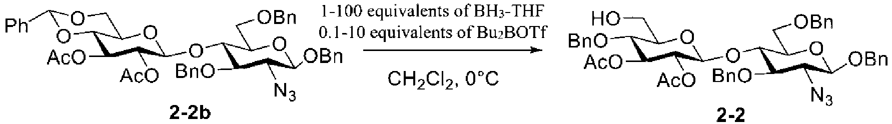

At 0 °C, compound **2-2b** (6.6 g, 8.15 mmol), a borane-tetrahydrofuran complex, and dibutylboron trifluoromethanesulfonate were sequentially added to a 250 mL Schlenk reaction flask in a nitrogen atmosphere. After dissolution, the mixture was stirred at 0 °C for about 40 min. After TLC monitoring indicated the depletion of the starting materials, triethylamine (6 mL) was added to the reaction system, methanol (6 mL) was slowly added, and the resulting mixture was concentrated with methanol three times at reduced pressure and purified by column chromatography (eluent: EtOAc/PE = 1/5-1/2) to afford compound **2-2** (2.4 g, 36% yield, colorless viscous oily liquid). ¹H NMR (400 MHz, DMSO-d) δ 7.47-7.25 (m, 20H), 5.09 (t, 1H, *J=* 9.4 Hz), 4.97 (d, 1H, *J* = 11.1 Hz), 4.82 (d, 1H, *J* = 12.0 Hz), 4.75 (d, 1H, *J* = 8.0 Hz), 4.70-4.60 (m, 6H), 4.55-4.49 (m, 3H), 3.85 (t, 1H, *J =* 8.9 Hz), 3.76-3.63 (m, 4H), 3.57-3.52 (m, 2H), 3.50-3.46 (m, 2H), 3.34-3.30 (m, 1H), 1.92 (s, 3H), 1.90 (s, 3H). MS (ESI) of C₄₄H₄₉N₃O₁₂Na⁺ [M+Na]⁺: calculated: 834.32, measured: 834.34.

### Example 7: Preparation of disaccharide substrate, compound 2-3

Compound **2-3** was prepared using the following steps, and its structure is as follows:

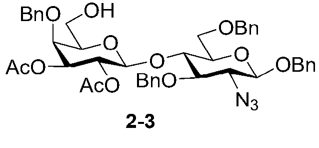

### (1) Preparation of compound 2-3a

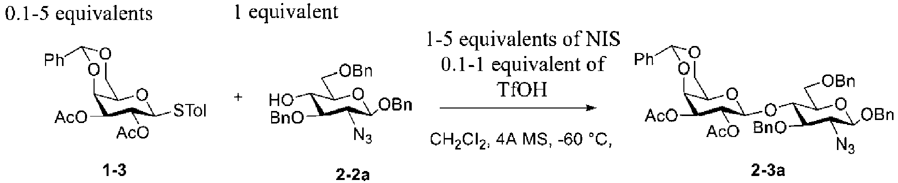

At room temperature, compound **2-2a** (5 g, 10.5 mmol, CAS No.: 342640-42-0), compound **1-3** (equivalent to 0.1-5 equivalents of **2-2a**), 4A molecular sieves, and dichloromethane (100 mL) were sequentially added to a 250 mL three-necked flask, and the mixture was stirred at room temperature for about 3 h. The reaction system was cooled to -60 °C, N-iodosuccinimide and trifluoromethanesulfonic acid were added to the reaction system, and the mixture was stirred at the temperature for 2 h. After TLC monitoring indicated the depletion of the starting materials, triethylamine was added to quench the reaction, and the mixture was concentrated at reduced pressure and then purified by column chromatography (eluent: EtOAc/PE = 1/10-1/5) to afford compound **2-3a** (6.6 g, 82% yield, colorless viscous oily liquid). ¹H NMR (400 MHz, DMSO-d) δ 7.41-7.17 (m, 20H), 5.58 (s, 1H), 5.07 (d, 1H, *J =* 11.0 Hz), 5.03-4.95 (m, 2H), 4.78 (d, 1H, *J* = 12.1 Hz), 4.72 (d, 1H, *J* = 7.7 Hz), 4.62 (d, 1H, *J=* 12.1 Hz), 4.60 (d, 1H, *J =* 10.9 Hz), 4.72 (d, 1H, *J=* 12.1 Hz), 4.51 (d, 1H, *J* = 7.7 Hz), 4.50 (d, 1H, *J =* 12.2 Hz), 4.34 (d, 1H, *J =* 3.4 Hz), 4.04-3.94 (m, 2H), 3.79 (d, 1H, *J =* 9.5 Hz), 3.75-3.64 (m, 2H), 3.55-3.49 (m, 2H), 3.46-3.43 (m, 2H), 1.96 (s, 3H), 1.93 (s, 3H). MS (ESI) of C₄₄H₃₁N₄O₁₂⁺ [M+NH₄]⁺: calculated: 827.35, measured: 827.31.

### (2) Preparation of compound 2-3

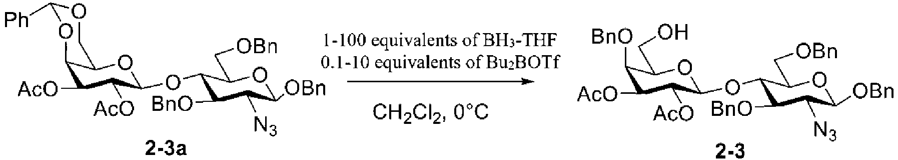

At 0 °C, compound **2-3a** (6.6 g, 8.15 mmol), a borane-tetrahydrofuran complex, and dibutylboron trifluoromethanesulfonate were sequentially added to a 250 mL Schlenk reaction flask in a nitrogen atmosphere. After dissolution, the mixture was stirred at 0 °C for about 40 min. After TLC monitoring indicated the depletion of the starting materials, triethylamine (6 mL) was added to the reaction system, methanol (6 mL) was slowly added, and the resulting mixture was concentrated with methanol three times at reduced pressure and purified by column chromatography (eluent: EtOAc/PE = 1/5-1/2) to afford compound **2-3** (2.4 g, 36% yield, colorless viscous oily liquid). ¹H NMR (400 MHz, DMSO-d) δ 7.39-7.20 (m, 20H), 5.06 (dd, 1H, *J =* 7.9, 10.4 Hz), 4.93 (d, 1H, *J* = 10.5 Hz), 4.89 (d, 1H, *J=* 10.2 Hz), 4.88 (d, 1H, *J* = 10.4 Hz), 4.81 (d, 1H, *J =* 12.1 Hz), 4.67-4.58 (m, 5H), 4.54-4.51 (m, 3H), 3.99 (d, 1H, *J* = 3.2 Hz), 3.81 (t, 1H, *J* = 9.2 Hz), 3.75-3.65 (m, 2H), 3.54-3.47 (m, 4H), 3.44-3.39 (m, 2H), 1.98 (s, 3H), 1.96 (s, 3H). MS (ESI) of C₄₄H₅₃N₄O₁₂⁺ [M+NH₄]⁺: calculated: 829.37, measured: 829.34.

### Example 8: Preparation of trisaccharide substrate, compound 3-1

Compound **3-1** was prepared using the following steps, and its structure is as follows:

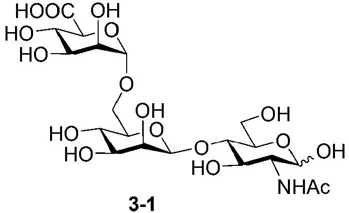

### (1) Preparation of compound 3-1a

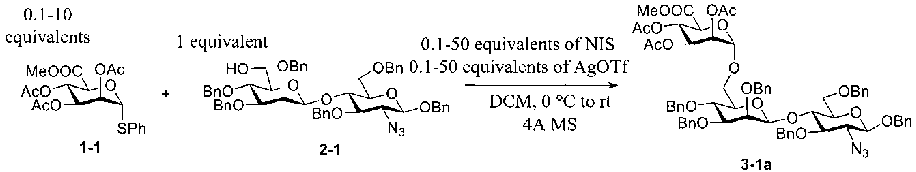

In a vacuum condition produced by an oil pump, a 25 mL Schlenk reaction flask was baked with a heat gun for 5 min. After cooling, activated molecular sieves were added to the system, and the flask was baked for another 5 min. The flask was evacuated and purged with nitrogen three times, and certain amounts of compound **1-1** (0.1 to 10 equivalents relative to compound **2-1**) and compound **2-1** (1.0 equivalent, 90.6 mg, 0.10 mmol) dissolved in anhydrous dichloromethane (6 mL) were added to the system in a nitrogen atmosphere. The reaction system was cooled to 0 °C and stirred for a period of time, and certain amounts of *N-*iodosuccinimide and silver trifluoromethanesulfonate were added to the system. The mixture was naturally warmed to room temperature and stirred until the reaction was completed. A saturated sodium bicarbonate solution (10 mL) was added to quench the reaction, and the reaction system was extracted with ethyl acetate (10 mL × 3). The resulting organic phase was washed with a saturated sodium thiosulfate solution (30 mL) and saturated brine (30 mL), dried over anhydrous sodium sulfate, concentrated at reduced pressure, and then purified by column chromatography (eluent: EtOAc/PE = 1/100-1/9) to afford compound **3-1a** (64 mg, 52% yield, colorless viscous oily liquid). ¹H NMR (400 MHz, Chloroform-d) δ 7.25-7.43 (m, 30H), 5.36-5.38 (m, 2H), 5.20 (t, 1H, *J =* 2.4 Hz), 5.02 (d, 1H, *J* = 11.2 Hz), 4.99 (d, 1H, *J =* 2.1 Hz), 4.97 (d, 2H, *J=* 11.5 Hz), 4.85 (s, 2H), 4.76 (d, 1H, *J* = 9.5 Hz), 4.73 (d, 1H, *J =* 10.1 Hz), 4.68 (d, 1H, *J* = 12.2 Hz), 4.61 (d, 1H, *J* = 11.1 Hz), 4.52 (d, 1H, *J* = 12.2 Hz), 4.47 (d, 1H, *J* = 9.5 Hz), 4.46 (s, 1H), 4.45 (d, 1H, *J* = 10.0 Hz), 4.36 (d, 1H, *J =* 8.3 Hz), 4.33-4.36 (m, 1H), 4.04 (t, 1H, *J* = 9.3 Hz), 3.77 (d, 1H, *J =* 2.8 Hz), 3.58-3.72 (m, 7H), 3.54 (d, 1H, *J =* 1.7, 9.8 Hz), 3.29-3.47 (m, 5H), 2.11 (s, 3H), 2.05 (s, 3H), 1.99 (s, 3H). MS (ESI) of C₆₇H₇₃N₃O₁₉Na⁺ [M+Na]⁺: calculated: 1246.47, measured: 1246.61.

### (2) Preparation of compound 3-1b

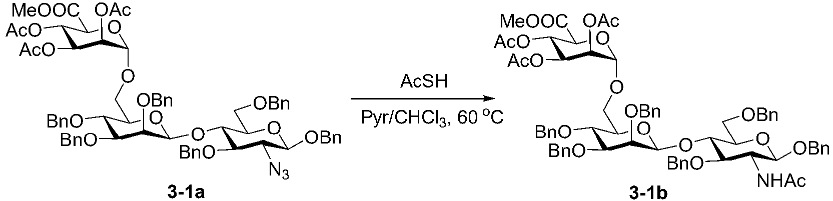

At room temperature, compound **3-1a** (150 mg, 0.12 mmol), chloroform, pyridine, and AcSH were sequentially added to a 10 mL Schlenk reaction flask for dissolution. The flask was stoppered, and the system was stirred at 60 °C until HPLC indicated a substantial completion of the reaction, which took about 18 h. After concentration at reduced pressure to remove most of the solvent, ethyl acetate (20 mL) was added to the system. The mixture was washed successively with a saturated sodium bicarbonate solution (20 mL), 1 M hydrochloric acid (20 mL × 4), a saturated sodium bicarbonate solution (20 mL), and saturated brine (20 mL). The organic phase was dried over anhydrous sodium sulfate, concentrated at reduced pressure, and then purified by column chromatography (eluent: EtOAc/PE = 1/10-1/1) to afford compound **3-1b** (72 mg, 47% yield, white solid). ¹H NMR (400 MHz, Chloroform-d) δ 7.15-7.32 (m, 30H), 5.25-5.27 (m, 2H), 5.16 (t, 1H, J = 2.4 Hz), 4.79-4.88 (m, 4H), 4.74 (s, 2H), 4.73 (d, 1H, J = 11.4 Hz), 4.55 (d, 1H, J = 11.4 Hz), 4.48-4.51 (m, 4H), 4.41 (d, 1H, J = 11.8 Hz), 4.38 (d, 1H, J = 11.8 Hz), 4.35 (d, 1H, J = 11.5 Hz), 4.24-4.28 (m, 1H), 3.90-3.92 (m, 2H), 3.59-3. 75 (m, 9H), 3.57 (s, 3H), 3.24-3. 31 (m, 2H), 1.97 (s, 3H), 1.89 (s, 3H), 1.85 (s, 3H), 1.67 (s, 3H). MS (ESI) of C₆₉H₇₇NO₂₀Na⁺ [M+Na]⁺: calculated 1262.49, measured 1262.67.

### (3) Preparation of compound 3-1c

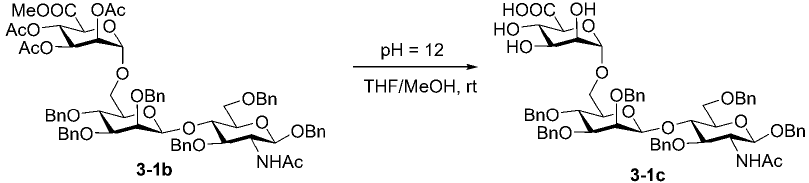

At room temperature, compound **3-1b** (72 mg, 0.06 mmol), methanol (2 mL), and sodium methoxide were sequentially added to a 25 mL single-necked flask. The reaction system was adjusted to pH 11-12 and stirred at room temperature for 4 h until HPLC indicated the completion of the reaction. The reaction system was adjusted to pH 6-7 and extracted with ethyl acetate (10 mL × 3). The resulting organic phase was washed with water (30 mL) and saturated brine (30 mL), dried over anhydrous sodium sulfate, filtered, and concentrated at reduced pressure. The reaction mixture was purified by semi-preparative HPLC to afford compound **3-1c** (42 mg, 66% yield, white solid). ¹H NMR (400 MHz, Chloroform-d) δ 7.21-7.41 (m, 30H), 4.94 (d, 1H, *J =* 11.0 Hz), 4.73-4.91 (m, 9H), 4.65 (d, 1H, *J =* 12.0 Hz), 4.58 (d, 1H, *J =* 10.4 Hz), 4.55 (d, 1H, *J =* 11.4 Hz), 4.51 (s, 1H), 4.50 (s, 1H), 4.46 (s, 1H), 4.45 (d, 1H, *J* = 11.9 Hz), 4.24 (d, 1H, *J =* 9.6 Hz), 3.66-4.03 (m, 11H), 3.59-3.62 (m, 1H), 3.39 (dd, 1H, *J =* 2.8, 9.2 Hz), 3.34 (dd, 1H, *J=* 4.8, 9.9 Hz), 1.66 (s, 3H). MS (ESI) of C₆₂H₇₀NO₁₇⁺ [M+H]⁺: calculated 1100.49, measured 1100.62.

### (4) Preparation of compound 3-1

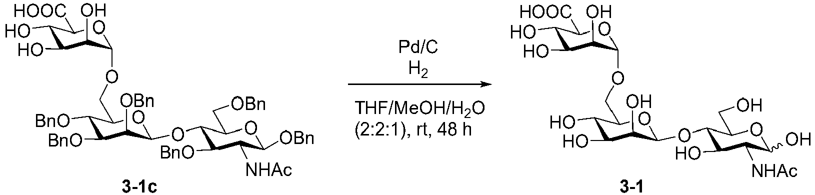

At room temperature, compound **3-1c** (14 mg, 0.01 mmol), tetrahydrofuran, methanol, and a certain amount of palladium on activated carbon catalyst were sequentially added to a 25 mL single-necked flask, and the mixture was stirred in a hydrogen atmosphere until LC-MS indicated the depletion of the starting materials. The reaction system was filtered and concentrated at reduced pressure, and the residue was dried with an oil pump to afford compound **3-1** (5 mg, 70% yield, white solid). MS (ESI) of C₂₀H₃₄NO₁₇⁺ [M+H]⁺: calculated 560.18, measured 560.30.

### Example 9: Preparation of trisaccharide substrate, compound 3-2

Compound **3-2** was prepared using the following steps, and its structure is as follows:

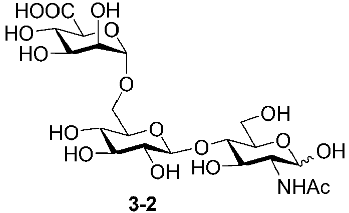

### (1) Preparation of compound 3-2a

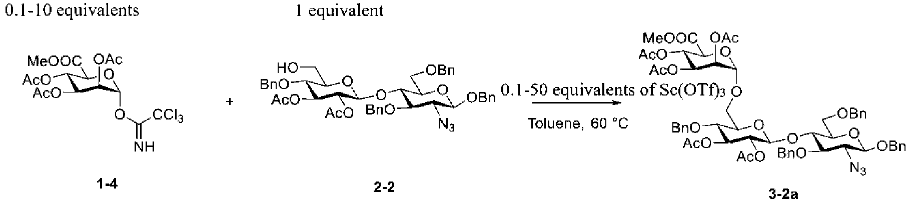

At room temperature, compound **1-4** (0.1-10 equivalents relative to compound **2-2**), compound **2-2** (1.2 g, 1.48 mmol), and toluene (100 mL) were sequentially added to a 250 mL three-necked flask in a nitrogen atmosphere. After the mixture was dissolved, scandium trifluoromethanesulfonate was added to the system, and the resulting mixture was heated to 60 °C and stirred for about 2 h. After TLC monitoring indicated the depletion of the starting materials, water (100 mL) was added to quench the reaction, and the mixture was extracted with dichloromethane (100 mL × 2). The organic phase was collected, dried over anhydrous sodium sulfate, concentrated at reduced pressure, and then purified by column chromatography (eluent: EtOAc/PE = 1/50-1/5) to afford compound **3-2a** (0.9 g, 55% yield, colorless viscous oily liquid). ¹H NMR (400 MHz, DMSO-d) δ 7.36-7.21 (m, 20H), 5.17-5.06 (m, 4H), 4.96 (d, 1H, *J* = 2.2 Hz), 4.81 (d, 1H, *J =* 12.0 Hz), 4.76 (d, 1H, *J =* 12.1 Hz), 4.71-4.65 (m, 3H), 4.61 (d, 1H, *J =* 12.3 Hz), 4.57 (d, 1H, *J =* 12.1 Hz), 4.55 (d, 1H, *J =* 11.3 Hz), 4.50-4.45 (m, 2H), 4.28 (d, 1H, *J* = 9.0 Hz), 3.80 (t, 1H, *J =* 9.0 Hz), 3.70-3.57 (m, 6H), 3.53-3.50 (m, 5H), 3.42-3.38 (m, 2H), 1.97 (s, 3H), 1.93 (s, 3H), 1.88 (s, 3H), 1.88 (s, 3H), 1.87 (s, 3H). MS (ESI) of C₅₇H₆₉N₄O₂₁⁺ [M+NH₄]⁺: calculated: 1145.45, measured: 1145.41.

### (2) Preparation of compound 3-2b

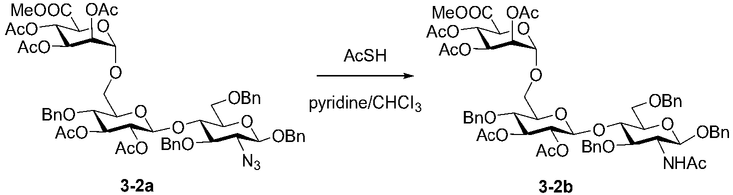

At room temperature, compound **3-2a** (900 mg, 0.798 mmol), chloroform, pyridine, and AcSH were sequentially added to a 25 mL Schlenk reaction flask for dissolution. The system was stirred at room temperature for 18 h. After TLC monitoring indicated the depletion of the starting materials, the reaction mixture was concentrated at reduced pressure and then purified by semi-preparative HPLC (mobile phase: H₂O/MeCN = 70%) to afford compound **3-2b** (550 mg, 60% yield, white solid). ¹H NMR (400 MHz, DMSO-d) δ 7.81 (d, 1H, *J =* 8.9 Hz), 7.38-7.24 (m, 20H), 5.18-5.10 (m, 4H), 4.96 (d, 1H, *J =* 2.4 Hz), 4.78-4.68 (m, 4H), 4.62-4.48 (m, 6H), 4.28 (d, 1H, *J =* 8.3 Hz), 3.81 (t, 1H, *J =* 9.0 Hz), 3.75-3.65 (m, 6H), 3.60-3.51 (m, 6H), 3.47-3.43 (m, 1H), 1.98 (s, 3H), 1.97 (s, 3H), 1.92 (s, 9H), 1.73 (s, 3H). MS (ESI) of C₅₉H₇₀NO₂₂⁺ [M+H]⁺: calculated: 1144.44, measured: 1144.46.

### (3) Preparation of compound 3-2c

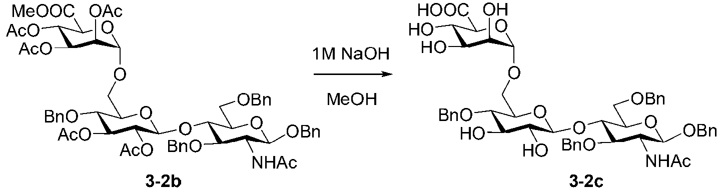

At room temperature, compound **3-2b** (550 mg, 0.481 mmol) and methanol (10 mL) were added to a 25 mL single-necked flask. After dissolution, a 1 M sodium hydroxide solution (1 mL) was slowly added (10 min), and the resulting mixture was stirred at room temperature for 2 h until HPLC indicated the completion of the reaction. Acetic acid (1 mL) was added to the reaction system to quench the reaction, and the reaction mixture was concentrated at reduced pressure to afford a crude compound **3-2c** (300 mg, 68% yield, colorless oily liquid). ¹H NMR (400 MHz, DMSO-d) δ 7.99 (d, 1H, *J =* 8.8 Hz), 7.37-7.23 (m, 20H), 5.43-5.39 (m, 1H), 4.90 (d, 1H, *J =* 10.7 Hz), 4.84 (d, 1H, *J* = 11.4 Hz), 4.76 (d, 1H, *J =* 11.4 Hz), 4.74 (s, 1H), 4.67-4.66 (m, 1H), 4.60-4.49 (m, 7H), 4.42 (d, 1H, *J =* 7.8 Hz), 3.90-3.80 (m, 3H), 3.72-3.61 (m, 3H), 3.58-3.49 (m, 6H), 3.37-3.35 (m, 2H), 3.15-3.14 (m, 1H), 3.08-3.04 (m, 1H), 1.77 (s, 3H). MS (ESI) of C₄₈H₅₈NO₁₇⁺ [M+H]⁺: calculated: 920.37, measured: 920.24.

### (4) Preparation of compound 3-2

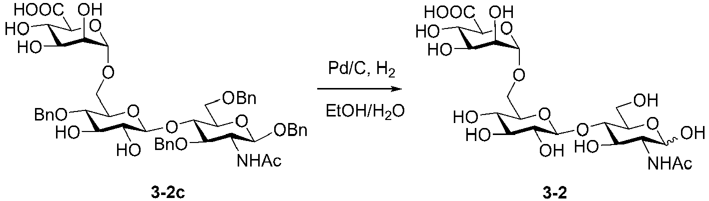

At room temperature, compound **3-2c** (150 mg, 0.163 mmol), ethanol (10 mL), and water (5 mL) were sequentially added to a 25 mL single-necked flask, and then Pd/C was added. The mixture was stirred in a hydrogen atmosphere for 21 h until LC-MS indicated the depletion of the starting materials. The reaction system was filtered and concentrated at reduced pressure, and the residue was dissolved in water and then lyophilized to afford compound **3-2** (72 mg, 79% yield, white solid). MS (ESI) of C₂₀H₃₄NO₁₇⁺ [M+H]⁺: calculated 560.18, measured 560.15.

### Example 10: Preparation of trisaccharide substrate, compound 3-3

Compound **3-3** was prepared using the following steps, and its structure is as follows:

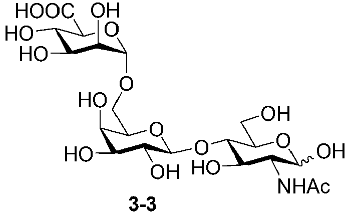

### (1) Preparation of compound 3-3a

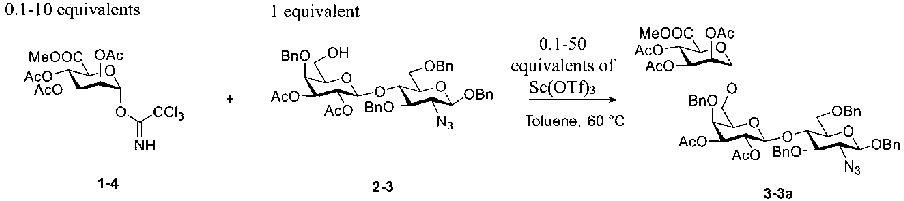

At room temperature, compound **1-4** (0.1-10 equivalents relative to compound **2-3**), compound **2-3** (1.2 g, 1.48 mmol), and toluene (100 mL) were sequentially added to a 250 mL three-necked flask in a nitrogen atmosphere. After the mixture was dissolved, scandium trifluoromethanesulfonate was added to the system, and the resulting mixture was heated to 60 °C and stirred for about 2 h. After TLC monitoring indicated the depletion of the starting materials, water (100 mL) was added to quench the reaction, and the mixture was extracted with dichloromethane (100 mL × 2). The organic phase was collected, dried over anhydrous sodium sulfate, concentrated at reduced pressure, and then purified by column chromatography (eluent: EtOAc/PE = 1/50-1/5) to afford compound **3-3a** (0.9 g, 55% yield, colorless viscous oily liquid). ¹H NMR (400 MHz, DMSO-d) δ 7.39-7.19 (m, 20H), 5.17-5.15 (m, 2H), 5.09-5.05 (m, 2H), 5.00 (dd, 1H, *J =* 3.0 Hz, 10.4 Hz), 4.87 (d, 1H, *J =* 10.7 Hz), 4.81 (d, 1H, *J =* 12.0 Hz), 4.77 (d, 1H, *J =* 2.4 Hz), 4.69 (d, 1H, *J=* 11.6 Hz), 4.65 (d, 1H, *J =* 12.2 Hz), 4.64 (d, 1H, *J* = 10.9 Hz), 4.63 (d, 1H, *J =* 3.3 Hz), 4.60-4.49 (m, 4H), 4.34-4.32 (m, 1H), 3.93 (d, 1H, *J =* 3.6 Hz), 3.83 (t, 1H, *J* = 9.0 Hz), 3.78 (t, 1H, *J =* 6.6 Hz), 3.74-3.71 (m, 1H), 3.65 (dd, 1H, *J =* 4.3 Hz, 11.2 Hz), 3.60-3.58 (m, 4H), 3.56-3.51 (m, 2H), 3.45-3.41 (m, 2H), 2.10 (s, 3H), 2.02 (s, 3H), 2.01 (s, 3H), 1.95 (s, 3H), 1.94 (s, 3H). MS (ESI) of C₅₇H₆₉N₄O₂₁⁺ [M+NH₄]⁺: calculated: 1145.45, measured: 1145.33.

### (2) Preparation of compound 3-3b

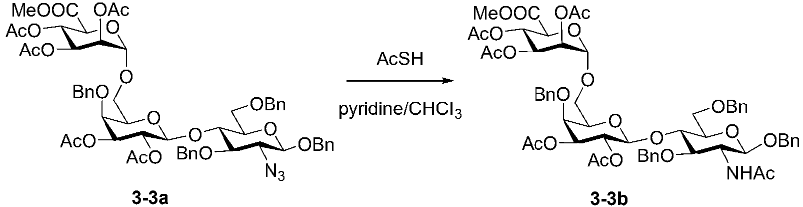

At room temperature, compound **3-3a** (900 mg, 0.798 mmol), chloroform, pyridine, and AcSH were sequentially added to a 25 mL Schlenk reaction flask for dissolution. The system was stirred at room temperature for 18 h. After TLC monitoring indicated the depletion of the starting materials, the mixture was concentrated at reduced pressure and then purified by column chromatography (eluent: EtOAc/PE = 1/4-2/3) to afford compound **3-3b** (550 mg, 60% yield, white solid). ¹H NMR (400 MHz, DMSO-d) δ 7.90 (d, 1H, *J =* 9.0 Hz), 7.41-7.18 (m, 20H), 5.16-5.14 (m, 2H), 5.06-5.03 (m, 2H), 4.78-4.71 (m, 3H), 4.68-4.63 (m, 3H), 4.56-4.51 (m, 2H), 4.49-4.42 (m, 2H), 4.30 (t, 1H, *J* = 4.2 Hz), 3.92 (d, 1H, *J =* 2.8 Hz), 3.80-3.74 (m, 2H), 3.72-3.67 (m, 3H), 3.61 (s, 3H), 3.56-3.53 (m, 2H), 3.50-3.40 (m, 4H), 2.10 (s, 3H), 2.03 (s, 3H), 2.01 (s, 3H), 1.97 (s, 3H), 1.94 (s, 3H), 1.80 (s, 3H). MS (ESI) of C₅₉H₇₀NO₂₂⁺ [M+H]⁺: calculated: 1144.44, measured: 1144.42.

### (3) Preparation of compound 3-3c

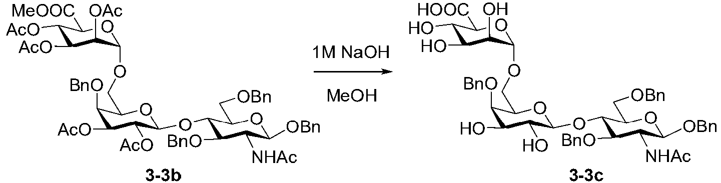

At room temperature, compound **3-3b** (550 mg, 0.481 mmol) and methanol (10 mL) were added to a 25 mL single-necked flask. After dissolution, a 1 M sodium hydroxide solution (1 mL) was slowly added (10 min), and the resulting mixture was stirred at room temperature for 2 h until HPLC indicated the completion of the reaction. Acetic acid (1 mL) was added to the reaction system to quench the reaction, and the reaction mixture was concentrated at reduced pressure to afford a crude compound **3-3c** (300 mg, 68% yield, colorless oily liquid). ¹H NMR (400 MHz, DMSO-d) δ 8.00 (d, 1H, *J =* 8.8 Hz), 7.36-7.17 (m, 20H), 5.17-5.14 (m, 2H), 4.92 (d, 1H, *J =* 11.4 Hz), 4.83 (d, 1H, *J* = 10.9 Hz), 4.76 (d, 1H, *J =* 12.5 Hz), 4.71 (d, 1H, *J =* 4.3 Hz), 4.64-4.60 (m, 2H), 4.57-4.44 (m, 6H), 4.35-4.33 (m, 1H), 4.87-4.86 (m, 2H), 3.80 (t, 1H, *J =* 9.1 Hz), 3.68-3.56 (m, 4H), 3.52-3.40 (m, 9H), 3.37-3.36 (m, 1H), 1.81 (s, 3H). MS (ESI) of C₄₈H₅₈NO₁₇⁺ [M+H]⁺: calculated: 920.37, measured: 920.34.

### (4) Preparation of compound 3-3

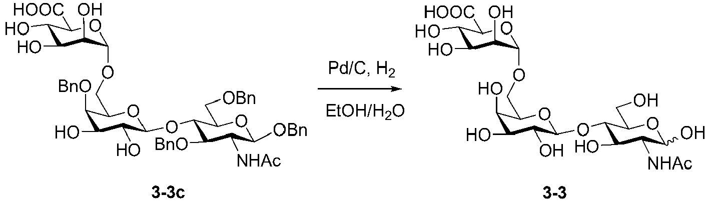

At room temperature, compound **3-3c** (150 mg, 0.163 mmol), ethanol (10 mL), and water (5 mL) were sequentially added to a 25 mL single-necked flask, and then Pd/C was added. The mixture was stirred in a hydrogen atmosphere for 21 h until LC-MS indicated the depletion of the starting materials. The reaction system was filtered and concentrated at reduced pressure, and the residue was dissolved in water and then lyophilized to afford compound **3-3** (73 mg, 80% yield, white solid). MS (ESI) of C₂₀H₃₄NO₁₇⁺ [M+H]⁺: calculated 560.18, measured 560.17.

### II. Preparation of linker-payload (abbreviated as LP) compound

### Example 11: Preparation of linker-payload compound 4-1

The structure of linker-payload compound **4-1** is as follows:

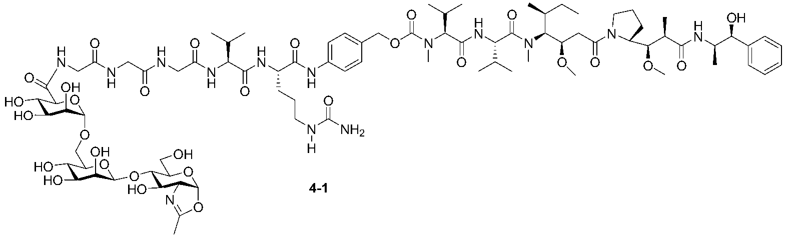

### (1) Preparation of compound 4-1b

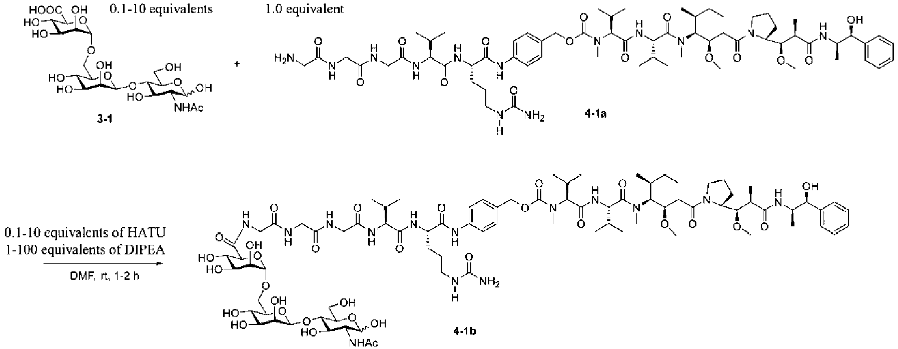

At room temperature, a certain amount of compound **3-1** (0.1-10 equivalents), compound **4-1a** (1.0 equivalent, 10.0 mg, 0.0077 mmol, CAS No.: 2684216-48-4), DMF (1.3 mL), and certain amounts of DIPEA and HATU were sequentially added to a 4 mL centrifuge tube, and the resulting reaction mixture was stirred at room temperature until HPLC indicated the completion of the reaction. The reaction mixture was purified by semi-preparative HPLC to afford compound **4-1b** (82% yield, white solid). MS (ESI) of C₈₄H₁₃₆N₁₄O₃₁²⁺ [M+2H]²⁺/2: calculated 918.47, measured 918.69.

### (2) Preparation of compound 4-1

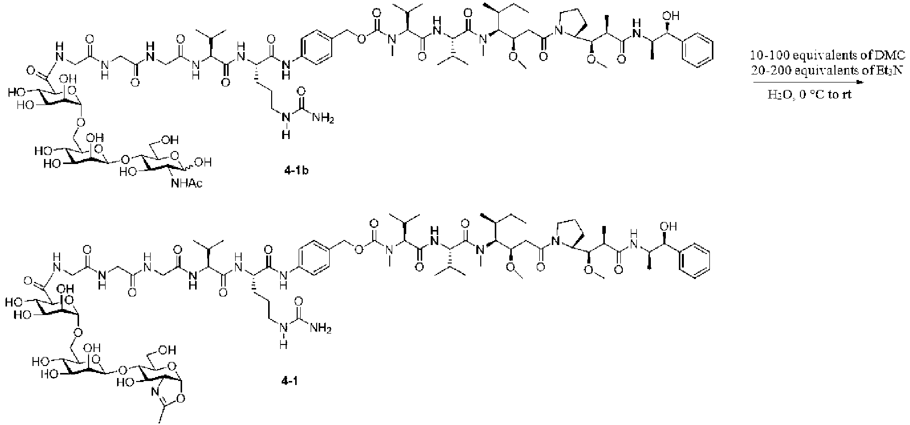

At room temperature, compound **4-1b** (2.5 mg, 0.0014 mmol), H₂O, and certain amounts of Et₃N and DMC (2-chloro-1,3-dimethylimidazolinium chloride, CAS No. 37091-73-9) were sequentially added to a 4 mL centrifuge tube, and the resulting reaction mixture was stirred at room temperature until HPLC indicated the completion of the reaction. The reaction mixture was purified by semi-preparative HPLC to afford compound **4-1** (2 mg, 81% yield, white solid). MS (ESI) of C₈₄H₁₃₄N₁₄O₃₀²⁺ [M+2H]²⁺/2: calculated 909.47, measured 909.97.

### Example 12: Preparation of linker-payload compound 4-2

The structure of linker-payload compound **4-2** is as follows:

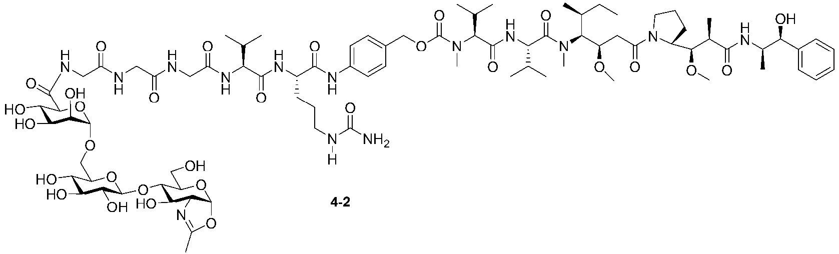

### (1) Preparation of compound 4-2a

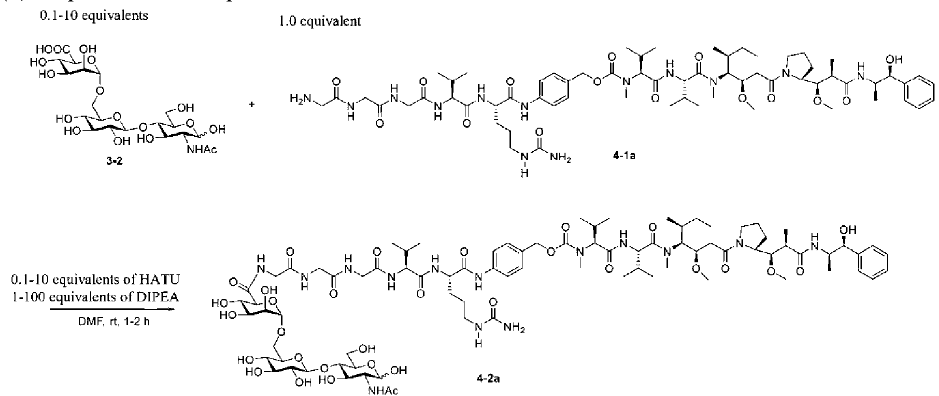

At room temperature, compound **3-2** (0.1-10 equivalents relative to compound **4-1a**), compound **4-la** (19.4 mg, 0.015 mmol, CAS No.: 2684216-48-4), DMF (500 µL), and certain amounts of DIPEA and HATU were sequentially added to a 4 mL centrifuge tube, and the resulting reaction mixture was stirred at room temperature until HPLC indicated the completion of the reaction. The reaction mixture was purified by semi-preparative HPLC to afford compound **4-2a** (white solid), which was directly used in the next step. MS (ESI) of C₈₄H₁₃₆N₁₄O₃₁²⁺ [M+2H]²⁺/2: calculated 918.47, measured 918.92.

### (2) Preparation of compound 4-2

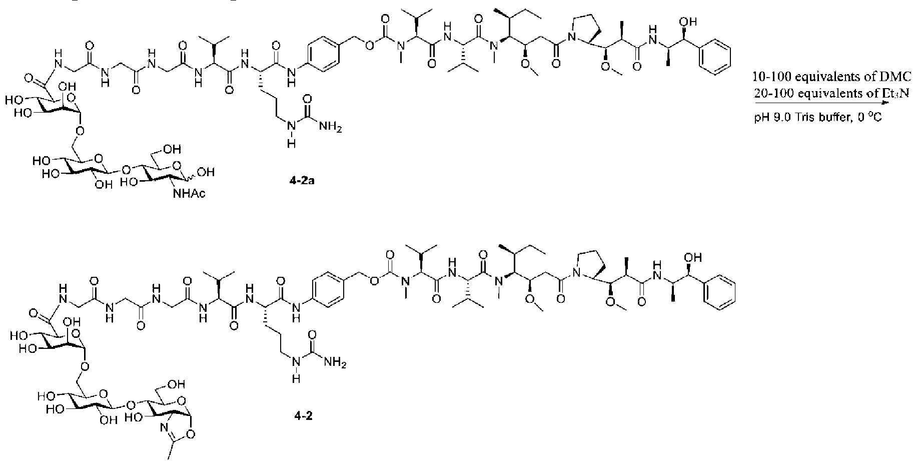

At 0 °C, compound **4-2a** (27.5 mg, 0.015 mmol) and Tris buffer (pH = 9.0, 2 mL) were added to a 15 mL centrifuge tube. After dissolution, certain amounts of triethylamine and 2-chloro-1,3-dimethylimidazolinium chloride were added, and the resulting reaction mixture was stirred at 0 °C until HPLC indicated the completion of the reaction. The reaction mixture was purified by semi-preparative HPLC to afford compound **4-2** (15.5 mg, 57% yield over two steps, white solid). MS (ESI) of C₈₄H₁₃₄N₁₄O₃₀²⁺ [M+2H]²⁺/2: calculated 909.47, measured 909.73.

### Example 13: Preparation of linker-payload compound 4-3

The structure of linker-payload compound **4-3** is as follows:

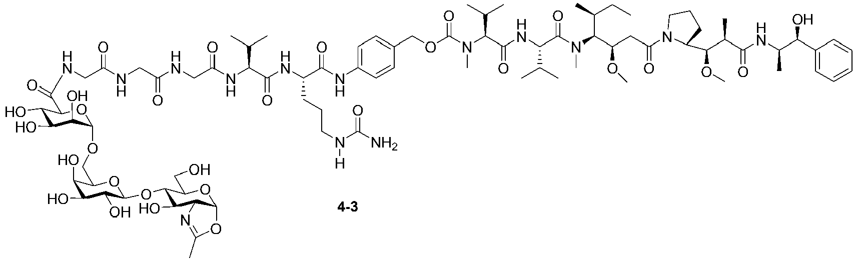

### (1) Preparation of compound 4-3a

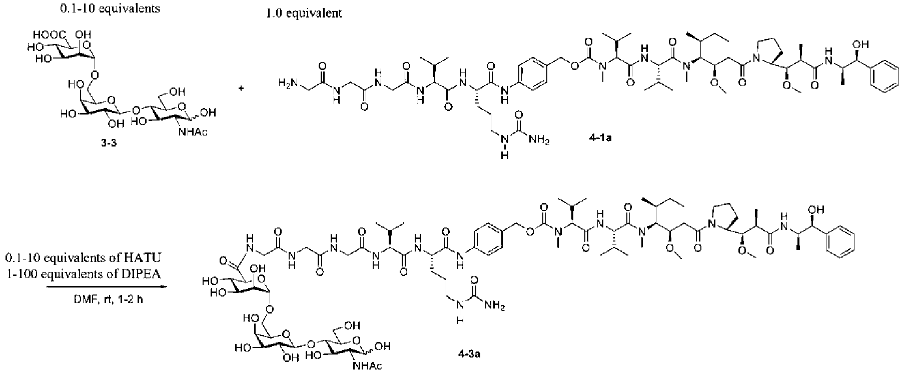

At room temperature, compound **3-3** (0.1-10 equivalents relative to compound **4-1a),** compound **4-1a** (19.8 mg, 0.015 mmol, CAS No.: 2684216-48-4), DMF (500 µL), and certain amounts of DIPEA and HATU were sequentially added to a 4 mL centrifuge tube, and the resulting reaction mixture was stirred at room temperature until HPLC indicated the completion of the reaction. The reaction mixture was purified by semi-preparative HPLC to afford compound **4-3a** (white solid), which was directly used in the next step. MS (ESI) of C₈₄H₁₃₆N₁₄O₃₁²⁺ [M+2H]²⁺/2: calculated 918.47, measured 918.94.

### (2) Preparation of compound 4-3

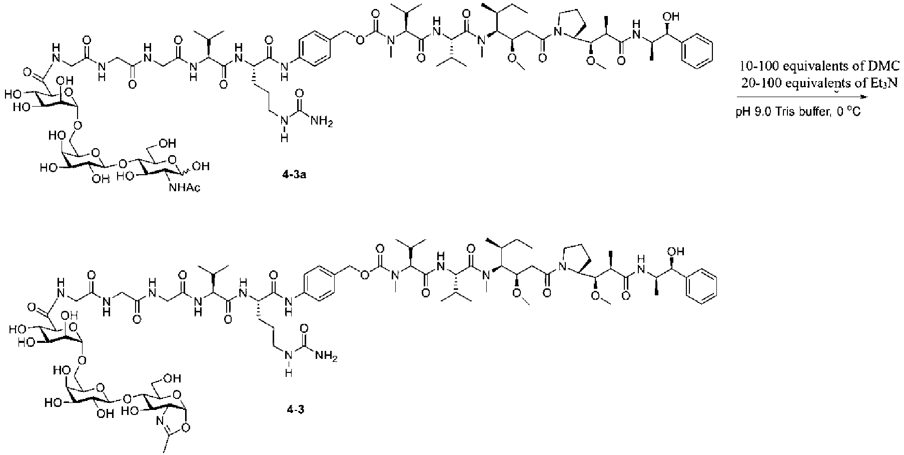

At 0 °C, compound 4-3a (28.0 mg, 0.015 mmol) and Tris buffer (pH = 9.0, 2 mL) were added to a 15 mL centrifuge tube. After dissolution, certain amounts of triethylamine and 2-chloro-1,3-dimethylimidazolinium chloride were added, and the resulting reaction mixture was stirred at 0 °C until HPLC indicated the completion of the reaction. The reaction mixture was purified by semi-preparative HPLC to afford compound 4-3 (14.4 mg, 52% yield over two steps, white solid). MS (ESI) of C₈₄H₁₃₄N₁₄O₃₀²⁺ [M+2H]²⁺/2: calculated 909.47, measured 909.71.

### Example 14: Preparation of linker-payload compound 4-4

The structure of linker-payload compound 4-4 is as follows:

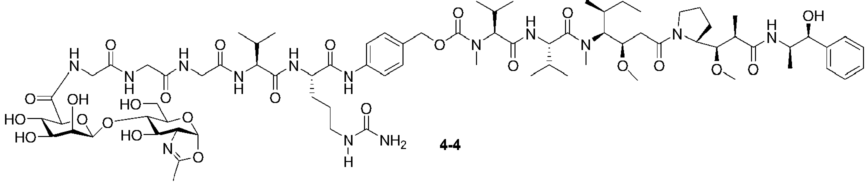

The synthesis of compound 4-4 was performed according to Example 6 in WO2023232144A1.

### Example 15: Preparation of linker-payload compound 4-5

The structure of linker-payload compound 4-5 is as follows:

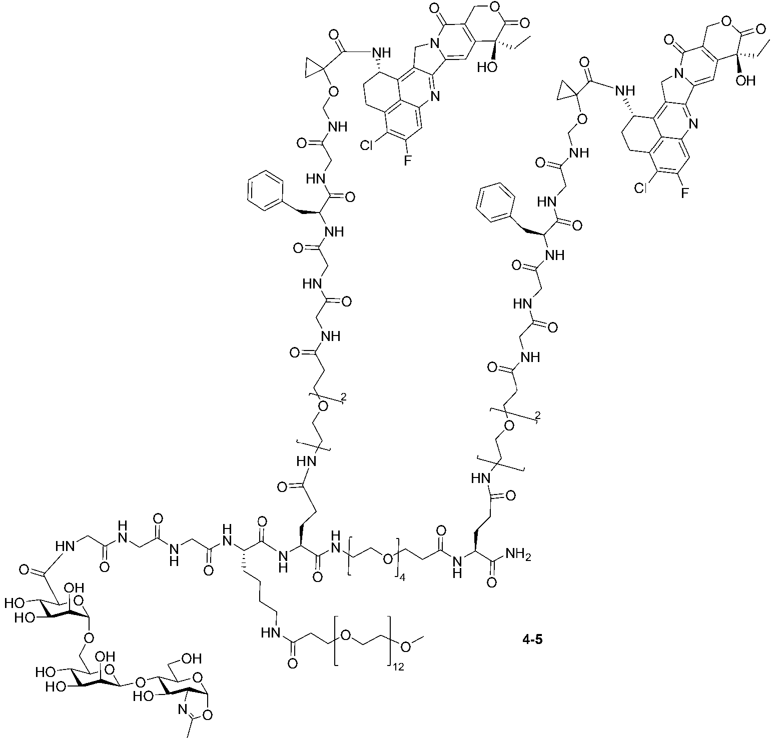

### (1) Preparation of compound 4-5-1

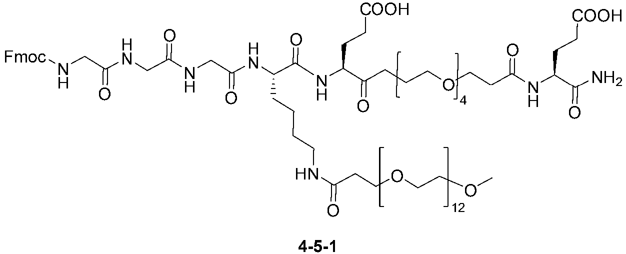

**4-5-1** was synthesized by using the solid-phase peptide synthesis, with procedures similar to those for the synthesis of **LP-6-2** described in Patent application WO2023232144A1. The crude product after cleavage was subjected to Prep-HPLC. After lyophilization, compound **4-5-1** was obtained. MS (ESI) of C₇₆H₁₂₅N₉O₃₁²⁺ [M+2H]²⁺/2: calculated 829.92, measured 830.56.

### (2) Preparation of compound 4-5-2

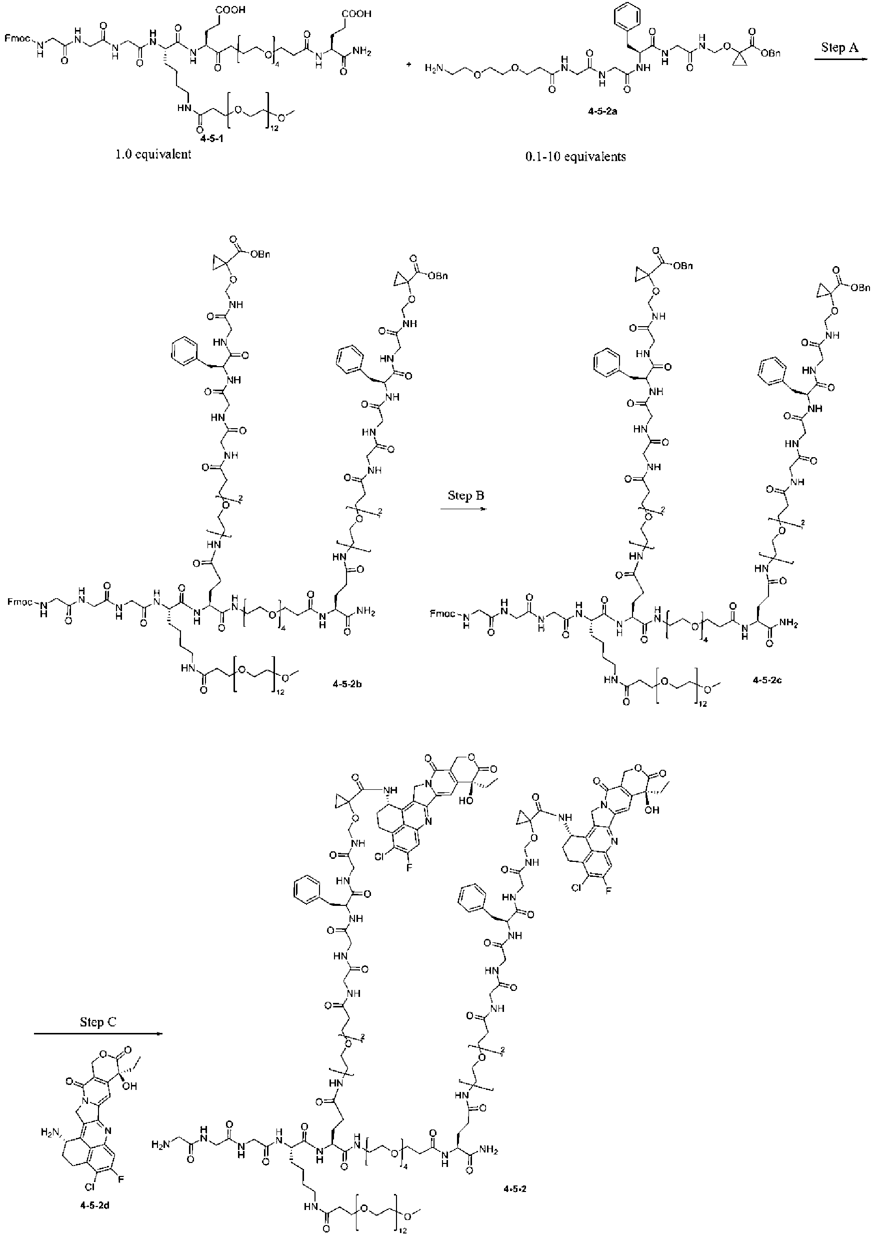

### Step A: Preparation of compound 4-5-2b

Proper amounts of **4-5-1** (21.4 g, 12.9 mmol) and **4-5-2a** (0.1-10 equivalents relative to **4-5-1)** were added to a round-bottom flask and dissolved in DMF (450 mL). After dissolution, the mixture was cooled in an ice bath to 0-5 °C and stirred for 5 min. HATU was added for complete dissolution, and 2,4,6-trimethylpyridine was finally added. The mixture was stirred at 0-5 °C for 1 h, and then stirred for another 1 h after being warmed to room temperature. After the depletion of the starting materials was detected, the mixture was subjected to prep-HPLC. After lyophilization, compound **4-5-2b** (31.5 g, 81.6% yield, white solid) was obtained. MS (ESI) of C₁₄₄H₂₂₃N₂₄O₄₉³⁺ [M+3NH₄]³⁺/3: calculated 1024.19, measured 1024.72.

### Steps B and C: Preparation of compound 4-5-2

**4-5-2b** (38.5 g, 12.7 mmol) was dissolved in DMF (400 mL) in a reaction flask, and a certain amount of Pd/C was added. After the system was purged with hydrogen for 3 times, the mixture was reacted at room temperature for 2 h. After atline HPLC indicated the depletion of the starting materials, the Pd/C was removed by filtration through celite, and the mixture was cooled in an ice bath to 0-5 °C. **4-5-2d** (0.1-10 equivalents relative to **4-5-2b)** and PyBop were added. After complete dissolution by stirring, DIEA was added dropwise, and the mixture was stirred at room temperature for 2 h. Samples were taken for HPLC analysis. After the depletion of the starting materials, DEA was added, and the mixture was stirred at room temperature for 30 min. After atline HPLC indicated the reaction was completed, the pH was adjusted to 5-6. The mixture was subjected to prep-HPLC. After lyophilization, compound **4-5-2** (27.4 g, 61.6% yield, pink solid) was obtained. MS (ESI) of C₁₆₁H₂₃₂Cl₂F₂N₂₉O₅₃³⁺ [M+H+2NH₄]³⁺/3: calculated 1175.86, measured 1176.45.

The synthesis of compound **4-5-2a** was performed according to Patent application WO2023232144A1.

The synthesis of compound **4-5-2d** was performed according to Patent No. CN202211428194.6.

### (3) Preparation of compound 4-5a

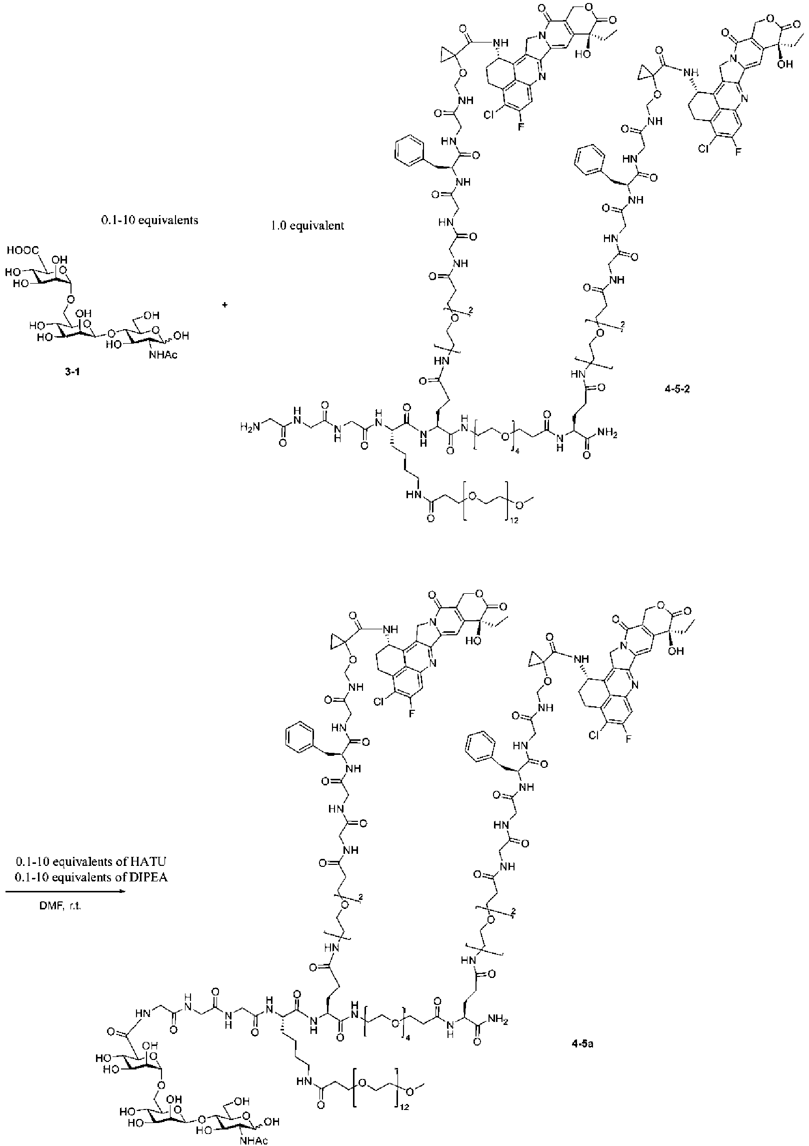

At room temperature, compound 3-1 (0.1-10 equivalents relative to **4-5-2),** compound **4-5-2** (33 mg, 0.010 mmol), DMF (500 µL), and certain amounts of DIPEA and HATU were sequentially added to a 4 mL centrifuge tube, and the resulting reaction mixture was stirred at room temperature until HPLC indicated the completion of the reaction. The reaction mixture was purified by semi-preparative HPLC to afford compound **4-5a** (22.4 mg, 59% yield, white solid). MS (ESI) of C₁₈₁H₂₆₀Cl₂F₂N₂₉O₆₉³⁺ [M+2H+NH₄]³⁺/3: calculated 1350.57, measured 1351.75.

### (4) Preparation of compound 4-5

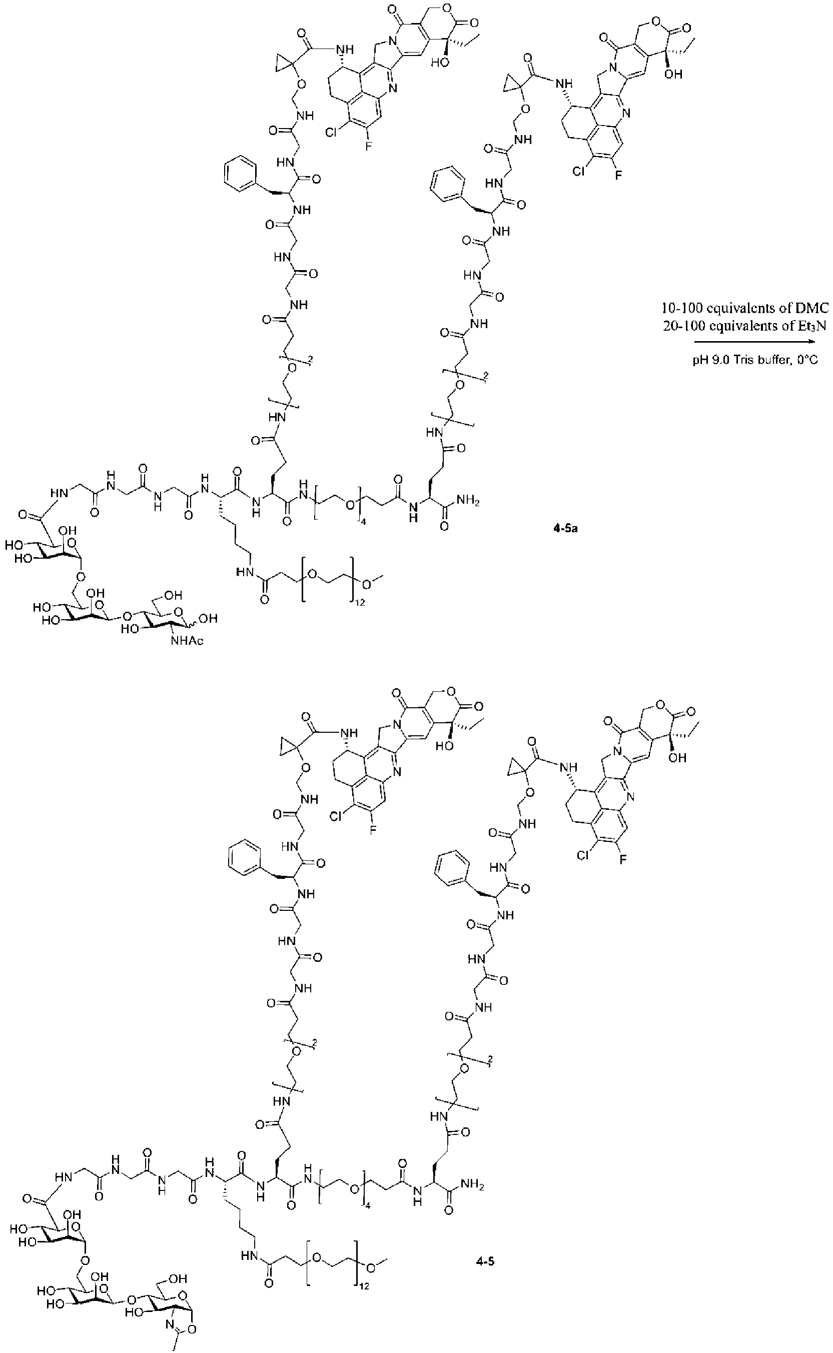

At 0 °C, compound **4-5a** (22.4 mg, 0.006 mmol) and Tris buffer (pH = 9.0, 2 mL) were added to a 15 mL centrifuge tube. After dissolution, certain amounts of triethylamine and 2-chloro-1,3-dimethylimidazolinium chloride were added, and the resulting reaction mixture was stirred at 0 °C until HPLC indicated the completion of the reaction. The reaction mixture was purified by semi-preparative HPLC to afford compound **4-5** (20.1 mg, 90% yield, white solid). MS (ESI) of C₁₈₁H₂₆₁Cl₂F₂N₃₀O₆₈³⁺ [M+H+2NH₄]³⁺/3: calculated 1350.24, measured 1352.14.

### Example 16: Preparation of linker-payload compound 4-6

The structure of linker-payload compound **4-6** is as follows:

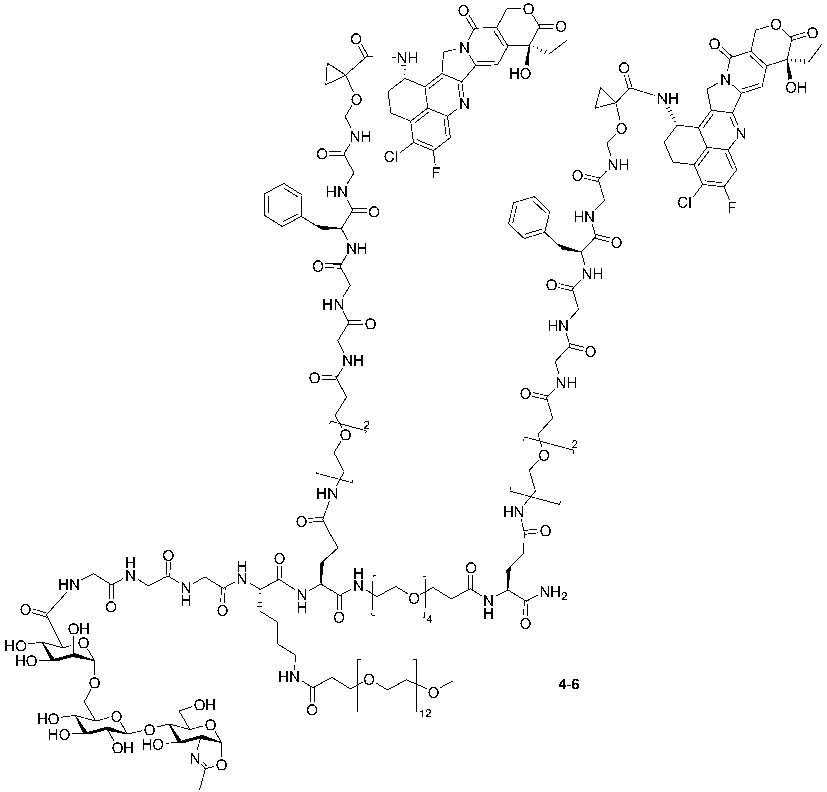

### (1) Preparation of compound 4-6a

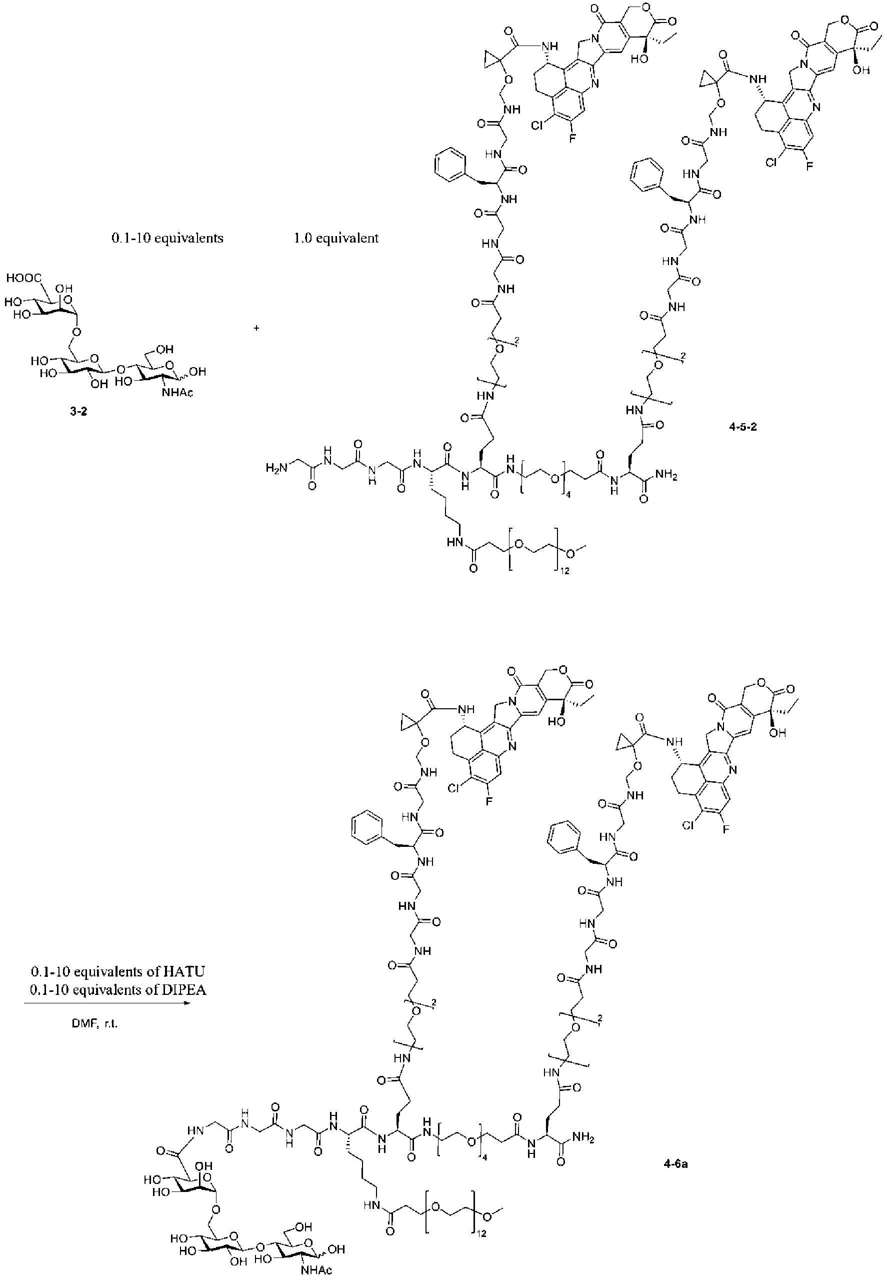

At room temperature, compound **3-2** (0.1-10 equivalents relative to **4-5-2),** compound **4-5-2** (32.5 mg, 0.009 mmol), DMF (500 µL), and certain amounts of DIPEA and HATU were sequentially added to a 4 mL centrifuge tube, and the resulting reaction mixture was stirred at room temperature until HPLC indicated the completion of the reaction. The reaction mixture was purified by semi-preparative HPLC to afford compound **4-6a** (25.6 mg, 68% yield, white solid). MS (ESI) of C₁₈₁H₂₆₃Cl₂F₂N₃₀O₆₉³⁺ [M+H+2NH₄]³⁺/3: calculated 1356.24, measured 1357.06.

### (2) Preparation of compound 4-6

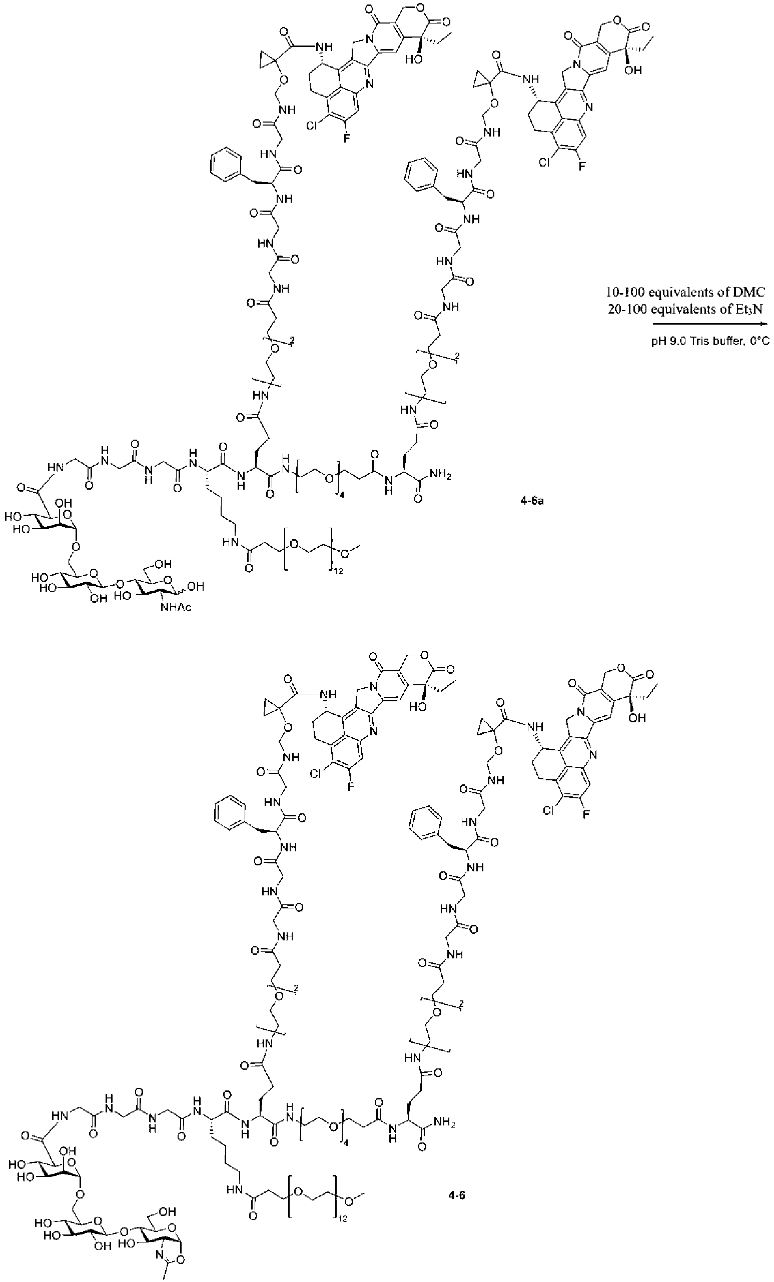

At 0 °C, compound **4-6a** (25.6 mg, 0.006 mmol) and Tris buffer (pH = 9.0, 2 mL) were added to a 15 mL centrifuge tube. After dissolution, certain amounts of triethylamine and 2-chloro-1,3-dimethylimidazolinium chloride were added, and the resulting reaction mixture was stirred at 0 °C until HPLC indicated the completion of the reaction. The reaction mixture was purified by semi-preparative HPLC to afford compound **4-6** (21.7 mg, 85% yield, white solid). MS (ESI) of C₁₈₁H₂₆₁Cl₂F₂N₃₀O₆₈³⁺ [M+H+2NH₄]³⁺/3: calculated 1350.24, measured 1351.43.

### Example 17: Preparation of linker-payload compound 4-7

The structure of linker-payload compound **4-7** is as follows:

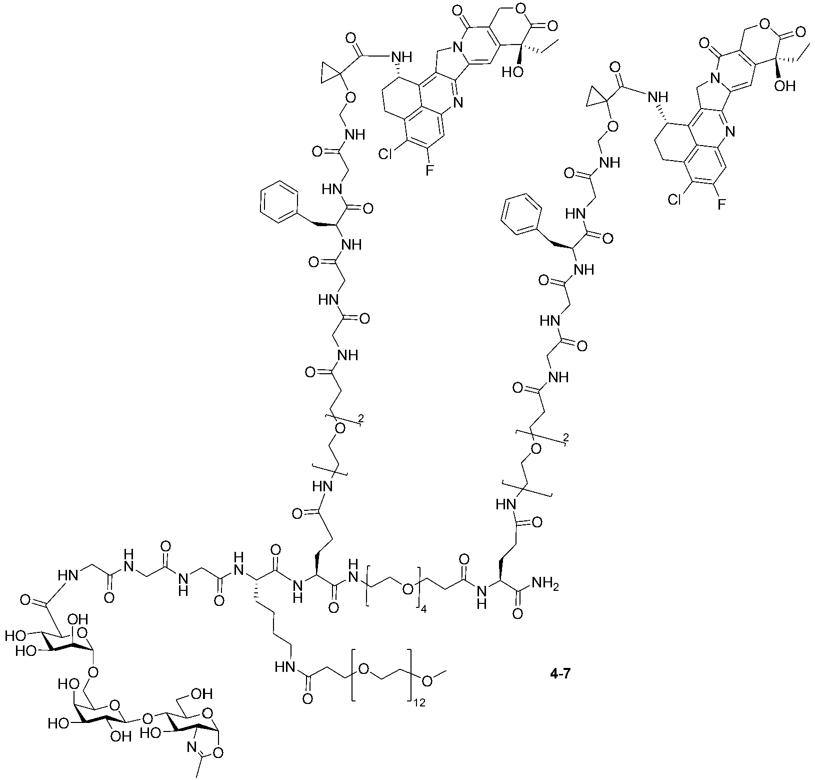

### (1) Preparation of compound 4-7a

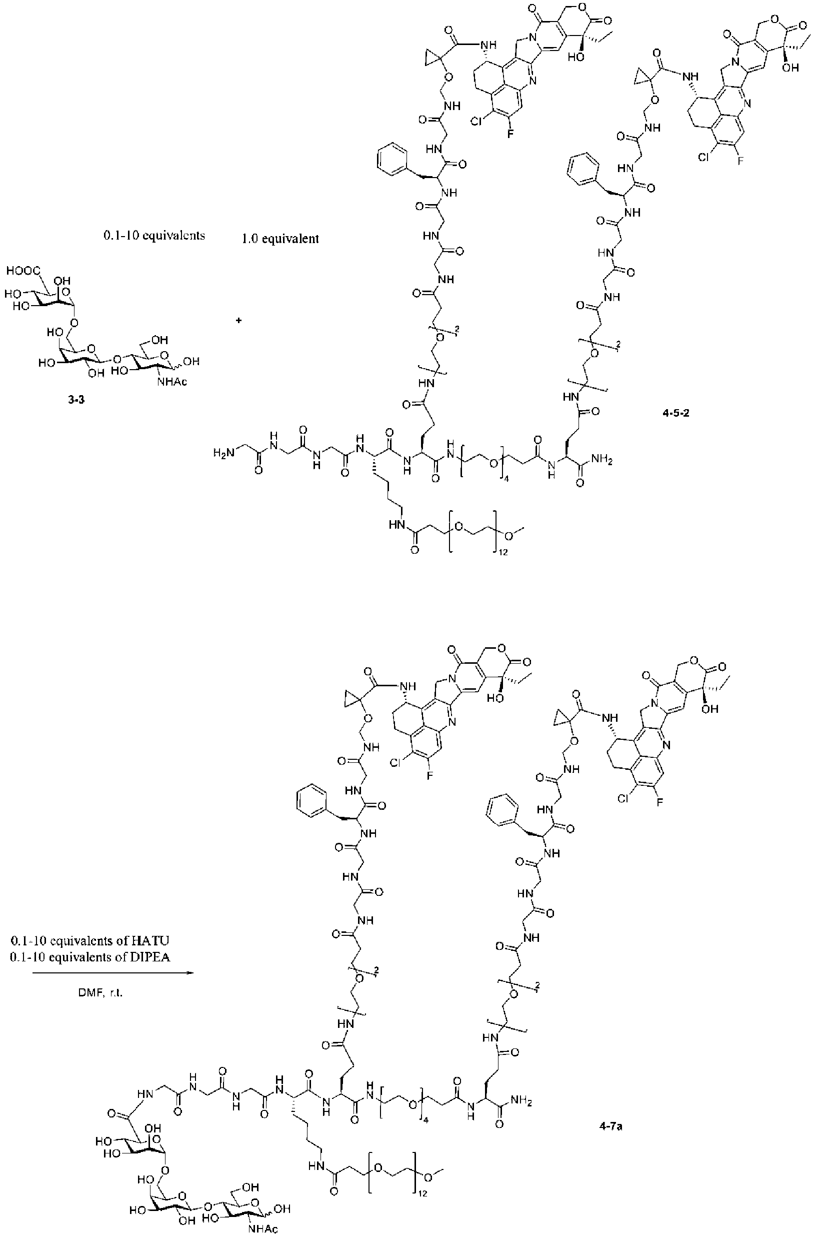

At room temperature, compound **3-3** (0.1-10 equivalents relative to **4-5-2),** compound **4-5-2** (31.7 mg, 0.009 mmol), DMF (500 µL), and certain amounts of DIPEA and HATU were sequentially added to a 4 mL centrifuge tube, and the resulting reaction mixture was stirred at room temperature until HPLC indicated the completion of the reaction. The reaction mixture was purified by semi-preparative HPLC to afford compound **4-7a** (29.2 mg, 80% yield, white solid). MS (ESI) of C₁₈₁H₂₆₆Cl₂F₂N₃₁O₆₉³⁺ [M+3NH₄]³⁺/3: calculated 1361.92, measured 1362.87.

### (2) Preparation of compound 4-7

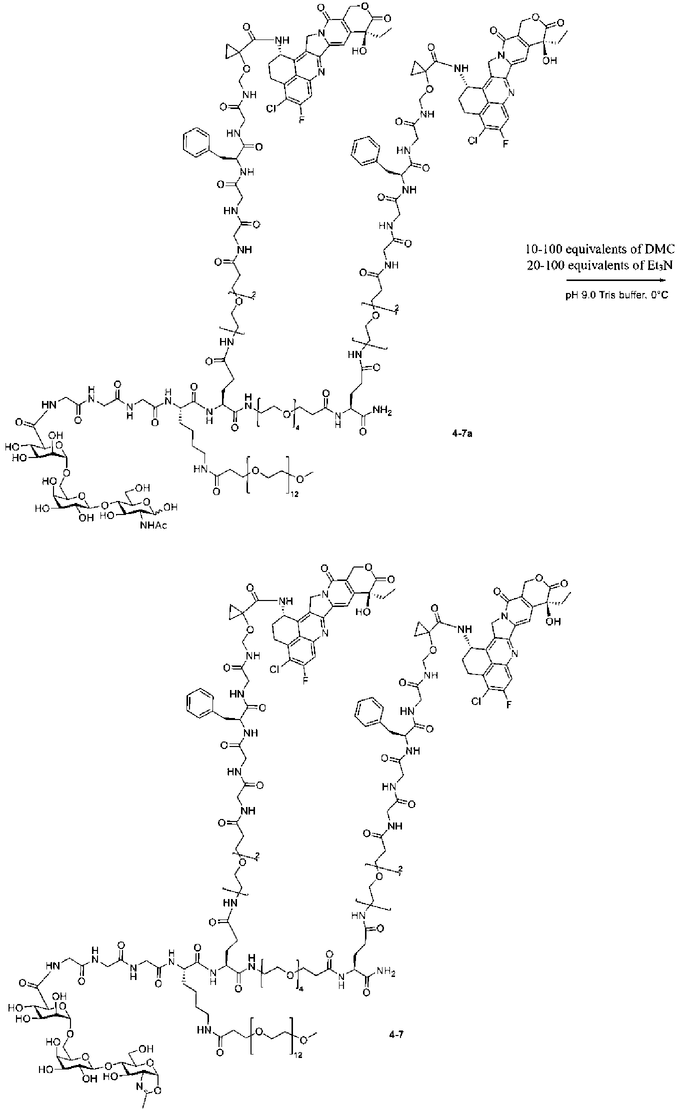

At 0 °C, compound **4-7a** (29.2 mg, 0.007 mmol) and Tris buffer (pH = 9.0, 2 mL) were added to a 15 mL centrifuge tube. After dissolution, certain amounts of triethylamine and 2-chloro-1,3-dimethylimidazolinium chloride were added, and the resulting reaction mixture was stirred at 0 °C until HPLC indicated the completion of the reaction. The reaction mixture was purified by semi-preparative HPLC to afford compound **4-7** (15.2 mg, 52% yield, white solid). MS (ESI) of C₁₈₁H₂₆₁Cl₂F₂N₃₀O₆₈³⁺ [M+H+2NH₄]³⁺/3: calculated 1350.24, measured 1351.67.

### Example 18: Preparation of linker-payload compound 4-8

The structure of linker-payload compound **4-8** is as follows:

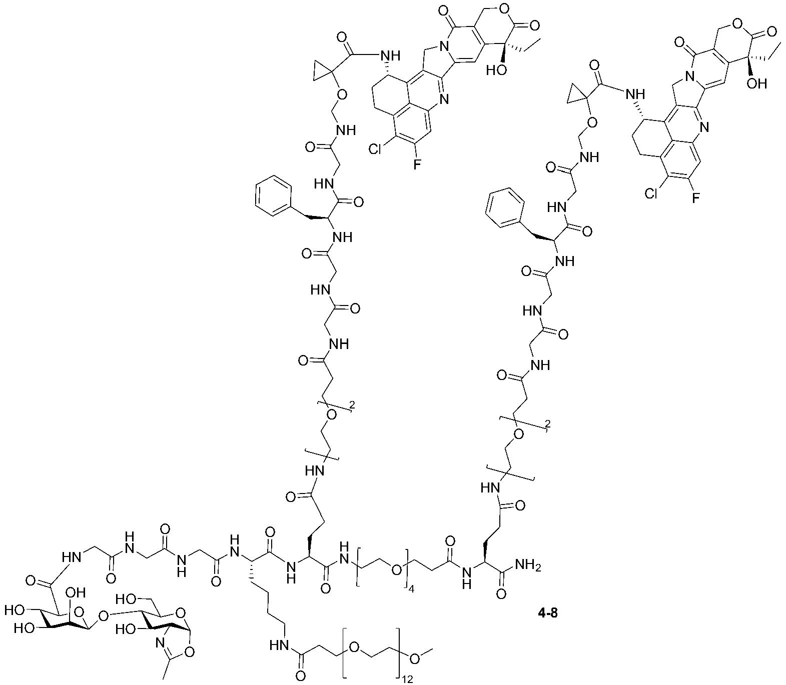

Linker-payload **4-8** was synthesized with procedures similar to those for the synthesis of **4-5.**

### Example 19: Preparation of linker-payload compound 4-9

The structure of linker-payload compound **4-9** is as follows:

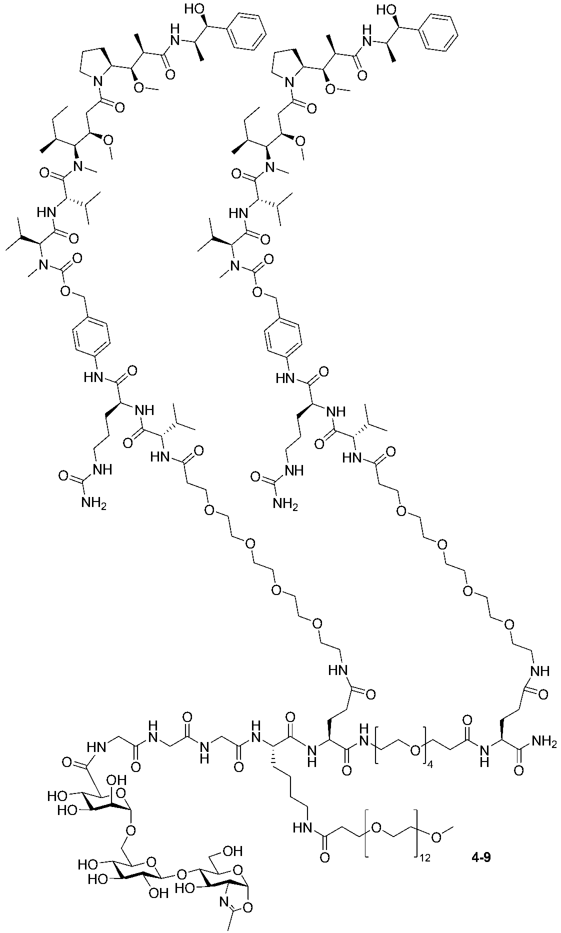

### (1) Preparation of compound 4-9-1

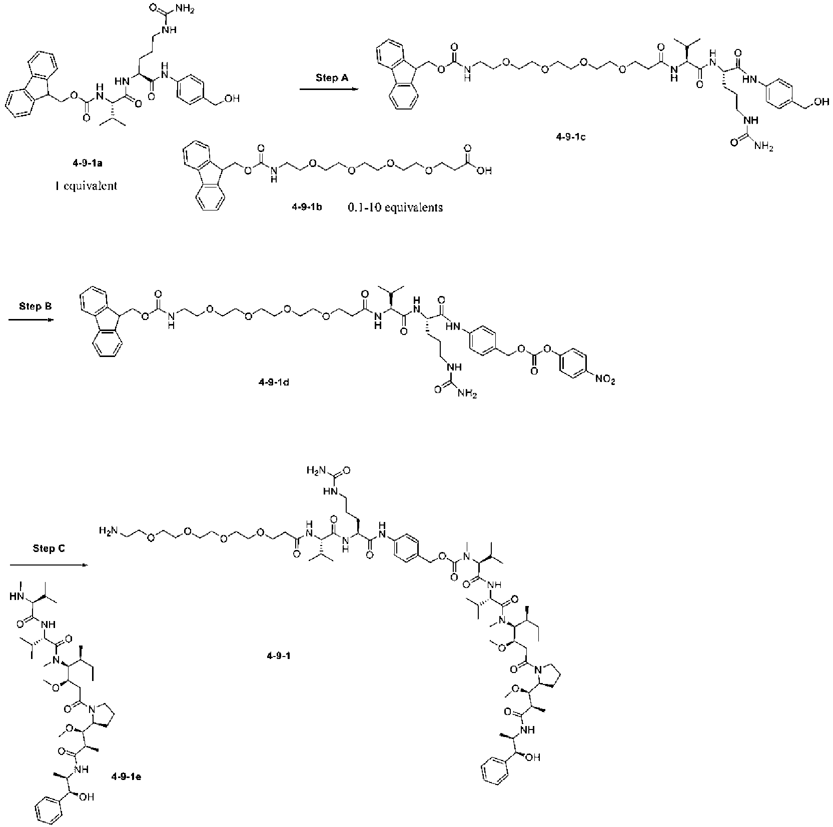

### Step A: Preparation of compound 4-9-1c

At room temperature, **4-9-1a** (1 g, 1.662 mmol, CAS No.: 159858-22-7) was added to a 250 mL round-bottom flask, and DMA (50 mL) was added. After complete dissolution, DBU was added, and the resulting mixture was stirred for 2.5 h. After HPLC monitoring indicated the depletion of the starting materials, PPTS was added to the reaction system, and after 10 min of stirring, EDCI, **4-9-1b** (0.1-10 equivalents relative to **4-9-1a,** CAS No.: 557756-85-1), and HOBT were sequentially added to the reaction mixture. The resulting mixture was stirred for 12 h until HPLC indicated the completion of the reaction. The reaction mixture was purified by semi-preparative HPLC to afford compound **4-9-1c** (1.3 g, 92% yield, white solid). MS (ESI) of C₄₄H₆₁N₆O₁₁⁺ [M+H]⁺: calculated 849.44, measured 849.33.

### Step B: Preparation of compound 4-9-1d

At 0 °C, **4-9-1c** (1.3 g, 1.531 mmol) was added to a 100 mL round-bottom flask, and DMF (25 mL) was added. After complete dissolution, p-(dinitrophenyl)carbonate and DIEA were added, and the resulting mixture was stirred at 0 °C for 0.5 h. The ice-water bath was then removed, and the mixture was stirred at room temperature for 12 h. After TLC monitoring indicated the depletion of the starting materials, water (200 mL) was added to the reaction system, and the mixture was extracted with EA (150 mL × 3). The organic phase was collected, washed with a saturated sodium chloride solution (500 mL), dried over anhydrous sodium sulfate, filtered, and concentrated, and the residue was triturated in diethyl ether and then filtered. The resulting solid was dried with an oil pump to afford compound **4-9-1d** (1.43 g, 92% yield, white solid). MS (ESI) of C₃₁H₆₄N₇O₁₅⁺ [M+H]⁺: calculated 1014.45, measured 1014.30.

### Step C: Preparation of compound 4-9-1

At room temperature, **4-9-1d** (1.43 g, 1.417 mmol) was added to a 100 mL round-bottom flask, and DMF (25 mL) was added. After complete dissolution, HOBT and DIEA were added. After the mixture was stirred for 5 min, **4-9-1e** (0.1-10 equivalents relative to **4-9-1d,** CAS No.: 474645-27-7) was added, and the resulting mixture was stirred at room temperature for 12 h until HPLC indicated the completion of the reaction. Diethylamine (2.5 mL) was added to the reaction mixture, and the resulting mixture was stirred for 0.5 h until HPLC indicated the completion of the reaction. The reaction mixture was purified by semi-preparative HPLC to afford compound **4-9-1** (856.6 mg, 44% yield, white solid). MS (ESI) of C₆₉H₁₁₇N₁₁O₁₇²⁺ [M+2H]²⁺/2: calculated 685.93, measured 686.20.

### (2) Preparation of compound 4-9-2

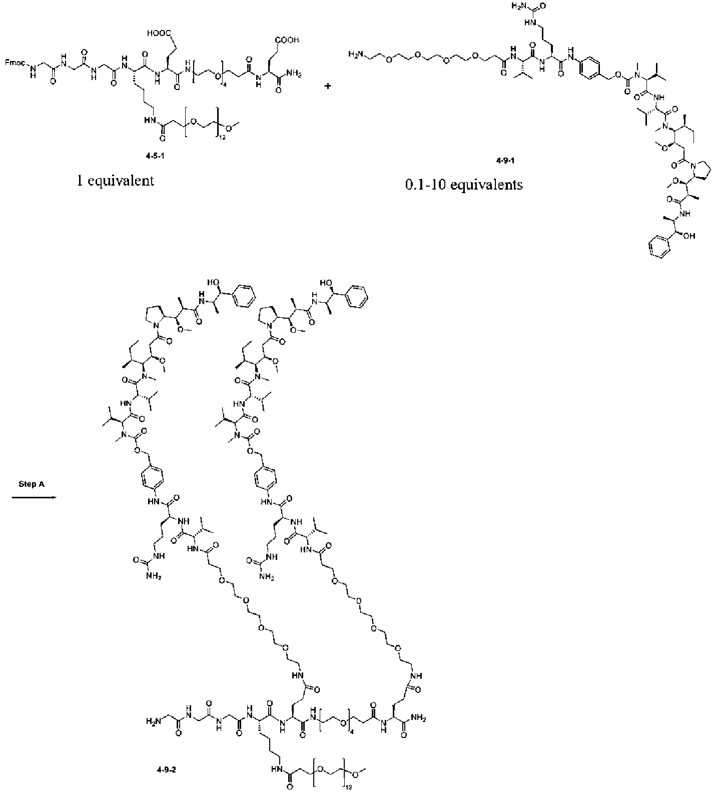

At room temperature, **4-5-1** (873 mg, 0.282 mmol) and **4-9-1** (0.1-10 equivalents relative to **4-5-1)** were added to a 25 mL round-bottom flask, and DMF (8 mL) was added. The mixture was cooled in an ice bath to 0 °C, and then DIEA was added. After the mixture was stirred for 5 min, HATU was added. The resulting mixture was stirred at room temperature for 2 h until HPLC indicated the completion of the reaction. Diethylamine (800 µL) was added to the reaction mixture, and the resulting mixture was stirred for 0.5 h until HPLC indicated the completion of the reaction. The reaction mixture was purified by semi-preparative HPLC to afford compound **4-9-2** (1.2 g, 88.8% yield, white solid). MS (ESI) of C₁₉₉H₃₄₆N₃₂O₆₁⁴⁺ [M+3H+NH₄]⁴⁺/4: calculated 1040.13, measured 1040.89.

### (2) Preparation of compound 4-9a

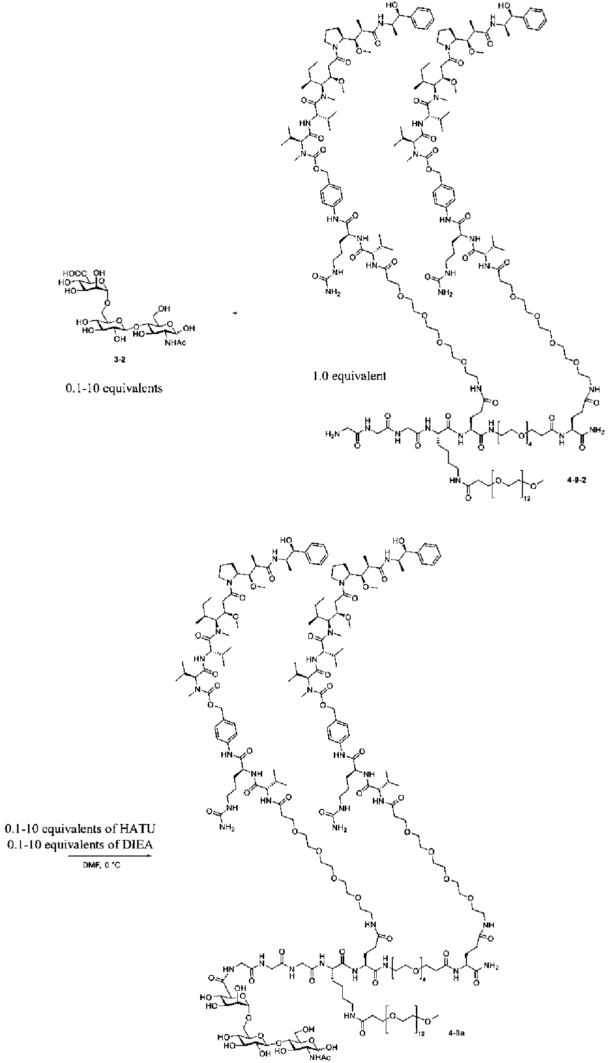

At room temperature, compound **3-2** (0.1-10 equivalents relative to **4-9-2),** compound **4-9-2** (29.6 mg, 0.007 mmol), DMF (500 µL), and certain amounts of DIPEA and HATU were sequentially added to a 4 mL centrifuge tube, and the resulting reaction mixture was stirred at room temperature until HPLC indicated the completion of the reaction. The reaction mixture was purified by semi-preparative HPLC to afford compound **4-9a** (white solid), which was directly used in the next step. MS (ESI) of C₂₁₉H₃₇₆N₃₃O₇₇³⁺ [M+2H+NH₄]³⁺/3: calculated 1566.88, measured 1567.40.

### (3) Preparation of compound 4-9

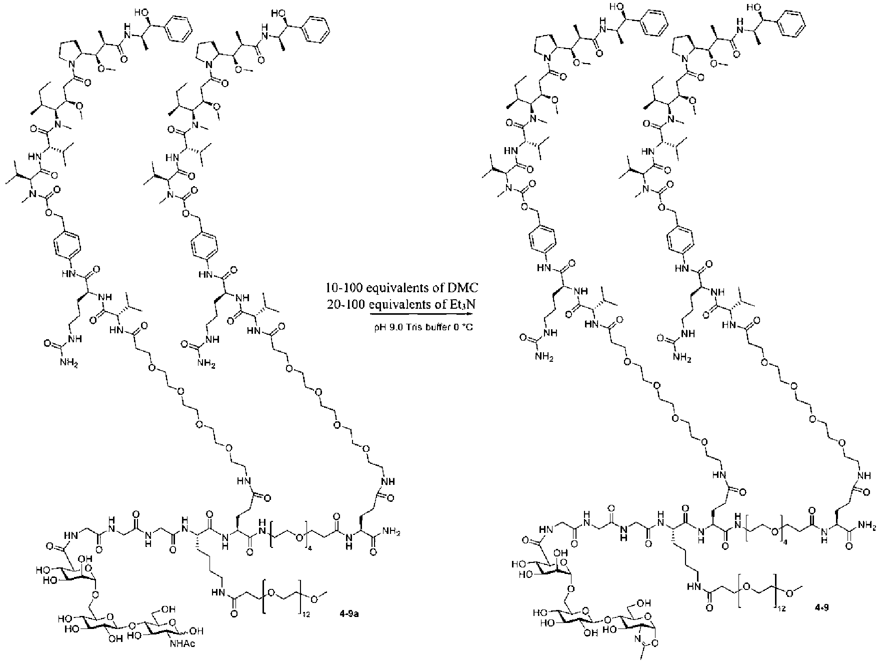

At 0 °C, compound **4-9a** (33 mg, 0.007 mmol) and Tris buffer (pH = 9.0, 2 mL) were added to a 15 mL centrifuge tube. After dissolution, certain amounts of triethylamine and 2-chloro-1,3-dimethylimidazolinium chloride were added, and the resulting reaction mixture was stirred at 0 °C until HPLC indicated the completion of the reaction. The reaction mixture was purified by semi-preparative HPLC to afford compound **4-9** (15.3 mg, 46% yield over two steps, white solid). MS (ESI) of C₂₁₉H₃₇₄N₃₃O₇₆³⁺ [M+2H+NH₄]³⁺/3: calculated 1560.88, measured 1561.60.

### Example 20: Preparation of linker-payload compound 4-10

The structure of linker-payload compound **4-10** is as follows:

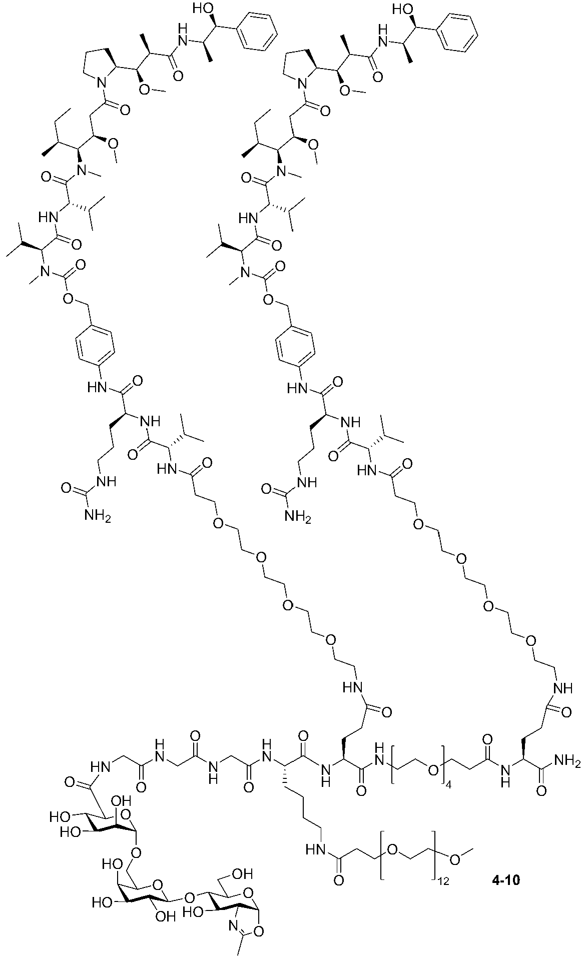

### (1) Preparation of compound 4-10a

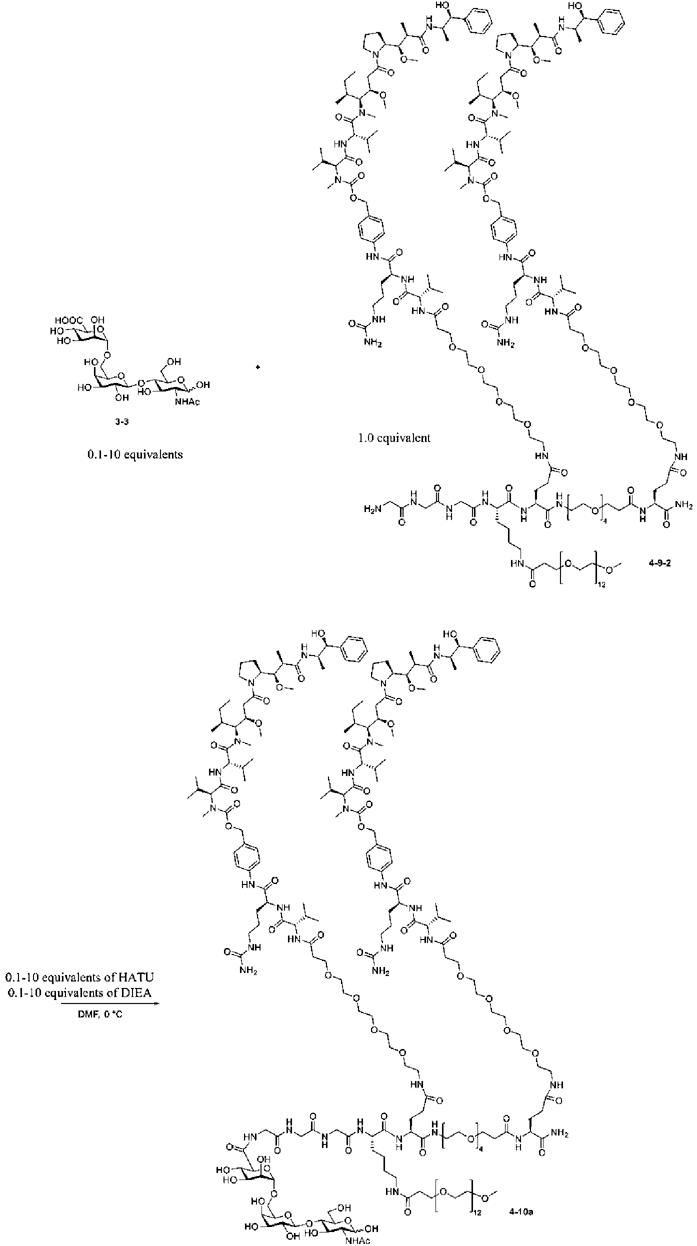

At room temperature, compound **3-3** (0.1-10 equivalents relative to **4-9-2),** compound **4-9-2** (29.6 mg, 0.007 mmol), DMF (500 µL), and certain amounts of DIPEA and HATU were sequentially added to a 4 mL centrifuge tube, and the resulting reaction mixture was stirred at room temperature until HPLC indicated the completion of the reaction. The reaction mixture was purified by semi-preparative HPLC to afford compound **4-10a** (white solid), which was directly used in the next step. MS (ESI) of C₂₁₉H₃₇₆N₃₃O₇₇³⁺ [M+2H+NH₄]³⁺/3: calculated 1566.88, measured 1567.56.

### (2) Preparation of compound 4-10

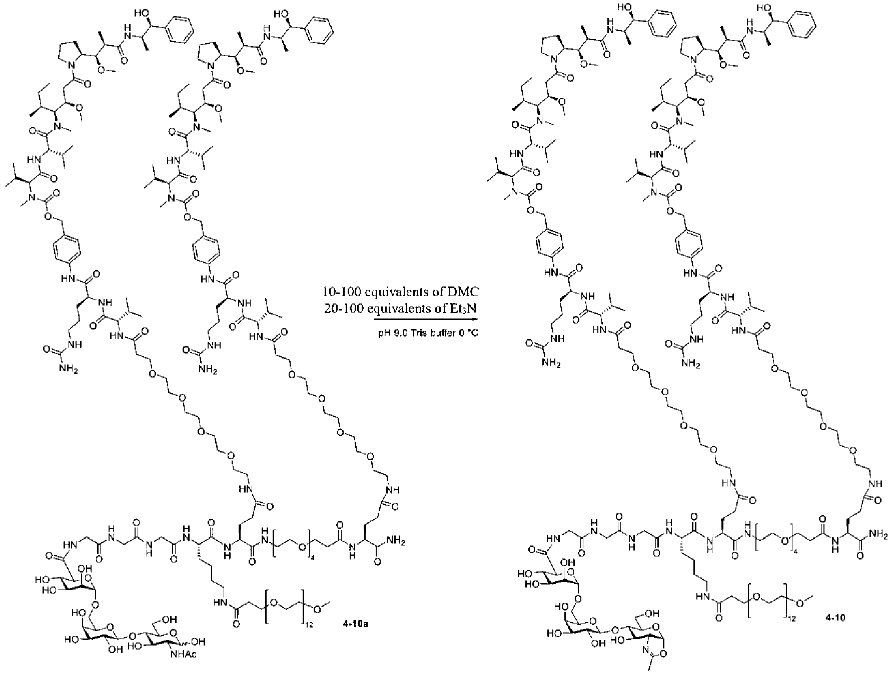

At 0 °C, compound **4-10a** (33 mg, 0.007 mmol) and Tris buffer (pH = 9.0, 2 mL) were added to a 15 mL centrifuge tube. After dissolution, certain amounts of triethylamine and 2-chloro-1,3-dimethylimidazolinium chloride were added, and the resulting reaction mixture was stirred at 0 °C until HPLC indicated the completion of the reaction. The reaction mixture was purified by semi-preparative HPLC to afford compound **4-10** (21.2 mg, 64% yield over two steps, white solid). MS (ESI) of C₂₁₉H₃₇₄N₃₃O₇₆³⁺ [M+2H+NH₄]³⁺/3: calculated 1560.88, measured 1561.44.

### Example 21: Preparation of linker-payload compound 4-11

The structure of linker-payload compound **4-11** is as follows:

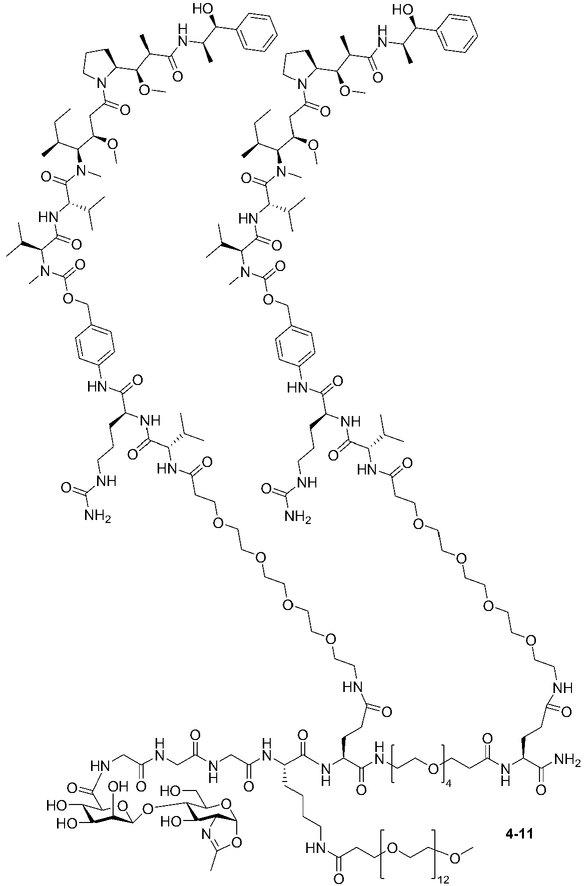

Linker-payload **4-11** was synthesized with procedures similar to those for the synthesis of **4-9.**

### Example 22: Preparation of linker-payload compound 4-12

The structure of linker-payload compound **4-12** is as follows:

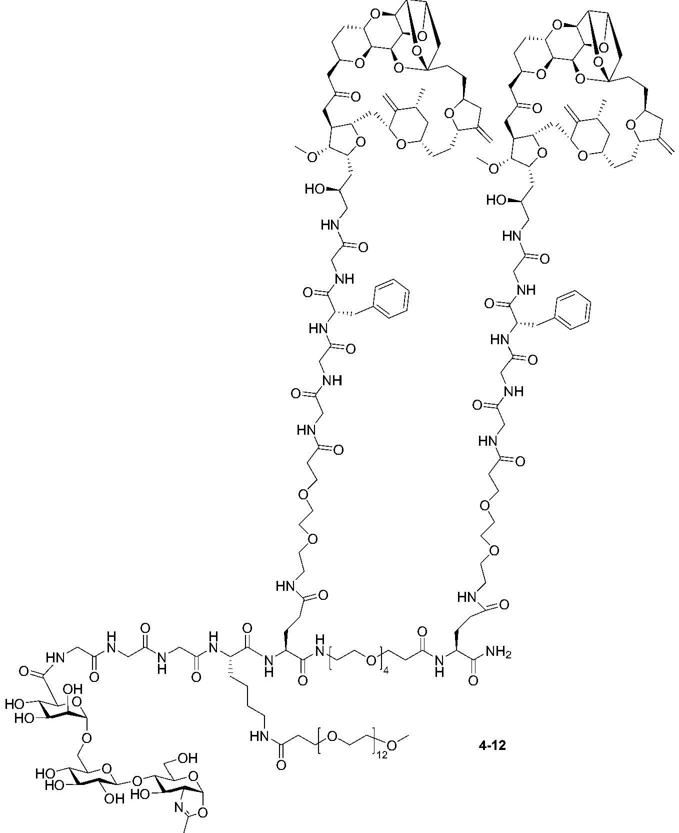

### (1) Preparation of compound 4-12-1

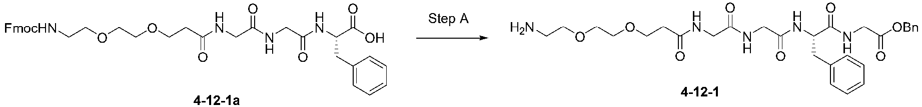

**4-12a** was synthesized by using solid-phase peptide synthesis, and the steps are as follows: Step I: Swelling of resin
19.2 g of 2-CTC-Resin (Sub = 1.04 mmol/g) was added to a synthesis tube and swelled with 190 mL of DCM for 30 min.

### Step II: Conjugation of Fmoc-Phe-OH

A proper amount of Fmoc-Phe-OH (1-10 equivalents) was dissolved in 190 mL of DCM, and after the dissolution, DIEA was added. Then, the mixture was added to a synthesis tube and purged with nitrogen for 2 h. 50 mL of MeOH:DIEA = 5:1 was then added for 30 min of terminal blocking. The solvent was removed, and the mixture was washed once with DCM and then washed 3 times with DMF.

### Step III: Conjugation of Fmoc-Gly-Gly-OH

A deprotective reagent (190 mL) was added, and the mixture was reacted for 10+10 min. After the mixture was washed 4 times with DMF, the Kaiser test demonstrated a black color. The mixture was washed 4 times with DMF.

Proper amounts of Fmoc-Gly-Gly-OH (1-10 equivalents) and ethyl cyanoglyoxylate-2-oxime were dissolved in 190 mL of DMF, and then DIC was added. After 3 min of activation, the resulting mixture was added to a reaction tube and purged with nitrogen for 2 h. The Kaiser test demonstrated a colorless result. The mixture was washed 3 times with DMF.

### Step IV: Conjugation of Fmoc-PEG₂-CH₂CH₂COOH

A deprotective reagent (190 mL) was added, and the mixture was reacted for 10+10 min. After the mixture was washed 4 times with DMF, the Kaiser test demonstrated a black color. The mixture was washed 4 times with DMF.

Proper amounts of Fmoc-PEG₂-CH₂CH₂COOH (1-10 equivalents) and ethyl cyanoglyoxylate-2-oxime were dissolved in 190 mL of DMF, and then DIC was added. After 3 min of activation, the resulting mixture was added to a reaction tube and purged with nitrogen for 2 h. The Kaiser test demonstrated a colorless result. The mixture was washed twice with DMF and 3 times with DCM, and dried overnight.

### Step V: Cleavage of resin

370 mL of TFA:H₂O = 95:5 solution was prepared. The resin was added to the solution, and the mixture was stirred for 2 h and then filtered. The filtrate was concentrated to remove the solvent, and the residue was subjected to prep-HPLC and lyophilized to afford **4-12-1a** (12 g, 91% yield, white solid). MS (ESI) of C₃₅H₄₁N₄O₉⁺ [M+H]⁺: calculated 661.29, measured 661.22.

### Step A: Preparation of compound 4-12-1

**4-12-1a** (12 g, 18.2 mmol) and Gly-OBn▪HCl were dissolved in DMF (120 mL). After the dissolution, HATU was added, and DIEA was then added dropwise. The resulting mixture was stirred at room temperature for 3 h. Samples were taken for HPLC analysis. After the reaction of the starting materials was completed, DEA was added, and the mixture was stirred for 30 min. After atline HPLC indicated the depletion of the starting materials, the mixture was subjected to prep-HPLC and lyophilized to afford **4-12-1** (8.9 g, 84% yield, white solid). MS (ESI) of C₂₉H₄₀N₅O₈⁺ [M+H]⁺: calculated 586.29, measured 586.02.

### (2) Preparation of compound 4-12-2

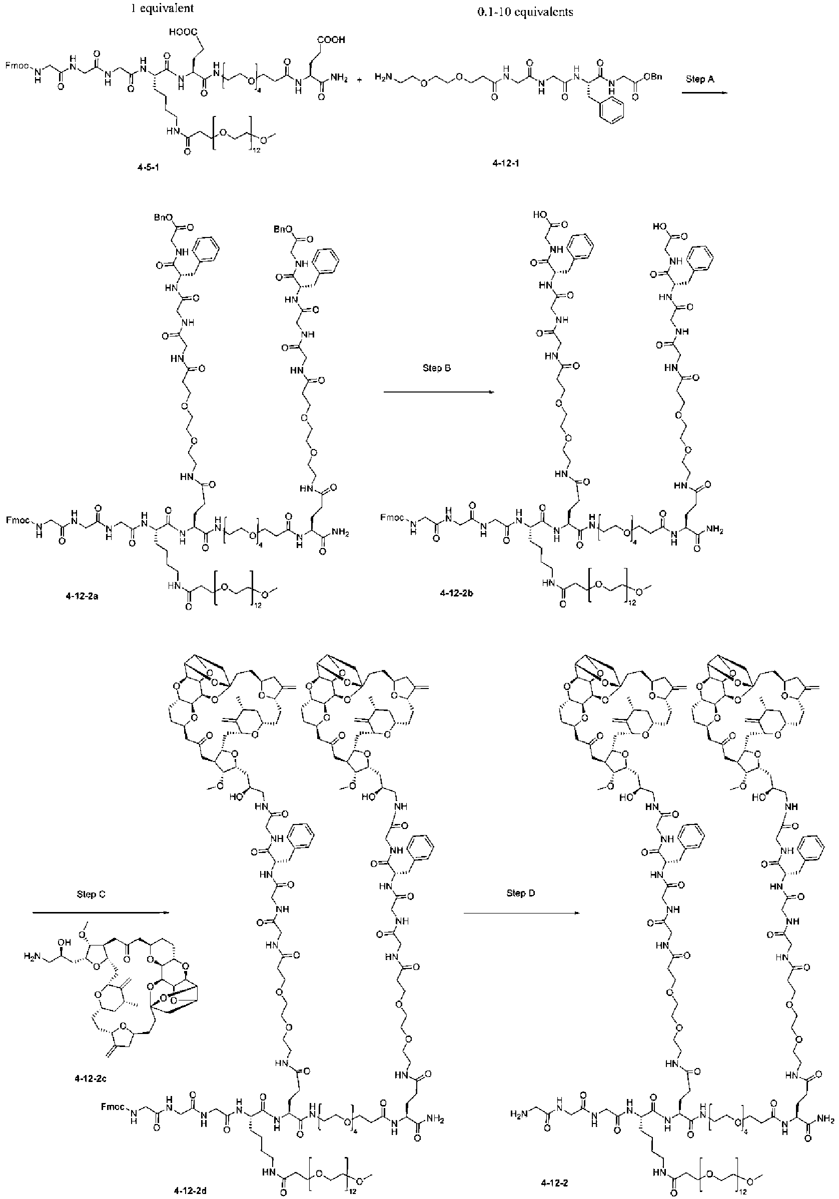

### Step A: Preparation of compound 4-12-2a

At room temperature, **4-5-1** (3.5 mg, 2.111 mmol) and **4-12-1** (0.1-10 equivalents relative to **4-5-1)** were added to a 100 mL round-bottom flask, and DMF (30 mL) was added. The mixture was cooled in an ice bath to 0 °C, and then DIEA was added. After the mixture was stirred for 5 min, HATU was added. The resulting mixture was stirred at room temperature for 1 h until HPLC indicated the completion of the reaction. The reaction mixture was purified by semi-preparative HPLC to afford compound **4-12-2a** (4.1 g, 69.5% yield, white solid). MS (ESI) of C₁₃₄H₂₀₅N₂₁O₄₅²⁺ [M+2NH₄]²⁺/2: calculated 1414.22, measured 1415.20.

### Step B: Preparation of compound 4-12-2b

At room temperature, **4-12-2a** (957 mg, 0.343 mmol) was added to a 50 mL round-bottom flask, and then DMF (10 mL) and Pd/C were sequentially added. After the system was purged with hydrogen, the mixture was stirred at room temperature for 1.5 h until HPLC indicated the completion of the reaction. The reaction mixture was filtered and then purified by semi-preparative HPLC to afford compound **4-12-2b** (668 mg, 74.4% yield, white solid). MS (ESI) of C₁₂₀H₁₉₀N₂₀O₄₅²⁺ [M+H+NH₄]²⁺/2: calculated 1315.66, measured 1316.51.

### Step C: Preparation of compound 4-12-2d

At room temperature, **4-12-2b** (250 mg, 0.096 mmol) and **4-12-2c** (0.1-10 equivalents relative to **4-12-12b,** CAS No.: 253128-41-5) were added to a 25 mL round-bottom flask, and DMF (5 mL) and DIEA were sequentially added. After the mixture was stirred for 5 min, HATU was added. The resulting mixture was stirred at room temperature for 1 h until HPLC indicated the completion of the reaction. The reaction mixture was purified by semi-preparative HPLC to afford compound **4-12-2d** (311.8 mg, 80.7% yield, white solid). MS (ESI) of C₂₀₀H₃₀₂N₂₁O₆₅³⁺ [M+3H]³⁺/3: calculated 1346.03, measured 1346.95.

### Step D: Preparation of compound 4-12-2

At room temperature, **4-12-2d** (311.8 mg, 0.077 mmol) was added to a 25 mL round-bottom flask, and DMF (4 mL) and diethylamine were sequentially added. The mixture was stirred at room temperature for 20 min until HPLC indicated the completion of the reaction. The reaction mixture was filtered and purified by semi-preparative HPLC to afford compound **4-12-2** (255.8 mg, 72.2% yield, white solid). MS (ESI) of C₁₈₅H₂₉₁N₂₁O₆₃²⁺ [M+2H]²⁺/2: calculated 1907.52, measured 1908.72.

### (3) Preparation of compound 4-12a

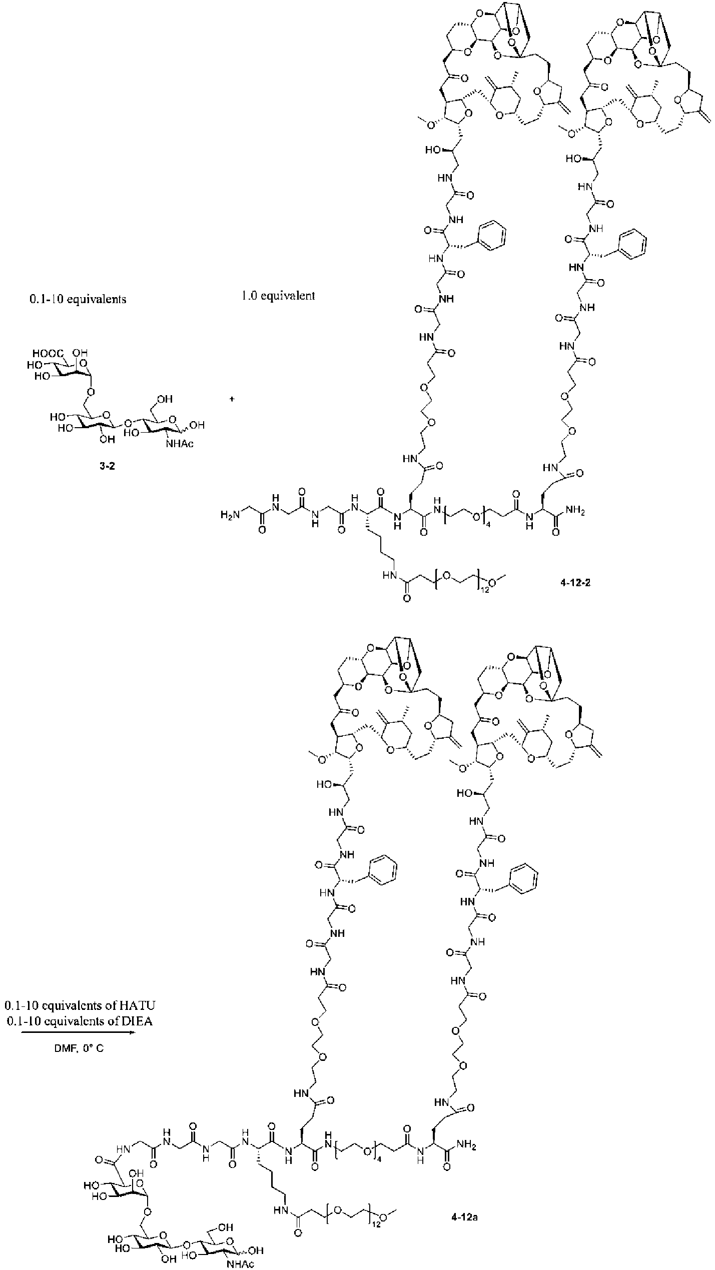

At room temperature, compound **3-2** (0.1-10 equivalents relative to **4-12-2),** compound **4-12-2** (27 mg, 0.007 mmol), DMF (500 µL), and certain amounts of DIPEA and HATU were sequentially added to a 4 mL centrifuge tube, and the resulting reaction mixture was stirred at room temperature until HPLC indicated the completion of the reaction. The reaction mixture was purified by semi-preparative HPLC to afford compound **4-12a** (21.2 mg, 70% yield, white solid). MS (ESI) of C₂₀₅H₃₂₉N₂₄O₇₉³⁺ [M+H+2NH₄]³⁺/3: calculated 1463.75, measured 1464.61.

### (4) Preparation of compound 4-12

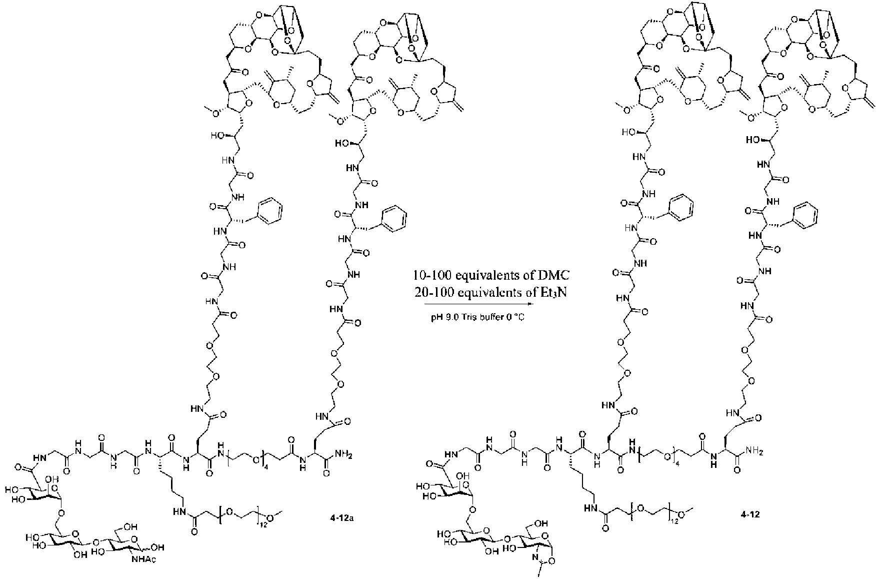

At 0 °C, compound **4-12a** (21.2 mg, 0.005 mmol) and Tris buffer (pH = 9.0, 2 mL) were added to a 15 mL centrifuge tube. After dissolution, certain amounts of triethylamine and 2-chloro-1,3-dimethylimidazolinium chloride were added, and the resulting reaction mixture was stirred at 0 °C until HPLC indicated the completion of the reaction. The reaction mixture was purified by semi-preparative HPLC to afford compound **4-12** (17.87 mg, 85% yield, white solid). MS (ESI) of C₂₀₅H₃₂₇N₂₄O₇₈³⁺ [M+H+2NH₄]³⁺/3: calculated 1457.75, measured 1458.33.

### Example 23: Preparation of linker-payload compound 4-13

The structure of linker-payload compound **4-13** is as follows:

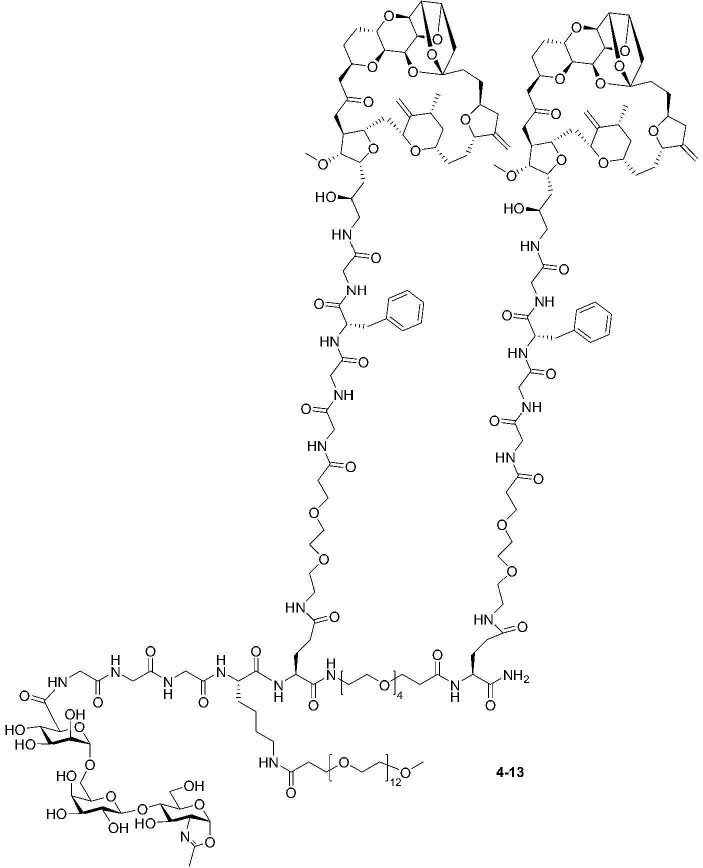

### (1) Preparation of compound 4-13a

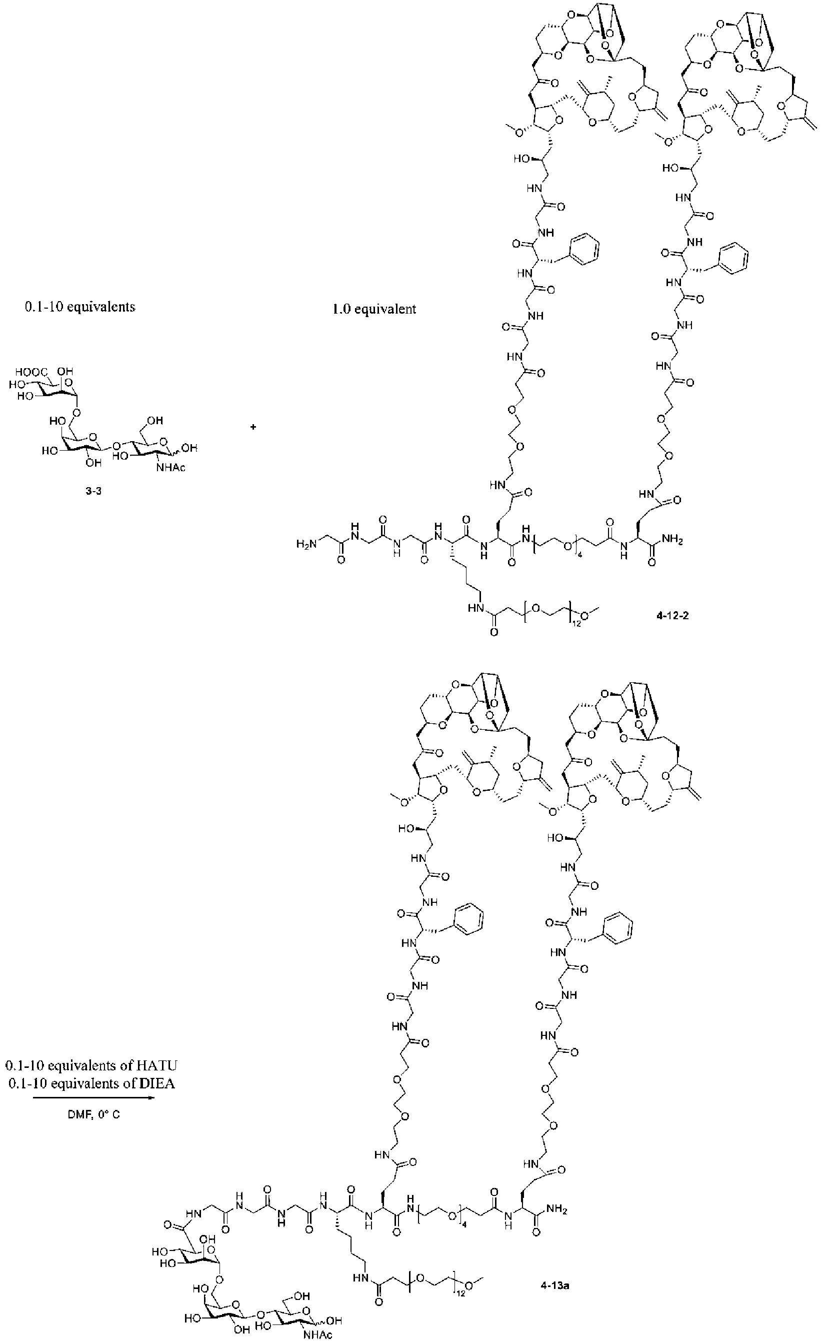

At room temperature, compound **3-3** (0.1-10 equivalents relative to **4-12-2),** compound **4-12-2** (27 mg, 0.007 mmol), DMF (500 µL), and certain amounts of DIPEA and HATU were sequentially added to a 4 mL centrifuge tube, and the resulting reaction mixture was stirred at room temperature until HPLC indicated the completion of the reaction. The reaction mixture was purified by semi-preparative HPLC to afford compound **4-13a** (26.2 mg, 84% yield, white solid). MS (ESI) of C₂₀₅H₃₂₉N₂₄O₇₉³⁺ [M+H+2NH₄]³⁺/3: calculated 1463.75, measured 1464.41.

### (2) Preparation of compound 4-13

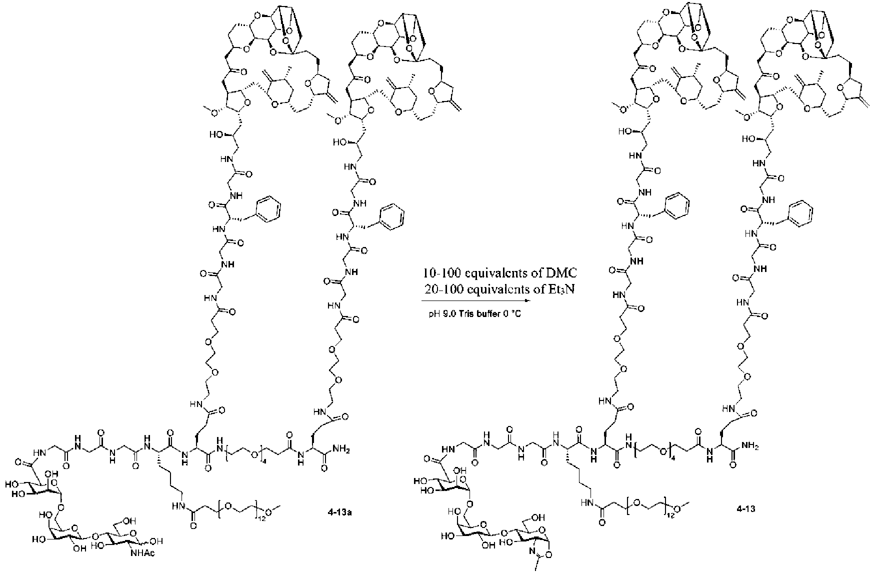

At 0 °C, compound **4-13a** (26.2 mg, 0.006 mmol) and Tris buffer (pH = 9.0, 2 mL) were added to a 15 mL centrifuge tube. After dissolution, certain amounts of triethylamine and 2-chloro-1,3-dimethylimidazolinium chloride were added, and the resulting reaction mixture was stirred at 0 °C until HPLC indicated the completion of the reaction. The reaction mixture was purified by semi-preparative HPLC to afford compound **4-13** (20.01 mg, 77% yield, white solid). MS (ESI) of C₂₀₅H₃₂₇N₂₄O₇₈³⁺ [M+H+2NH₄]³⁺/3: calculated 1457.75, measured 1458.28.

### Example 24: Preparation of linker-payload compound 4-14

The structure of linker-payload compound **4-14** is as follows:

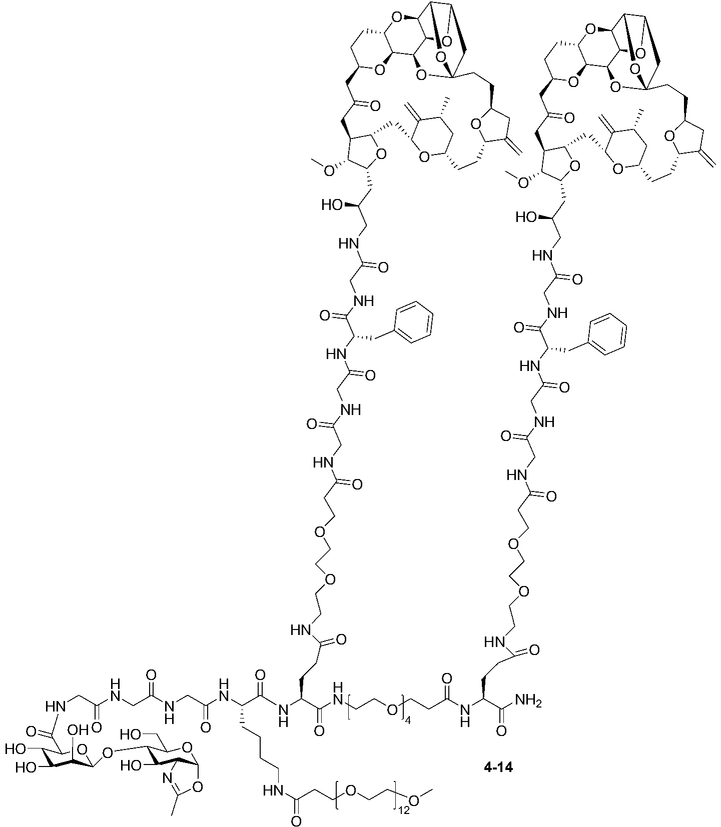

Linker-payload **4-14** was synthesized with procedures similar to those for the synthesis of **4-12.**

### Example 25: Preparation of linker-payload compound 4-15

The structure of linker-payload compound **4-15** is as follows:

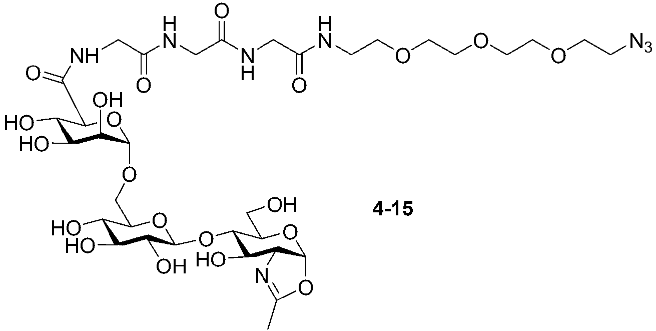

### (1) Preparation of compound 4-15-1

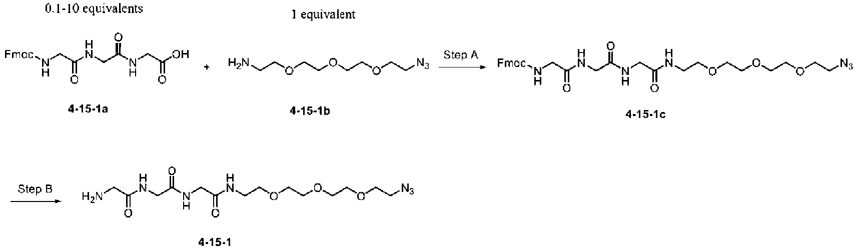

### Step A: Preparation of compound 4-15-1c

At room temperature, **4-15-1a** (0.1-10 equivalents relative to **4-15-1b)** and **4-15-1b** (1 g, 4.582 mmol) were added to a 100 mL round-bottom flask, and DMF (25 mL) was added. The mixture was cooled in an ice bath to 0 °C, and then DIEA was added. After the mixture was stirred for 5 min, HATU was added. The resulting mixture was stirred at room temperature for 1 h until HPLC indicated the completion of the reaction. Water (150 mL) was added to the reaction mixture, and the resulting mixture was washed with ethyl acetate (150 mL × 3). The organic phase was collected, washed with a saturated sodium chloride solution (450 mL), dried over anhydrous sodium sulfate, concentrated at reduced pressure, and then purified by column chromatography (eluent: MeOH/DCM = 1/50-1/15) to afford compound **4-15-1c** (white solid), which was directly used in the next step. MS (ESI) of C₂₉H₄₁N₈O₈⁺ [M+NH₄]⁺: calculated 629.30, measured 629.25.

### Step B: Preparation of compound 4-15-1

At room temperature, **4-15-1c** was added to a 50 mL round-bottom flask, and DMF (15 mL) and diethylamine were sequentially added. The mixture was stirred at room temperature for 20 min until HPLC indicated the completion of the reaction. The reaction mixture was filtered and purified by semi-preparative HPLC to afford compound **4-15-1** (1.32 g, 74.2% yield, white solid). MS (ESI) of C₁₄H₂₈N₇O₆⁺ [M+H]⁺: calculated 390.21, measured 390.25.

### (2) Preparation of compound 4-15a

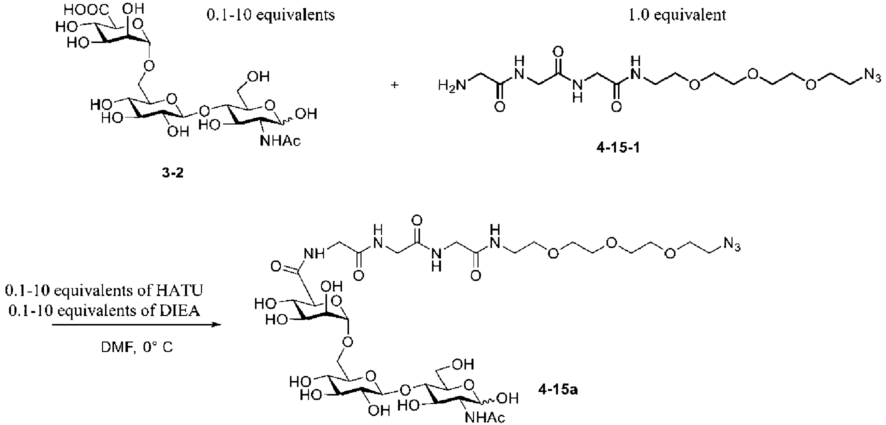

At room temperature, compound **3-2** (0.1-10 equivalents relative to **4-15-1),** compound **4-15-1** (30 mg, 0.077 mmol), DMF (1 mL), and certain amounts of DIPEA and HATU were sequentially added to a 4 mL centrifuge tube, and the resulting reaction mixture was stirred at room temperature until HPLC indicated the completion of the reaction. The reaction mixture was purified by semi-preparative HPLC to afford compound **4-15a** (57.4 mg, 80% yield, white solid). MS (ESI) of C₃₄H₆₂N₉O₂₂⁺ [M+NH₄]⁺: calculated 948.40, measured 948.45.

### (3) Preparation of compound 4-15

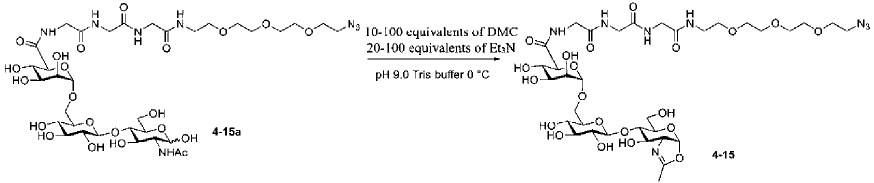

At 0 °C, compound **4-15a** (57.4 mg, 0.062 mmol) and Tris buffer (pH = 9.0, 2 mL) were added to a 15 mL centrifuge tube. After dissolution, certain amounts of triethylamine and 2-chloro-1,3-dimethylimidazolinium chloride were added, and the resulting reaction mixture was stirred at 0 °C until HPLC indicated the completion of the reaction. The reaction mixture was purified by semi-preparative HPLC to afford compound **4-15** (41.1 mg, 73% yield, white solid). MS (ESI) of C₃₄H₅₇N₈O₂₁⁺ [M+H]⁺: calculated 913.36, measured 913.38.

### III. Selection or preparation of antibodies

### Selection of antibodies

For the antibody-conjugated drugs prepared using the substrate and the conjugation method of the present disclosure, any molecule containing antibody Fc region with a glycan chain can be selected for the macromolecular moiety thereof, including but not limited to antibodies, diabodies, Fc fusion proteins, single-chain antibodies, and the like. For example, trastuzumab, which is commercially available, can be selected.

In addition, the antibody can also be an engineered antibody. For the production, purification, and identification of the anti-human ErbB2/HER2 antibody mAb-1, reference can be made to Example 1 of Patent CN 106856656 B, which is incorporated herein by reference in its entirety.

### IV. Preparation and characterization of ADCs

### Example 26: Preparation and characterization of ADC-1

### 26.1 Preparation of antibody-conjugated drug ADC-1

In the following description, the glycan chain at the Fc terminus of antibody mAb-1 was remodeled using endoglycosidases, and the linker-payload was specifically conjugated to the antibody to form a corresponding ADC drug.
1) Processing of antibody mAb-1: The antibody was processed via ultrafiltration, dialysis, or desalting column chromatography, and the storage buffer was replaced with 50 mM Tris-HCl (pH 5-8) and 150 mM NaCl.
2) Preparation of ADC-1: The conjugation reaction between the antibody mAb-1 and the linker-payload compound **4-1** was catalyzed by endoglycosidase Endo S2 to prepare ADC-1. In a 1× endonuclease buffer, the antibody mAb-1 and compound **4-1** were thoroughly mixed in an appropriate molar ratio (1:1 to 1:100), and then endoglycosidase Endo S2 was added. The mixture was thoroughly mixed. In the thoroughly mixed state, the conjugating reaction was conducted at 4-40 °C for 0.5-20 h. After the reaction was completed, purification was performed. The purified ADC-1 was stored in 1 × PBS (pH 7.4) at 4 °C or -80 °C.

The characterization and detection methods and results for the antibody-conjugated drug ADC-1 are described in 26.2-26.3 below.

### 26.2 Detection and analysis of antibody-conjugated drug ADC-1 by hydrophobic interaction chromatography-high performance liquid chromatography (HIC-HPLC)

A Proteomix HIC Butyl-NP5 4.6 × 35 mm, 5 µm Non-Porous chromatographic column (manufacturer: Sepax, PN: 431NP5-4603) was used; 1.5 M ammonium sulfate + 20 mM phosphate buffer (pH 7.0) was used as mobile phase A; 20 mM phosphate buffer (pH 7.0):isopropanol = 7:3 (v/v) was used as mobile phase B; the flow rate was 0.8 mL/min; gradient: phase B was increased from 10% to 70% within 8 min. 280 nm was selected as the detection wavelength to detect the DAR distribution of the ADC drug ADC-1.

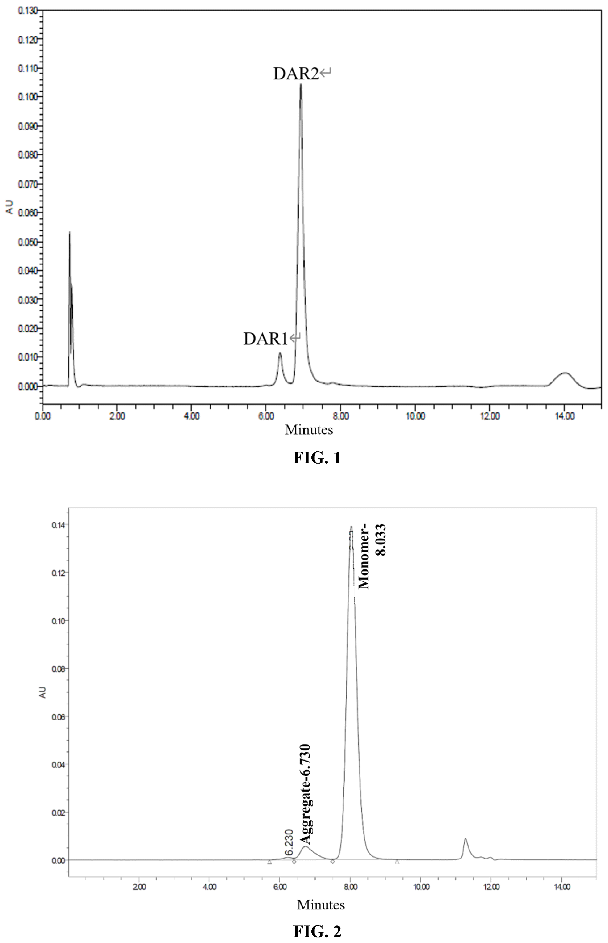

The detection results are shown in FIG. 1. The content of unconjugated antibody was < 5%; the conjugation product was predominantly DAR2, and the overall DAR of the ADC drug was about 1.88 (in a preferred conjugation condition).

### 26.3 Detection and analysis of ADC-1 by size exclusion chromatography-high performance liquid chromatography (SEC-HPLC)

A TSKgel G3000SWXL 7.8 mm I.D. × 30 cm, 5 µm chromatographic column (manufacturer: Tosoh, PN: 0008541) was used, 2× PBS:acetonitrile = 9:1 (v/v) was used as the mobile phase, the isocratic run was performed at room temperature for 15 min, and the flow rate was 1.0 mL/min. 280 nm was selected as the detection wavelength to analyze and detect the degree of high molecular weight aggregation of the ADC drug.

The detection results are shown in FIG. 2. The content of high molecular weight aggregates in the ADC drug was < 6%, the monomer predominated in the ADC samples (8.03 min), and the damage to the antibody caused by the conjugation reaction was almost negligible.

### Example 27: Preparation and characterization of ADC-2

### 27.1 Preparation of antibody-conjugated drug ADC-2

In the following description, the glycan chain at the Fc terminus of antibody mAb-1 was remodeled using endoglycosidases, and the linker-payload was specifically conjugated to the antibody to form a corresponding ADC drug.
1) Processing of antibody mAb-1: The antibody was processed via ultrafiltration, dialysis, or desalting column chromatography, and the storage buffer was replaced with 50 mM Tris-HCl (pH 5-8) and 150 mM NaCl.
2) Preparation of ADC-2: The conjugation reaction between the antibody mAb-1 and the linker-payload compound **4-2** was catalyzed by endoglycosidase Endo S2 to prepare ADC-2. In a 1× endonuclease buffer, the antibody mAb-1 and compound **4-2** were thoroughly mixed in an appropriate molar ratio (1:1 to 1:100), and then endoglycosidase Endo S2 was added. The mixture was thoroughly mixed. In the thoroughly mixed state, the conjugating reaction was conducted at 4-40 °C for 0.5-20 h. After the reaction was completed, purification was performed. The purified ADC-2 was stored in 1 × PBS (pH 7.4) at 4 °C or -80 °C.

The characterization and detection methods and results for the antibody-conjugated drug ADC-2 are described in 27.2-27.3 below.

### 27.2 Detection and analysis of antibody-conjugated drug ADC-2 by hydrophobic interaction chromatography-high performance liquid chromatography (HIC-HPLC)

A Proteomix HIC Butyl-NP5 4.6 × 100 mm, 5 µm Non-Porous chromatographic column (manufacturer: Sepax, PN: 431NP5-4610) was used; 1.5 M ammonium sulfate + 20 mM phosphate buffer, pH 7.0, was used as mobile phase A; 20 mM phosphate buffer, pH 7.0:isopropanol = 8:2 (v/v) was used as mobile phase B; the flow rate was 0.8 mL/min; gradient: phase B was increased from 10% to 100% within 8 min; 280 nm was selected as the detection wavelength to detect the DAR distribution of the ADC drug.

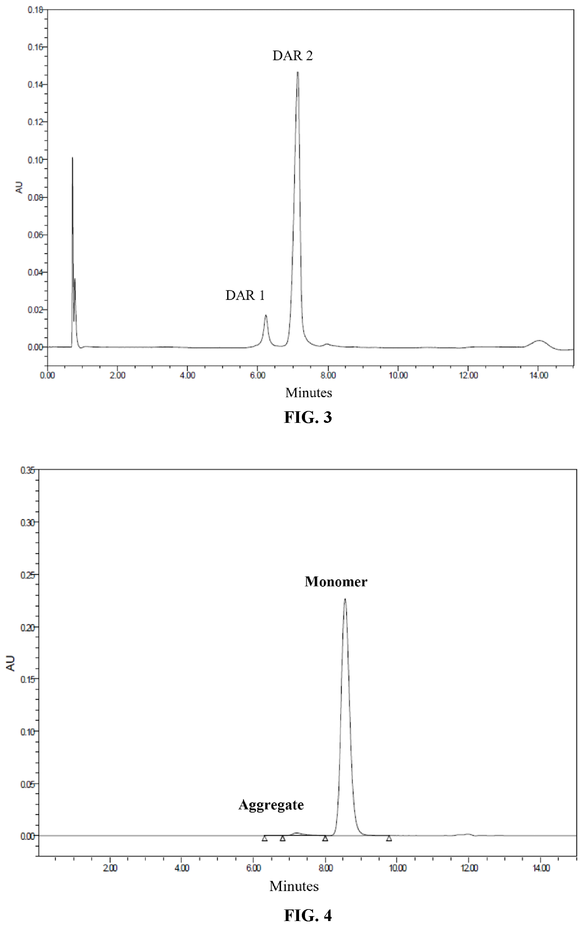

The detection results are shown in FIG. 3. The content of unconjugated antibody was < 1%; the conjugation product was predominantly DAR2, and the overall DAR of the ADC drug was about 1.90.

### 27.3 Detection and analysis of ADC-2 by size exclusion chromatography-high performance liquid chromatography (SEC-HPLC)

A BioCore SEC-300 5 µm, 7.8 × 300 mm chromatographic column (PN: B213-050030-07830S) was used; 2× PBS:acetonitrile = 9:1 (v/v) was used as the mobile phase; room temperature; the isocratic run was performed for 15 min, and the flow rate was 1.0 mL/min; 280 nm was selected as the detection wavelength to analyze and detect the degree of high molecular weight aggregation of the ADC drug.

The detection results are shown in FIG. 4. The content of high molecular weight aggregates in the ADC drug was < 2%, the monomer predominated in the ADC samples (8.54 min), and the damage to the antibody caused by the conjugation reaction was almost negligible.

### Example 28: Preparation and characterization of ADC-3

The preparation and characterization were performed with reference to the method described in Example 27, except that the linker-payload used was 4-3. The characterization data of the antibody-conjugated drug ADC-3 are as described below.

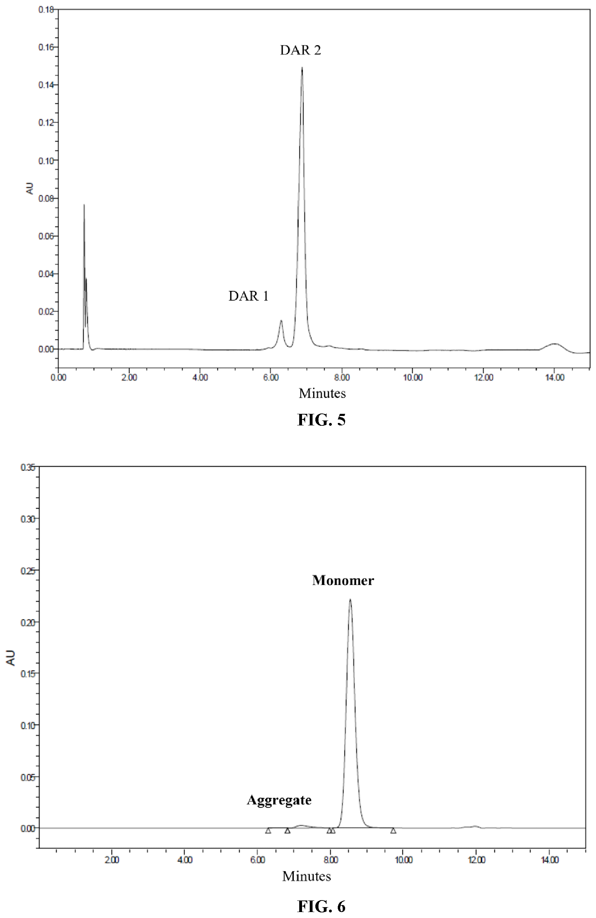

ADC-3 was subjected to hydrophobic interaction chromatography-high performance liquid chromatography (HIC-HPLC), and the results are shown in FIG. 5. The content of unconjugated antibody was < 1%; the conjugation product was predominantly DAR2, and the overall DAR of the ADC drug was about 1.90.

ADC-3 was subjected to size exclusion chromatography-high performance liquid chromatography (SEC-HPLC), and the results are shown in FIG. 6. The content of high molecular weight aggregates in the ADC drug was < 2%, the monomer predominated in the ADC samples (8.53 min), and the damage to the antibody caused by the conjugation reaction was almost negligible.

### Example 29: Preparation and characterization of ADC-4

The preparation and characterization were performed with reference to the method described in Example 27, except that the linker-payload used was **4-4.** The characterization data of the antibody-conjugated drug ADC-4 are as described below.

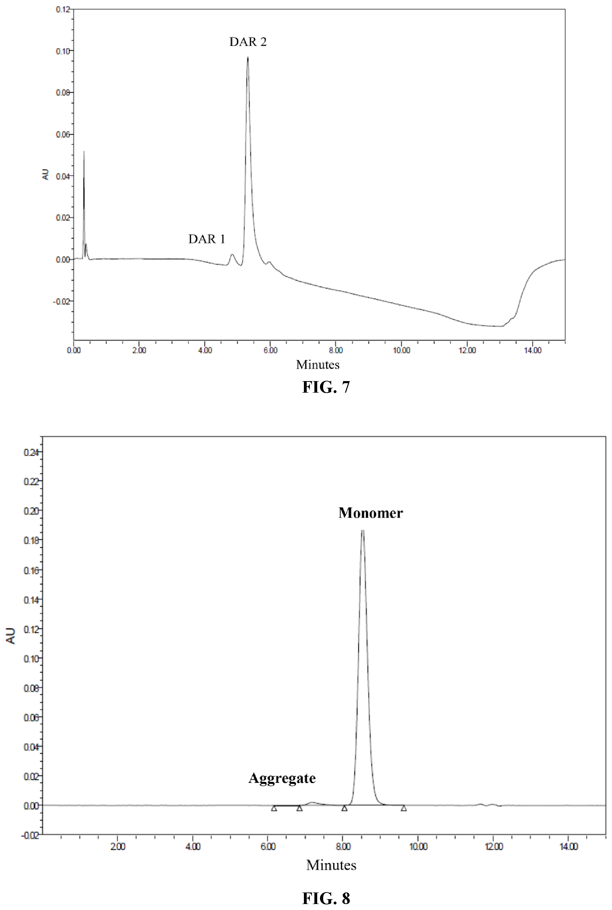

ADC-4 was subjected to hydrophobic interaction chromatography-high performance liquid chromatography (HIC-HPLC), and the results are shown in FIG. 7. The content of unconjugated antibody was < 1%; the conjugation product was predominantly DAR2, and the overall DAR of the ADC drug was about 1.95.

ADC-4 was subjected to size exclusion chromatography-high performance liquid chromatography (SEC-HPLC), and the results are shown in FIG. 8. The content of high molecular weight aggregate in the ADC drugs was < 2%, the monomer predominated in the ADC samples (8.53 min), and the damage to the antibody caused by the conjugation reaction was almost negligible.

### Example 30: Preparation and characterization of ADC-5

The preparation and characterization were performed with reference to the method described in Example 27, except that the linker-payload used was **4-5.** The characterization data of the antibody-conjugated drug ADC-5 are as described below.

ADC-5 was subjected to hydrophobic interaction chromatography-high performance liquid chromatography (HIC-HPLC). The content of unconjugated antibody was < 1%; the conjugation product was predominantly DAR4, and the overall DAR of the ADC drug was about 3.82.

ADC-5 was subjected to size exclusion chromatography-high performance liquid chromatography (SEC-HPLC). The content of high molecular weight aggregates in the ADC drug was < 3%, the monomer predominated in the ADC samples (8.55 min), and the damage to the antibody caused by the conjugation reaction was almost negligible.

### Example 31: Preparation and characterization of ADC-6

The preparation and characterization were performed with reference to the method described in Example 27, except that the linker-payload used was **4-6.** The characterization data of the antibody-conjugated drug ADC-6 are as described below.

ADC-6 was subjected to hydrophobic interaction chromatography-high performance liquid chromatography (HIC-HPLC). The content of unconjugated antibody was < 1%; the conjugation product was predominantly DAR4, and the overall DAR of the ADC drug was about 3.91.

ADC-6 was subjected to size exclusion chromatography-high performance liquid chromatography (SEC-HPLC). The content of high molecular weight aggregates in the ADC drug was < 3%, the monomer predominated in the ADC samples (8.54 min), and the damage to the antibody caused by the conjugation reaction was almost negligible.

### Example 32: Preparation and characterization of ADC-7

The preparation and characterization were performed with reference to the method described in Example 27, except that the linker-payload used was **4-7.** The characterization data of the antibody-conjugated drug ADC-7 are as described below.

ADC-7 was subjected to hydrophobic interaction chromatography-high performance liquid chromatography (HIC-HPLC). The content of unconjugated antibody was < 1%; the conjugation product was predominantly DAR4, and the overall DAR of the ADC drug was about 3.98.

ADC-7 was subjected to size exclusion chromatography-high performance liquid chromatography (SEC-HPLC). The content of high molecular weight aggregates in the ADC drug was < 3%, the monomer predominated in the ADC samples (8.53 min), and the damage to the antibody caused by the conjugation reaction was almost negligible.

### Example 33: Preparation and characterization of ADC-8

The preparation and characterization were performed with reference to the method described in Example 27, except that the linker-payload used was **4-8.** The characterization data of the antibody-conjugated drug ADC-8 are as described below.

ADC-8 was subjected to hydrophobic interaction chromatography-high performance liquid chromatography (HIC-HPLC). The content of unconjugated antibody was < 1%; the conjugation product was predominantly DAR4, and the overall DAR of the ADC drug was about 3.87.

ADC-8 was subjected to size exclusion chromatography-high performance liquid chromatography (SEC-HPLC). The content of high molecular weight aggregates in the ADC drug was < 3%, the monomer predominated in the ADC samples (8.52 min), and the damage to the antibody caused by the conjugation reaction was almost negligible.

### Example 34: Preparation and characterization of ADC-9

The preparation and characterization were performed with reference to the method described in Example 27, except that the linker-payload used was **4-9.** The characterization data of the antibody-conjugated drug ADC-9 are as described below.

ADC-9 was subjected to hydrophobic interaction chromatography-high performance liquid chromatography (HIC-HPLC). The content of unconjugated antibody was < 1%; the conjugation product was predominantly DAR4, and the overall DAR of the ADC drug was about 3.76.

ADC-9 was subjected to size exclusion chromatography-high performance liquid chromatography (SEC-HPLC). The content of high molecular weight aggregates in the ADC drug was < 2%, the monomer predominated in the ADC samples (8.53 min), and the damage to the antibody caused by the conjugation reaction was almost negligible.

### Example 35: Preparation and characterization of ADC-10

The preparation and characterization were performed with reference to the method described in Example 27, except that the linker-payload used was **4-10.** The characterization data of the antibody-conjugated drug ADC-10 are as described below.

ADC-10 was subjected to hydrophobic interaction chromatography-high performance liquid chromatography (HIC-HPLC). The content of unconjugated antibody was < 2%; the conjugation product was predominantly DAR4, and the overall DAR of the ADC drug was about 3.72.

ADC-10 was subjected to size exclusion chromatography-high performance liquid chromatography (SEC-HPLC). The content of high molecular weight aggregates in the ADC drug was < 3%, the monomer predominated in the ADC samples (8.51 min), and the damage to the antibody caused by the conjugation reaction was almost negligible.

### Example 36: Preparation and characterization of ADC-11

The preparation and characterization were performed with reference to the method described in Example 27, except that the linker-payload used was **4-11.** The characterization data of the antibody-conjugated drug ADC-11 are as described below.

ADC-11 was subjected to hydrophobic interaction chromatography-high performance liquid chromatography (HIC-HPLC). The content of unconjugated antibody was < 1%; the conjugation product was predominantly DAR4, and the overall DAR of the ADC drug was about 3.86.

ADC-11 was subjected to size exclusion chromatography-high performance liquid chromatography (SEC-HPLC). The content of high molecular weight aggregates in the ADC drug was < 2%, the monomer predominated in the ADC samples (8.52 min), and the damage to the antibody caused by the conjugation reaction was almost negligible.

### Example 37: Preparation and characterization of ADC-12

The preparation and characterization were performed with reference to the method described in Example 27, except that the linker-payload used was **4-12.** The characterization data of the antibody-conjugated drug ADC-12 are as described below.

ADC-12 was subjected to hydrophobic interaction chromatography-high performance liquid chromatography (HIC-HPLC). The content of unconjugated antibody was < 1%; the conjugation product was predominantly DAR4, and the overall DAR of the ADC drug was about 3.64.

ADC-12 was subjected to size exclusion chromatography-high performance liquid chromatography (SEC-HPLC). The content of high molecular weight aggregates in the ADC drug was < 2%, the monomer predominated in the ADC samples (8.56 min), and the damage to the antibody caused by the conjugation reaction was almost negligible.

### Example 38: Preparation and characterization of ADC-13

The preparation and characterization were performed with reference to the method described in Example 27, except that the linker-payload used was **4-13.** The characterization data of the antibody-conjugated drug ADC-13 are as described below.

ADC-13 was subjected to hydrophobic interaction chromatography-high performance liquid chromatography (HIC-HPLC). The content of unconjugated antibody was < 1%; the conjugation product was predominantly DAR4, and the overall DAR of the ADC drug was about 3.74.

ADC-13 was subjected to size exclusion chromatography-high performance liquid chromatography (SEC-HPLC). The content of high molecular weight aggregates in the ADC drug was < 2%, the monomer predominated in the ADC samples (8.55 min), and the damage to the antibody caused by the conjugation reaction was almost negligible.

### Example 39: Preparation and characterization of ADC-14

The preparation and characterization were performed with reference to the method described in Example 27, except that the linker-payload used was **4-14.** The characterization data of the antibody-conjugated drug ADC-14 are as described below.

ADC-14 was subjected to hydrophobic interaction chromatography-high performance liquid chromatography (HIC-HPLC). The content of unconjugated antibody was < 1%; the conjugation product was predominantly DAR4, and the overall DAR of the ADC drug was about 3.84.

ADC-14 was subjected to size exclusion chromatography-high performance liquid chromatography (SEC-HPLC). The content of high molecular weight aggregates in the ADC drug was < 2%, the monomer predominated in the ADC samples (8.56 min), and the damage to the antibody caused by the conjugation reaction was almost negligible.

### Example 40: Preparation and characterization of ADC-15

The preparation and characterization were performed with reference to the method described in Example 27, except that the linker-payload used was **4-15.** The characterization data of the antibody-conjugated drug ADC-15 are as described below.

ADC-15 was subjected to hydrophobic interaction chromatography-high performance liquid chromatography (HIC-HPLC). The content of unconjugated antibody was < 1%; the conjugation product was predominantly DAR2, and the overall DAR of the ADC drug was about 1.92.

ADC-15 was subjected to size exclusion chromatography-high performance liquid chromatography (SEC-HPLC). The content of high molecular weight aggregate in the ADC drugs was < 2%, the monomer predominated in the ADC samples (8.53 min), and the damage to the antibody caused by the conjugation reaction was almost negligible.

### V. In vitro activity assay for ADCs

### Example 41. In vitro activity assay for ADC-1, ADC-2, ADC-3, and ADC-4 (SKBR-3, NCI-N87, and MDA-MB-468)

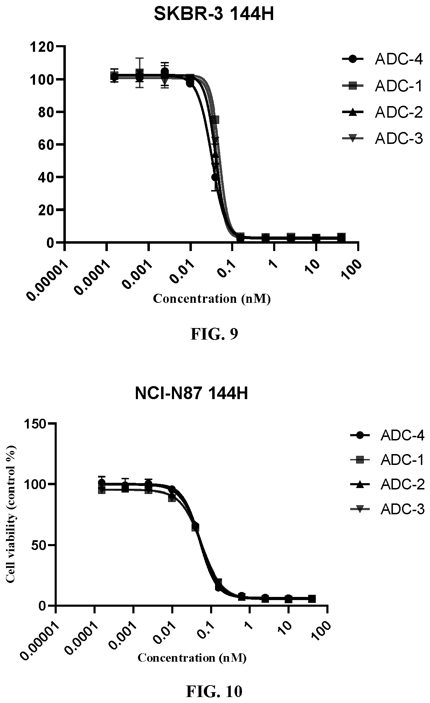

The effects of ADC-1, ADC-2, ADC-3, and ADC-4 on the proliferation of tumor cells with different HER2 expression levels were tested according to the following procedures:
1) HER2-positive human breast cancer cells SKBR-3, HER2-positive human gastric cancer cells NCI-N87, and HER2-negative human breast cancer cells MDA-MB-468 were seeded into a 96-well cell plate at 100 µL per well (containing 1000-10,000 cells) and cultured overnight in a cell incubator in the conditions of 37 °C, 5% CO₂, 95% air, and 100% humidity.
2) ADC-1, ADC-2, ADC-3, and ADC-4 at different concentrations (40, 10, 2.5, 0.625, 0.156, 0.039, 0.00977, 0.00244, 0.00061, and 0.00015) were added to the HER2-positive cells and HER2-negative cells cultured overnight, and an untreated group and a 5 µM puromycin group were set as controls for 100% cell viability and 0% cell viability, respectively. After treatment, the cells were placed in the cell incubator and incubated at 37 °C for another 72-120 h.
3) The cell plate was taken out of the cell incubator and equilibrated to room temperature. 100 µL of a CellTiter Glo reagent was added to each well. The plate was shaken on a shaker for 2 min and then left to stand in the dark at room temperature for 10 min. The cold luminescence value (RLU) was measured using a Cytation 3 microplate reader.
4) The results of the inhibitory effects of different drugs against the proliferation of tumor cells are shown in Table 1 and FIGs. 9-11, wherein ADC-1, ADC-2, ADC-3, and ADC-4 exhibited significant inhibitory effects against the proliferation of HER2-positive cells, while no significant difference in efficacy was found between disaccharide ADC-4 and trisaccharides ADC-1, ADC-2, and ADC-3 in the case of identical DARs. ADC-1, ADC-2, ADC-3, and ADC-4 exhibited no inhibitory effects against HER2-negative cells.

**Table 1. Inhibitory effects of different drugs against proliferation of tumor cells (IC₅₀, nM)**

| | ADC-1 | ADC-2 | ADC-3 | ADC-4 |
|---|---|---|---|---|
| SKBR-3 | 0.04495 | 0.03978 | 0.04304 | 0.03202 |
| NCI-N87 | 0.05671 | 0.05407 | 0.05671 | 0.05117 |
| MDA-MB-468 | - | - | - | - |
| Note: "-" indicates not detected. | | | | |

### Example 42. In vitro activity assay for ADC-5, ADC-6, ADC-7, and ADC-8 (SKBR-3, NCI-N87, and MDA-MB-468)

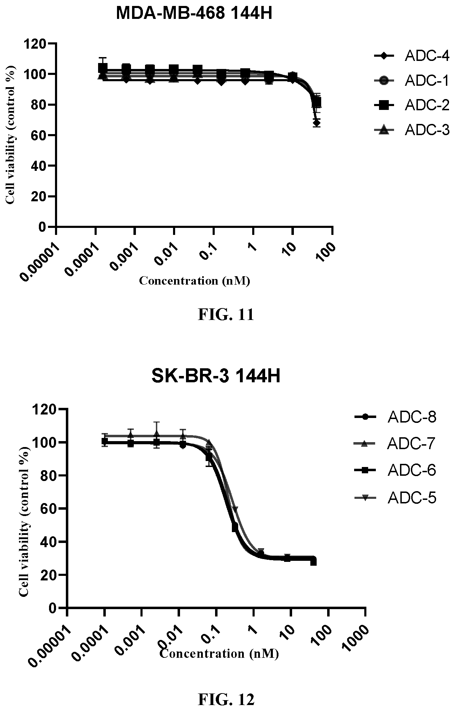

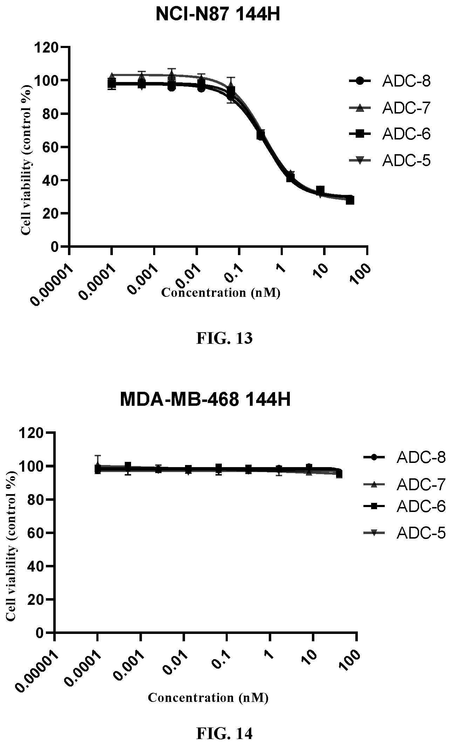

The effects of ADC-5, ADC-6, ADC-7, and ADC-8 on the proliferation of tumor cells with different HER2 expression levels were tested according to the following procedures:
With reference to the method described in Example 41, the effects of antibody-conjugated drugs ADC-5, ADC-6, ADC-7, and ADC-8 on the proliferation of tumor cells with different HER2 expression levels were tested. The results of the inhibitory effects of different drugs against the proliferation of tumor cells are shown in Table 2 and FIGs. 12-14, wherein ADC-5, ADC-6, ADC-7, and ADC-8 exhibited significant inhibitory effects against the proliferation of HER2-positive cells, while no significant difference in efficacy was found between disaccharide ADC-8 and trisaccharides ADC-5, ADC-6, and ADC-7 in the case of identical DARs. ADC-5, ADC-6, ADC-7, and ADC-8 exhibited no inhibitory effects against HER2-negative cells.

**Table 2. Inhibitory effects of different drugs against proliferation of tumor cells (IC₅₀, nM)**

| | ADC-5 | ADC-6 | ADC-7 | ADC-8 |
|---|---|---|---|---|
| SKBR-3 | 0.2415 | 0.1809 | 0.207 | 0.1955 |
| NCI-N87 | 0.4454 | 0.3952 | 0.385 | 0.3826 |
| MDA-MB-468 | - | - | - | - |
| Note: "-" indicates not detected. | | | | |

### Example 43. In vitro activity assay for ADC-9, ADC-10, and ADC-11 (SKBR-3, NCI-N87, and MDA-MB-468)

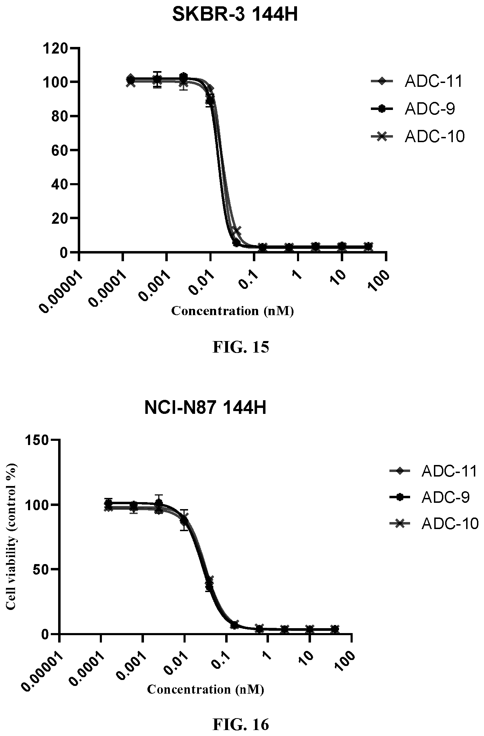

The effects of ADC-9, ADC-10, and ADC-11 on the proliferation of tumor cells with different HER2 expression levels were tested according to the following procedures:
With reference to the method described in Example 41, the effects of antibody-conjugated drugs ADC-9, ADC-10, and ADC-11 on the proliferation of tumor cells with different HER2 expression levels were tested. The results of the inhibitory effects of different drugs against the proliferation of tumor cells are shown in Table 3 and FIGs. 15-17, wherein ADC-9, ADC-10, and ADC-11 exhibited significant inhibitory effects against the proliferation of HER2-positive cells, while no significant difference in efficacy was found between disaccharide ADC-11 and trisaccharides ADC-9 and ADC-10 in the case of identical DARs. ADC-9, ADC-10, and ADC-11 exhibited no inhibitory effects against HER2-negative cells.

**Table 3. Inhibitory effects of different drugs against proliferation of tumor cells (IC₅₀, nM)**

| | ADC-9 | ADC-10 | ADC-11 |
|---|---|---|---|
| SKBR-3 | 0.01545 | 0.01925 | 0.0185 |
| NCI-N87 | 0.02701 | 0.03206 | 0.0317 |
| MDA-MB-468 | - | - | - |
| Note: "-" indicates not detected. | | | |

### Example 44. In vitro activity assay for ADC-12, ADC-13, and ADC-14 (SKBR-3, NCI-N87, and MDA-MB-468)

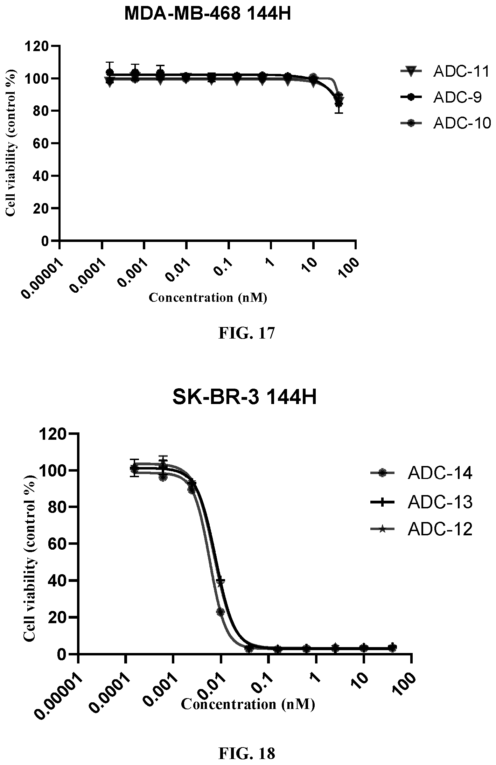

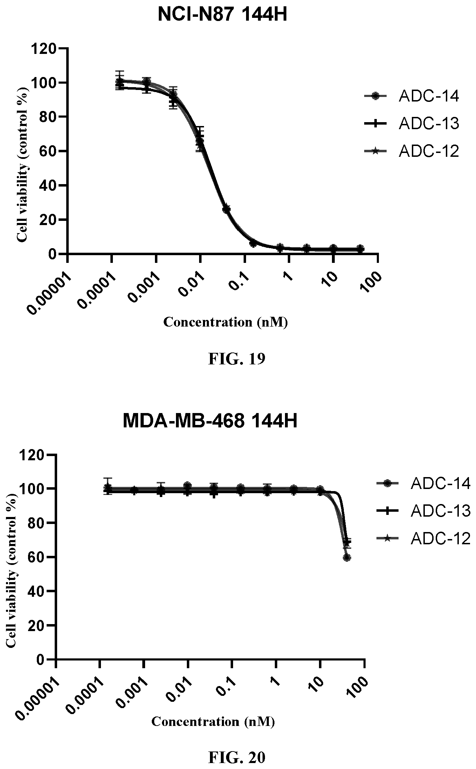

The effects of ADC-12, ADC-13, and ADC-14 on the proliferation of tumor cells with different HER2 expression levels were tested according to the following procedures:
With reference to the method described in Example 41, the effects of antibody-conjugated drugs ADC-12, ADC-13, and ADC-14 on the proliferation of tumor cells with different HER2 expression levels were tested. The results of the inhibitory effects of different drugs against the proliferation of tumor cells are shown in Table 4 and FIGs. 18-20, wherein ADC-12, ADC-13, and ADC-14 exhibited significant inhibitory effects against the proliferation of HER2-positive cells, while no significant difference in efficacy was found between disaccharide ADC-14 and trisaccharides ADC-12 and ADC-13 in the case of identical DARs. ADC-12, ADC-13, and ADC-14 exhibited no inhibitory effects against HER2-negative cells.

**Table 4. Inhibitory effects of different drugs against proliferation of tumor cells (IC₅₀, nM)**

| | ADC-12 | ADC-13 | ADC-14 |
|---|---|---|---|
| SKBR-3 | 0.007199 | 0.007704 | 0.005805 |
| NCI-N87 | 0.01488 | 0.01752 | 0.01513 |
| MDA-MB-468 | - | - | - |
| Note: "-" indicates not detected. | | | |

### Example 45. Affinity assay of ADC-5, ADC-6, ADC-7, and ADC-8 for cell surface HER2 (NCI-N87)

The antigen-binding rates of ADC-5, ADC-6, ADC-7, and ADC-8 to NCI-N87 cells were detected according to the following procedures:
1. Preparation of cell samples
   1) Cell collection: After adherent cells were detached from the culture flask using a 0.25% trypsin cell digestion solution, the cell suspension was transferred into a 15 mL centrifuge tube. The cell suspension was centrifuged at 1000 rpm for 5 min, and the supernatant was discarded. The cells were resuspended in an appropriate amount of FACS buffer. The cell viability was determined using trypan blue staining.
   2) Cell dilution: An appropriate amount of cell suspension was added to FACS buffer to prepare 10 mL of cell suspension with a concentration of 1-2 × 10⁶ cells/mL.
2. Binding of cells to antibodies and ADC drugs
   1) The cells were seeded on a V-bottom plate. Prior to plating, the cell suspension was inverted to thoroughly mix the cells several times, and 100 µL of cell suspension was added to each well. The cell control group was treated with 100 µL of FACS buffer only, while the other groups were treated with 100 µL of antibody solution. The mixtures were immediately incubated in an ice bath for 60 min.
   2) Washing unbound antibodies or ADCs: The cell pellet was collected by centrifugation at 2000 rpm at 4 °C for 3 min, and the supernatant was discarded. The cells were resuspended in 200 µL/well FACS buffer on an ice bath. The cell pellet was collected by centrifugation again at 2000 rpm at 4 °C for 3 min, and the supernatant was discarded.
   3) Secondary antibody binding: The cells were resuspended in 500-fold diluted Goat anti-Human IgG (H+L), Superclonal^{™} Recombinant Secondary Antibody, and Alexa Fluor^{™} Plus 647 at 100 µL/well and incubated in an ice bath in the dark for 60 min. Unbound antibodies were washed by repeating step 2. The cells were resuspended in a 100 µL/well of buffer and stored at 4 °C in the dark for detection on a flow cytometer.
3. Cytometry
   1) Appropriate acquisition parameter setting
   2) Cell sample injection
   3) Analysis using FACSDiva software

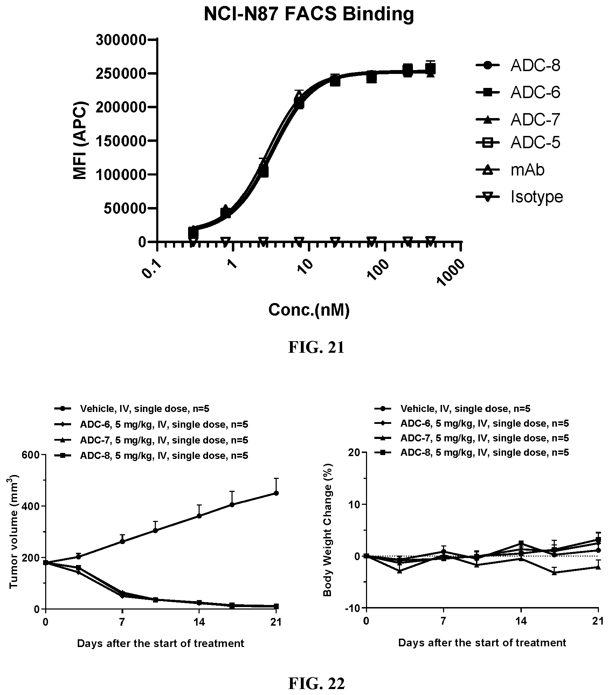

The results of binding of different drugs to tumor cells are shown in Table 5 and FIG. 21. On NCI-N87 cells, disaccharide ADC-8, trisaccharides ADC-5, ADC-6, and ADC-7, and monoclonal antibody OL1302 exhibited comparable binding affinities and concentration-dependent profiles. The negative control antibody Isotype exhibited no significant specific binding to NCI-N87 cells.

**Table 5. Binding affinity of different drugs for NCI-N87 cells (EC₅₀, nM)**

| | ADC-5 | ADC-6 | ADC-7 | ADC-8 | OL1302 | Isotype |
|---|---|---|---|---|---|---|
| NCI-N87 | 3.282 | 3.127 | 3.334 | 3.149 | 2.849 | - |
| Note: "-" indicates not detected. | | | | | | |

### VI. In vivo activity assay for ADCs

### Example 46. In vivo efficacy evaluation of ADC drugs ADC-6, 7, and 8

1) NCI-N87 gastric cancer cells in the logarithmic growth phase were collected, and the cell density was adjusted to 10 × 10⁶ cells/mL using a Matrigel buffer (PBS:Matrigel = 1:1). Each Balb/c nude mouse was inoculated subcutaneously at the right scapula with 0.2 mL of the prepared NCI-N87 cell suspension.
2) The diameters of tumors were measured with a vernier caliper, and the tumor volume was calculated according to the formula V = 0.5 × a × b² (wherein a is the longest diameter of the tumor, and b is the shortest diameter of the tumor). On day 6 after cell inoculation, the mice were grouped for administration when the mean tumor volume reached 180 mm³. The animals were randomized into a vehicle control group, a 5 mg/kg ADC-6 group, a 5 mg/kg ADC-7 group, and a 5 mg/kg ADC-8 group, with 5 animals in each group, and were then administered via tail vein injection. The tumor volumes of the animals in each group were measured twice a week after treatment, and the tumor volumes of the animals on day 21 were compared. The T/C (%) value and TGI (%) value were calculated on the basis of the tumor volume. The calculation formula is as follows: T/C% = T_{RTV}/C_{RTV} × 100% (T_{RTV}: RTV of treatment group; C_{RTV}: RTV of vehicle control group). The relative tumor volume (RTV) was calculated on the basis of tumor measurement. The calculation formula is RTV = Vt/V₀, wherein V₀ is the mean tumor volume measured at the time of grouping (i.e., D₀), and Vt is the mean tumor volume at a certain measurement. T_{RTV} and C_{RTV} were calculated using data on the same day. Calculation of TGI (%): TGI (%) = [1 - (mean tumor volume at the end of treatment in a treatment group - mean tumor volume at the start of treatment in the treatment group)/(mean tumor volume at the end of treatment in the vehicle control group - mean tumor volume at the start of treatment in the vehicle control group)] × 100%.
3) On day 21 of treatment, the results of the inhibitory effects of different drugs against xenograft tumors of the mice are shown in Table 6 and FIG. 22. The mean tumor volume of the vehicle control group was 450 mm³, and the mean tumor volumes of the 5 mg/kg ADC-6 group, the 5 mg/kg ADC-7 group, and the 5 mg/kg ADC-8 group were 12 mm³, 10 mm³, and 12 mm³, respectively. It can be seen that all the three ADC drugs exhibited significant and superior antitumor effects, while no significant difference in efficacy was found between ADC-6, ADC-7, and ADC-8.

**Table 6. Inhibitory effects of ADC-6, ADC-7, and ADC-8 against xenograft tumors in NCI-N87 mice**

| **Treatment group** | **Tumor volume (mm³)^{a}** | **Tumor volume (mm³)** | **T/C^{b}** | **TGI^{c}** | ***P* value^{d}** |
|---|---|---|---|---|---|
| | **Day 0** | **Day 21** | **(%)** | **(%)** | |
| Vehicle | 180 ± 8 | 450 ± 58 | - | - | - |
| ADC-6 (5 mg/kg) | 180 ± 8 | 12 ± 3 | 2.77 | 162.00 | <0.0001 |
| ADC-7 (5 mg/kg) | 180 ± 8 | 10 ± 1 | 2.23 | 162.90 | <0.0001 |
| ADC-8 (5 mg/kg) | 180 ± 8 | 12 ± 2 | 2.68 | 162.14 | <0.0001 |

| | | | | | |
|---|---|---|---|---|---|
| a. Mean ± SEM; b. T/C % = T_{RTV}/C_{RTV} × 100% (T_{RTV}: RTV of treatment group; C_{RTV}: RTV of vehicle control group). The relative tumor volume (RTV) was calculated on the basis of tumor measurement. The calculation formula is RTV = V₂₁/V₀, wherein V₀ is the mean tumor volume measured at the time of grouping (i.e., D₀), and V₂₁ is the mean tumor volume on day 21 of treatment. T_{RTV} and C_{RTV} were calculated using data on the same day; c. $TGI \left(%\right) = \left[1-\left({T}_{21}-{T}_{0}\right)/\left({V}_{21}-{V}_{0}\right)\right] \times 100 ;$ d. The statistical analysis was performed by means of One-way ANOVA. | | | | | |

### VII. ADCC activity assay for ADCs

### Example 47. ADCC activity evaluation of ADC-5, 6, 7, and 8

The antibody-dependent cell-mediated cytotoxicity (ADCC) functionality is closely related to the affinity of FcγR series receptors of antibodies, and is directly affected by the glycoform structure at position N-297 in the antibody Fc region. Through enzyme-catalyzed glycosyl conjugation reactions, oligosaccharide linkers can be introduced to remodel the glycosyl structure of the antibody Fc region, thereby regulating the ADCC function of the resulting ADC molecule. In view of the difference between the trisaccharide structure and the existing disaccharide structure, this example is intended to test the difference between the ADCC activity of the ADCs obtained using the trisaccharide linkers of the present disclosure and the activity of the disaccharide linker.

The experiment used HER2-positive SK-BR-3 cells as the target cell and human primary NK cells as the effector cell. The two were combined to form a cell co-culture system in a ratio of 1:5 (SK-BR-3:NK). After the effector cells and the target cells were co-incubated with the test samples for 4 h, the ADCC effects of the drugs of interest were reflected by detecting the release of LDH.

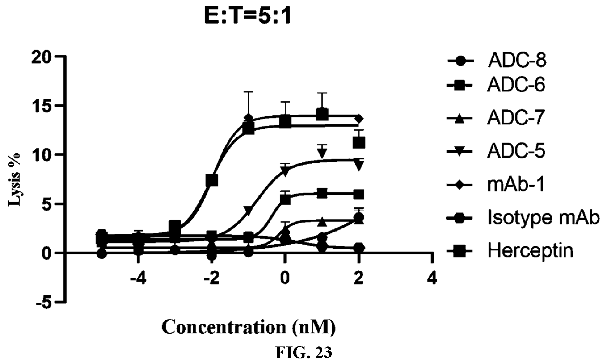

The results showed that the ADCC functions of ADCs with trisaccharide linker structures were stronger than that of the ADC with the disaccharide linker structure, and different glycoforms exhibited different degrees of effects on the ADCC function. The specific results are shown in FIG. 23.

Sequence Listing

SEQ ID No. 1: mAb-1 light chain:
DIQMTQSPSSLSASVGDRVTITCRASQDVNTAVAWYQQKPGKAPKLLIYSASFLYSG
VPSRFSGSRSGTDFTLTISSLQPEDFATYYCQQHYTTPPTFGQGTKVEIKRTVAAPSVFI
FPPSDEQLKSGTASVVCLLNNFYPREAKVQWKVDNALQSGNSQESVTEQDSKDSTYS
LSSTLTLSKADYEKHKVYACEVTHQGLSSPVTKSFNRGECGALPETGG

SEQ ID No. 2: mAb-1 heavy chain:
EVQLVESGGGLVQPGGSLRLSCAASGFNIKDTYIHWVRQAPGKGLEWVARIYPTNGY
TRYADSVKGRFTISADTSKNTAYLQMNSLRAEDTAVYYCSRWGGDGFYAMDYWGQ
GTLVTVSSASTKGPSVFPLAPSSKSTSGGTAALGCLVKDYFPEPVTVSWNSGALTSGV
HTFPAVLQSSGLYSLSSVVTVPSSSLGTQTYICNVNHKPSNTKVDKKVEPKSCDKTHT
CPPCPAPELLGGPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKA
KGQPREPQVYTLPPSREEMTKNQVSLTCLVKGFYPSDIAVEWESNGQPENNYKTTPP
VLDSDGSFFLYSKLTVDKSRWQQGNVFSCSVMHEALHNHYTQKSLSLSPGK

SEQ ID No. 3: GGGGS

SEQ ID No. 4: GGGGSGGGGS

SEQ ID No. 5: GGGGSGGGGSGGGGS

## Claims

1. A linker-payload compound, having the structure of formula (I):
D-L-(P)ₜ formula (I)
wherein
P is the payload;
L is a linker, and L is directly linked to D via the terminal -NH-, wherein when L is an unbranched linker, it is linked to one P, and t is 1, and when L is a branched linker, each branch can be linked to one P, and t is an integer greater than 1 (e.g., 2, 3, 4, 5, 6, 7, 8, 9, or 10);
D is a trisaccharide unit structure comprising a first hexose unit or a derivative moiety thereof, a second hexose unit or a derivative moiety thereof, and a third hexose unit or a derivative moiety thereof;
the -OH at position 6 of the first hexose unit or the derivative moiety thereof is oxidized to - C(O)- to link to the terminal -NH- of L;
the first hexose unit or the derivative moiety thereof is linked to the second hexose unit or the derivative moiety thereof via an α-(1→6) glycosidic bond;
the second hexose unit or the derivative moiety thereof is linked to the third hexose unit or the derivative moiety thereof via a β-(1→4) glycosidic bond;
the third hexose unit or the derivative moiety thereof has the following structure:

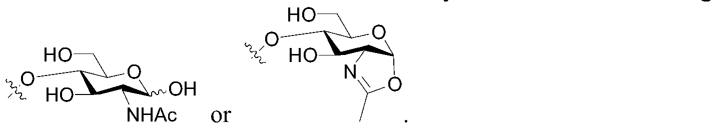

2. The linker-payload compound according to claim 1, wherein
the first hexose unit or the derivative moiety thereof is selected from glucosyl, mannosyl, galactosyl, fructosyl, gulosyl, idosyl, and derivatives thereof.

3. The linker-payload compound according to claim 2, wherein the first hexose unit or the derivative moiety thereof is selected from 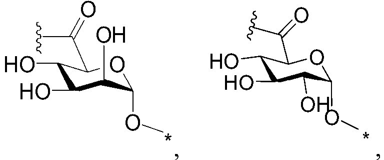 and 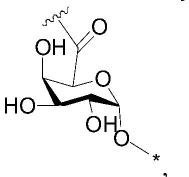 wherein 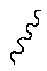 denotes the site for linking to L; * denotes the site for linking to the second hexose unit or the derivative moiety thereof.

4. The linker-payload compound according to claim 1,
wherein the second hexose unit or the derivative moiety thereof is selected from glucosyl, mannosyl, galactosyl, fructosyl, and derivatives thereof.

5. The linker-payload compound according to claim 4, wherein the second hexose unit or the derivative moiety thereof is selected from 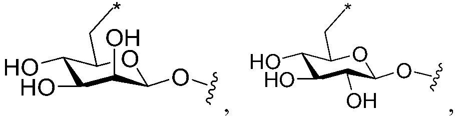 and 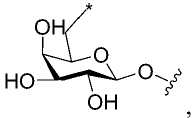 wherein 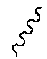 denotes the site for linking to the third hexose unit or the derivative moiety thereof; * denotes the site for linking to the first hexose unit or the derivative moiety thereof.

6. The linker-payload compound according to claim 1, wherein the trisaccharide unit structure has the following structure:

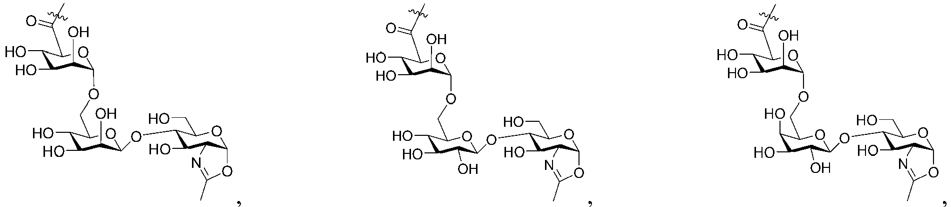

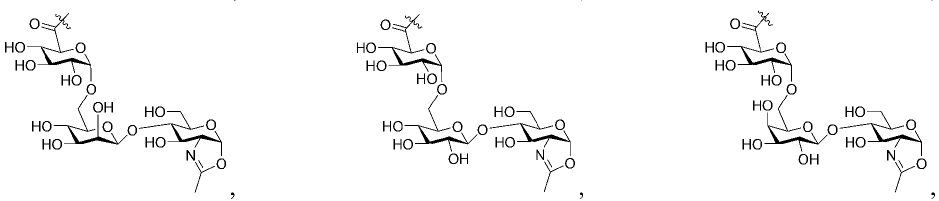

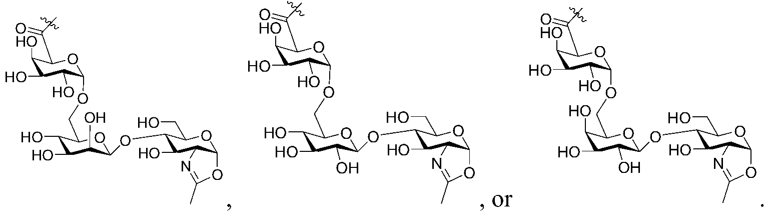

7. The linker-payload compound according to claim 1, wherein
-L-(P)t is -L²-L¹-B-P; that is, formula (I) is:
D-L²-L¹-B-P formula (I-1)
wherein
each B is independently absent, or is 1), 2), or the combination of 1) and 2) below: 1) a self-immolative spacer Sp1; and 2) one divalent group, or a combination of two or more divalent groups, wherein the divalent group is selected from: -CR¹R²-, C₁₋₁₀ alkylene, C₄₋₁₀ cycloalkylene, C₄₋₁₀ heterocyclylene, and -(CO)-;
each L¹ is independently absent, or is a non-cleavable sequence, or is a cleavable sequence comprising an enzymatically cleavable amino acid sequence, wherein the enzymatically cleavable amino acid sequence comprises 1, 2, 3, 4, 5, 6, 7, 8, 9, or 10 amino acids;
each L² is independently absent, or is 1), 2), or the combination of 1) and 2) below:
1) -NH-C₂₋₂₀ alkylene, wherein one or more -CH₂- structures in the alkylene are optionally substituted by the following groups: -CR³R⁴-, -O-, -(CO)-, -S-, -S(=O)₂-, -NR⁵-, -N⊕R⁶R⁷-, C₄₋₁₀ cycloalkylene, C₄₋₁₀ heterocyclylene, and phenylene, wherein the cycloalkylene, heterocyclylene, and phenylene are each independently unsubstituted or substituted with at least one substituent selected from halogen, -C₁₋₁₀ alkyl, -C₁₋₁₀ haloalkyl, -C₁₋₁₀ alkylene-NH-R⁸, and -C₁₋₁₀ alkylene-O-R⁹; and
2) an amino acid residue sequence, i.e., -*(AA)ₙ**-, wherein n is an integer of 1-100, AA is each independently an amino acid residue, * denotes the N-terminus of a corresponding amino acid, ** denotes the C-terminus of a corresponding amino acid, and -(C₂H₄-O)ₘ-(CH₂)ₚ- is optionally present between the amino group and the α-carbon of one amino acid, wherein m is 1, 2, 3, 4, 5, 6, 7, 8, 9, or 10; p is 0, 1, 2, or 3, and the * terminus forms an amide bond with the carbonyl group in the trisaccharide structure,
wherein, B, L¹, and L² are not simultaneously absent;
R¹, R², R³, R⁴, R⁵, R⁶, R⁷, R⁸, and R⁹ are each independently selected from hydrogen, halogen, substituted or unsubstituted -C₁₋₁₀ alkyl, and C₄₋₁₀ cycloalkylene; or R¹ and R², together with the carbon atom to which they are attached, form a 3-6 membered cycloalkylene, and/or R³ and R⁴, together with the carbon atom to which they are attached, form a 3-6 membered cycloalkylene;
P is the payload linked to B moiety, L¹ moiety, or L² moiety;
D is as defined in claim 1.

8. The linker-payload compound according to claim 1, wherein -L-(P)ₜ is 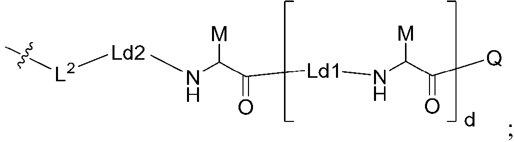 that is, formula (I) is: 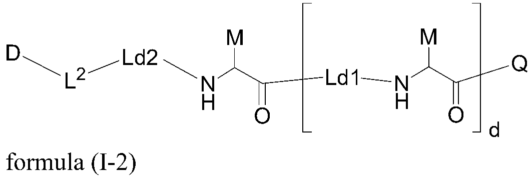 wherein,
Ld2 and each Ld1 are independently a bond, or are selected from -NH-C₁₋₂₀ alkylene-(CO)- and -NH-(PEG)ᵢ-(CO)-, or are natural amino acids each independently unsubstituted or substituted with -CO-(PEG)ⱼ-R¹¹ on the side chain or oligomeric natural amino acids with a degree of polymerization of 2-10 (i.e., 2, 3, 4, 5, 6, 7, 8, 9, or 10);
the -(PEG)ᵢ- and -(PEG)ⱼ- are each a PEG fragment comprising a specified number of consecutive -(O-C₂H₄)- structural units or consecutive -(C₂H₄-O)- structural units, optionally with an additional C₁₋₁₀ alkylene at one terminal;
M is hydrogen or LKa-L²-L¹-B-P;
Q is NH₂ or L²-L¹-B-P;
provided that the following condition is excluded: M is hydrogen and Q is NH₂;
each LKa is independently selected from 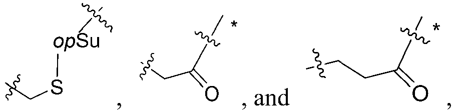
*opSu* is 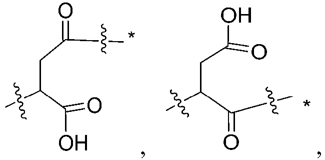 or a mixture thereof, wherein * denotes the linker moiety to L²;
wherein D, L², L¹, and B are as defined in claim 7;
R¹¹ is C₁₋₁₀ alkyl;
d is 0, 1, 2, 3, 4, 5, or 6;
each i is independently an integer of 1-100 (e.g., 1, 2, 3, 4, 5, 6, 7, 8, 9, or 10);
each j is independently an integer of 1-100 (e.g., 1, 2, 3, 4, 5, 6, 7, 8, 9, or 10).

9. The linker-payload compound according to claim 7 or 8, wherein L² is an amino acid residue sequence, i.e., -*(AA)ₙ**-, wherein n is an integer of 1-100, AA is each independently an amino acid residue, * denotes the N-terminus of a corresponding amino acid, ** denotes the C-terminus of a corresponding amino acid, and -(C₂H₄-O)ₘ-(CH₂)ₚ- is optionally present between the amino group and the α-carbon of one amino acid, wherein m is 1, 2, 3, 4, 5, 6, 7, 8, 9, or 10; p is 0, 1, 2, or 3, and the * terminus forms an amide bond with the carbonyl group in the trisaccharide structure.

10. The linker-payload compound according to claim 9, wherein AA is each independently any one of Phe, Lys, Gly, Ala, Leu, Asn, Val, Ile, Pro, Trp, Ser, Tyr, Cys, Met, Asp, Gln, Glu, Thr, Arg, and His, or any combination thereof.

11. The linker-payload compound according to claim 7 or 8, wherein n is an integer of 1-50, preferably an integer of 1-30, preferably an integer of 1-20, preferably an integer of 1-10, and preferably 1, 2, 3, 4, 5, 6, 7, 8, 9, or 10.

12. The linker-payload compound according to claim 7 or 8, wherein L¹ is a cleavable sequence comprising an enzymatically cleavable amino acid sequence, and the enzymatically cleavable amino acid sequence comprises 1, 2, 3, 4, 5, 6, 7, 8, 9, or 10 amino acids; preferably, L¹ is any one of Val, Cit, Phe, Lys, Gly, Ala, Leu, and Asn, or any combination thereof, more preferably -Val-Ala-, -Gly-Gly-Phe-Gly-, -Phe-Lys-, -Val-Cit-, -Val-Lys-, -Gly-Phe-Leu-Gly-, -Ala-Leu-Ala-Leu-, -Ala-Ala-Ala-, or a combination thereof, and even more preferably -Val-Cit- or -Gly-Gly-Phe-Gly-.

13. The linker-payload compound according to claim 7 or 8, wherein B is absent or is selected from: 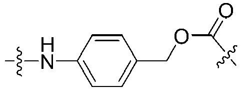 (-PABC-), -NH-CH₂-U-, and -NH-CH₂-U-(CH₂)_{g}-(CO)-, wherein g is 1, 2, 3, 4, 5, or 6; U is absent, or is CH₂, O, S, or NH, preferably O or S.

14. The linker-payload compound according to claim 7 or 8, wherein -L¹-B- denotes -Val-Cit-PABC-, -Gly-Gly-Phe-Gly-, or -Gly-Gly-Phe-Gly-NH-CH₂-U-, wherein U is absent, or is O, S, or NH, preferably O or S.

15. The linker-payload compound according to claim 7 or 8, wherein L² is an amino acid residue sequence, i.e., -*(AA)ₙ**-, wherein n is an integer of 1-10, AA is each independently an amino acid residue, * denotes the N-terminus of a corresponding amino acid, ** denotes the C-terminus of a corresponding amino acid, and -(C₂H₄-O)ₘ-(CH₂)ₚ- is optionally present between the amino group and the α-carbon of one amino acid, wherein m is 1, 2, 3, 4, 5, 6, 7, 8, 9, or 10; p is 0, 1, 2, or 3, and the * terminus forms an amide bond with the carbonyl group in the trisaccharide structure; preferably, -L²- is -Gly-Gly-Gly-; or
-L²- is -NH-C₂₋₂₀ alkylene, wherein one or more -CH₂- structures in the alkylene are optionally substituted by the following group: -O- or -(CO)-.

16. The linker-payload compound according to any one of claims 1-15, wherein the payload P is selected from the group consisting of a small molecule compound, a nucleic acid and nucleic acid analog, a tracer molecule, a short peptide, a polypeptide, a peptidomimetic, and a protein.

17. The linker-payload compound according to claim 7, wherein the linker-payload compound is selected from: 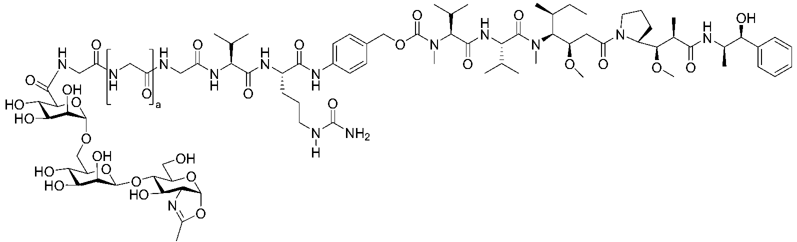 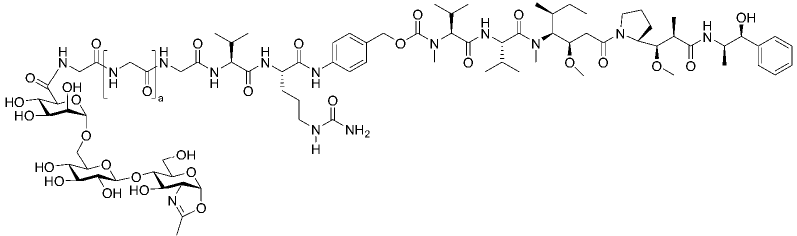 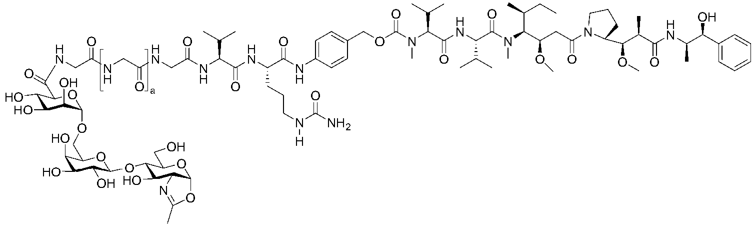 each a is independently 0, 1, 2, 3, 4, or 5.

18. The linker-payload compound according to claim 8, wherein the linker-payload compound is selected from: 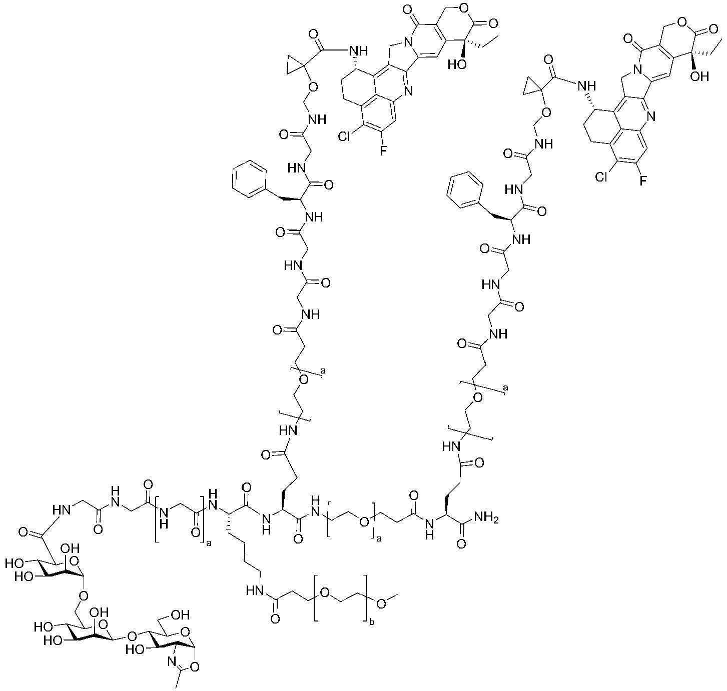 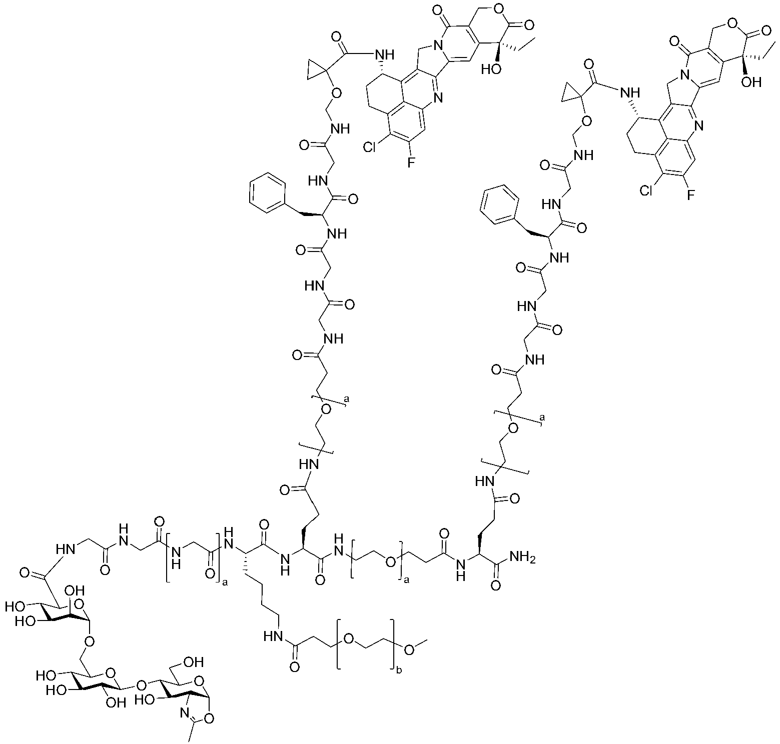 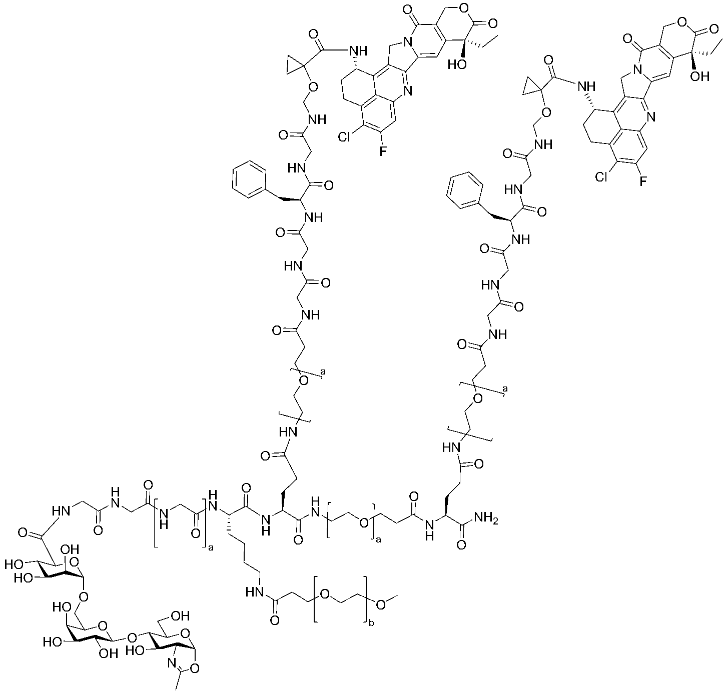 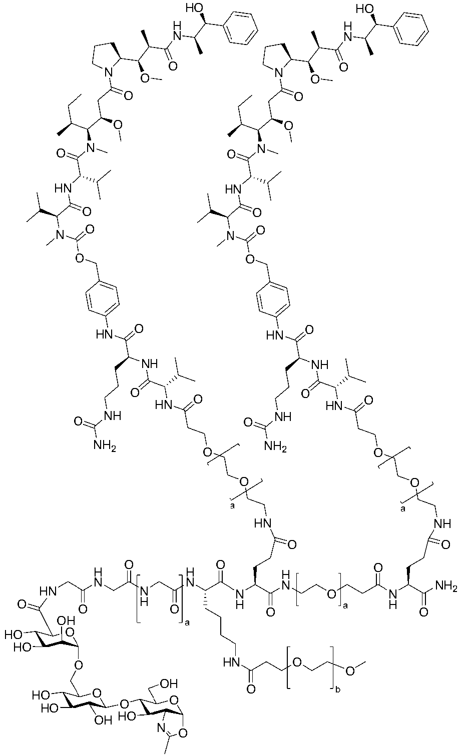 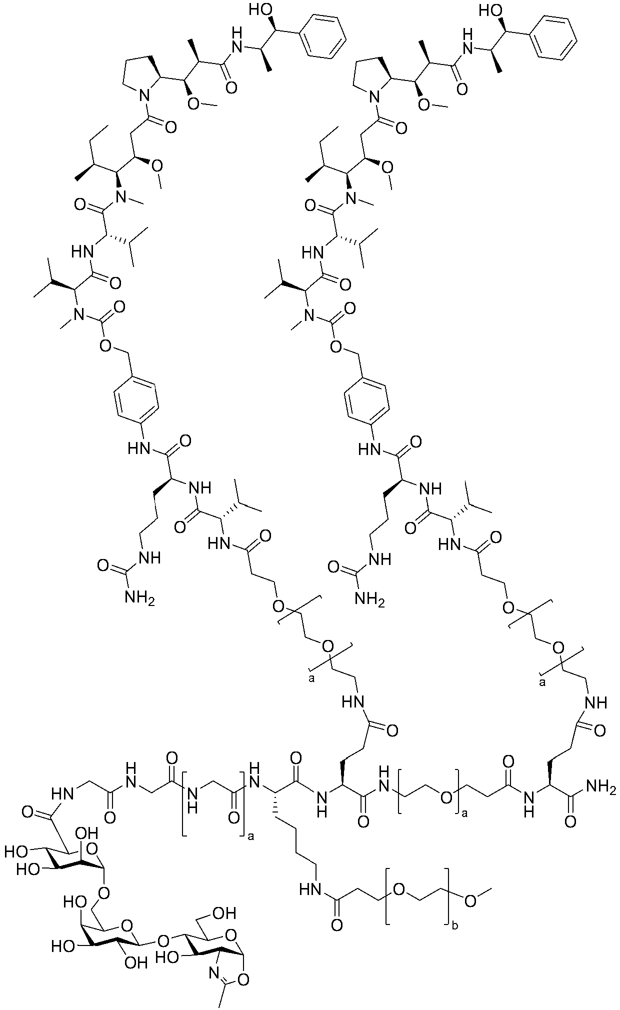 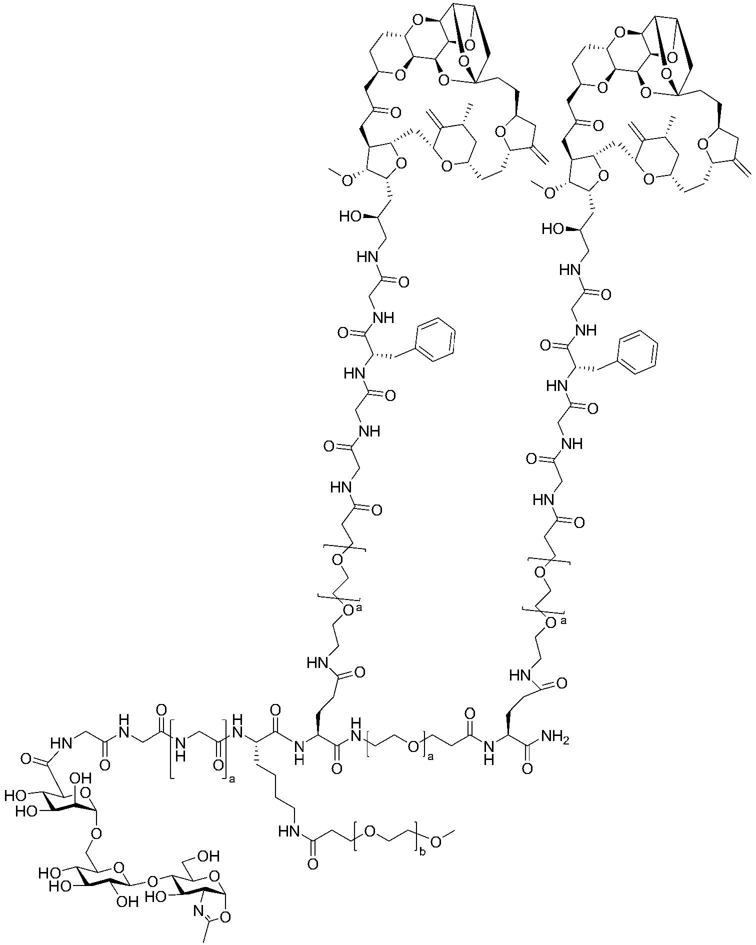 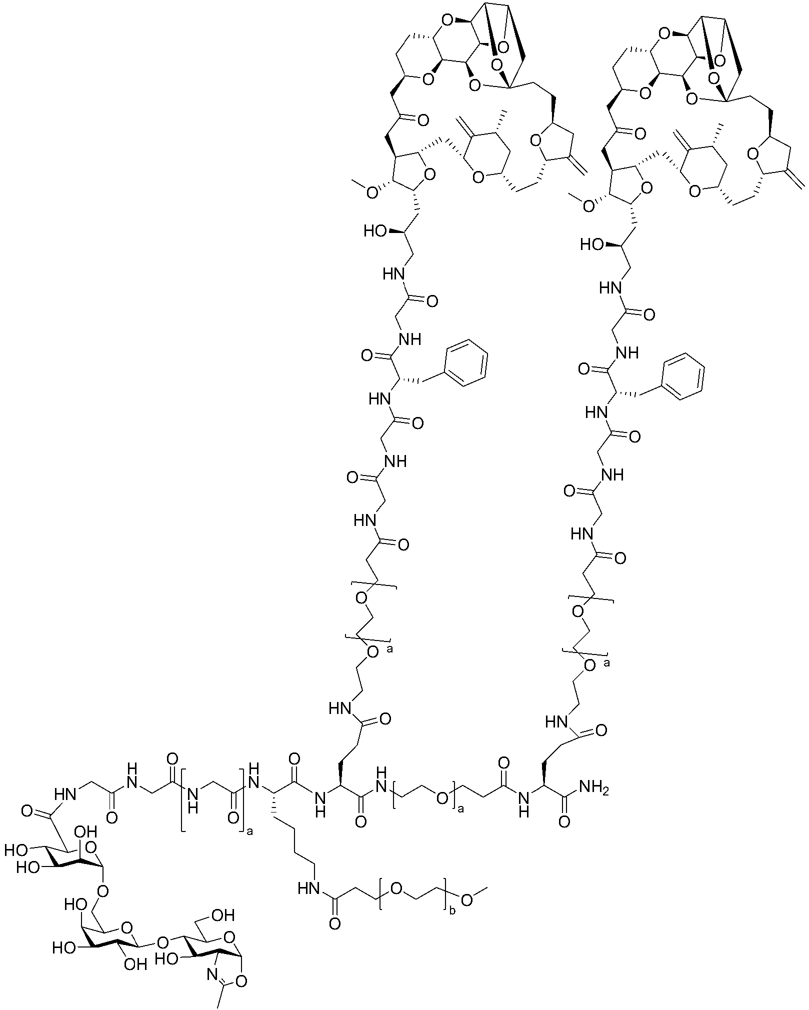
each a is independently 0, 1, 2, 3, 4, or 5;
each b is independently selected from integers of 1-30, preferably integers of 1-15, and more preferably 6, 7, 8, 9, 10, 11, and 12.

19. An antibody-conjugated drug based on site-specific linkage at an N-glycosylation site in an antibody Fc region, having the structure of formula (II): 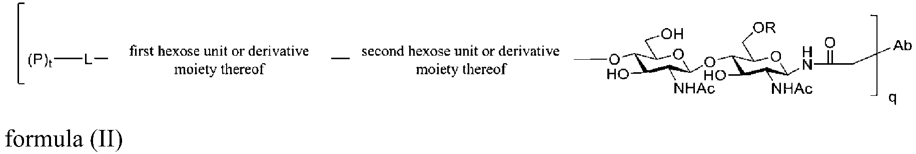 wherein
P is the payload;
R is hydrogen or α-L-fucosyl;
q is 1 or 2;
Ab is an antibody or an antigen-binding fragment;
the first hexose unit or the derivative moiety thereof is as defined in claim 2 or 3; and/or
the second hexose unit or the derivative moiety thereof is as defined in claim 4 or 5; and/or
the derivative is independently selected from a uronic acid and a monosaccharide with hydroxyl being substituted by an acylamino; and/or
the first hexose unit or the derivative moiety thereof is linked to the second hexose unit or the derivative moiety thereof via an α-(1→6) glycosidic bond;
the other monosaccharide moieties are linked via β-(1→4) glycosidic bonds;
-L-(P)t is as defined in any one of claims 1-18.

20. The antibody-conjugated drug according to claim 19, wherein formula (II) is selected from formula (II-1), (II-2), (II-3), (II-4), (II-5), (II-6), (II-7), (II-8), and (II-9): 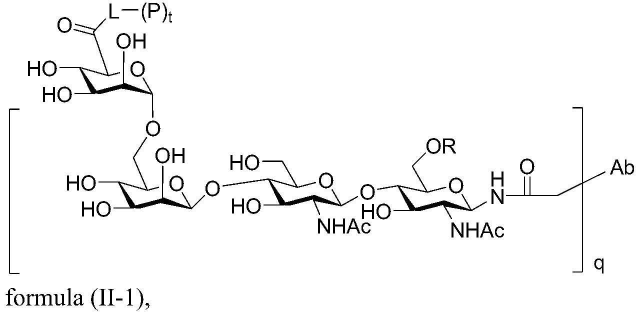 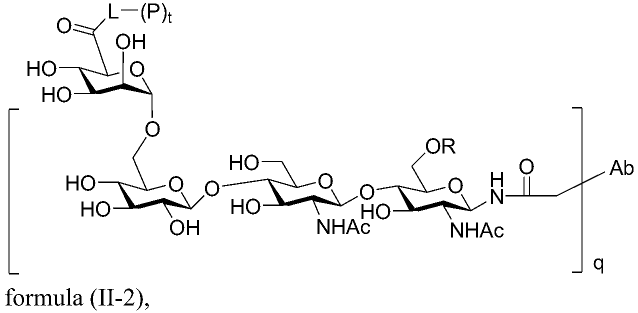 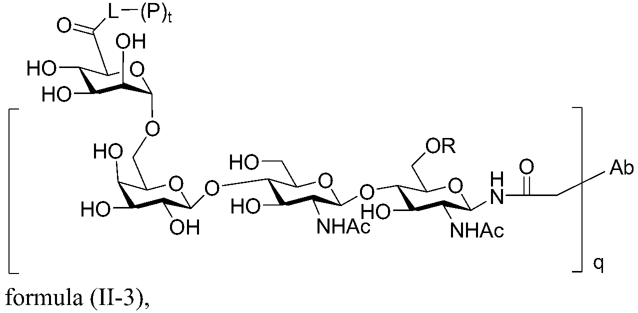 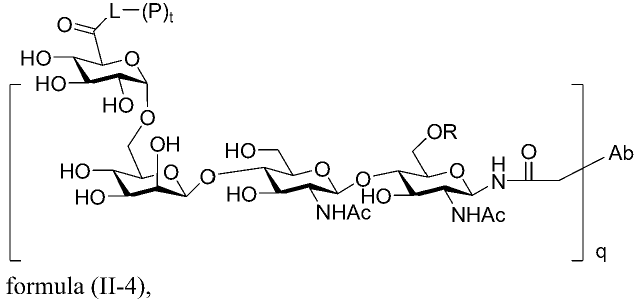 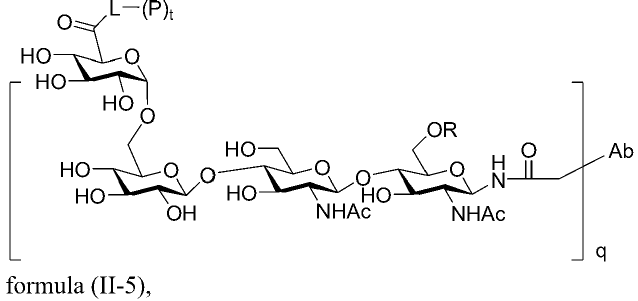 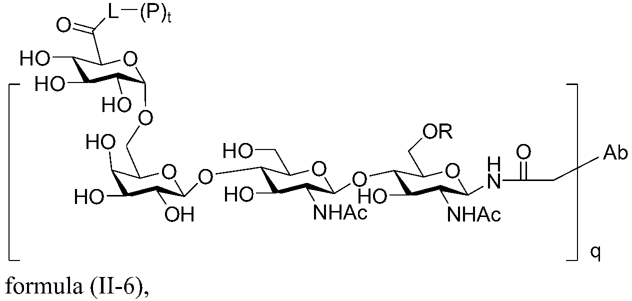 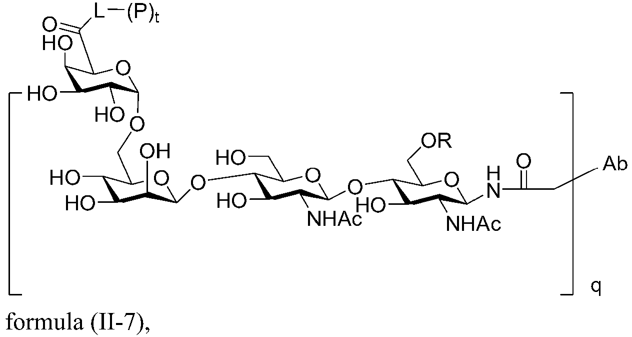 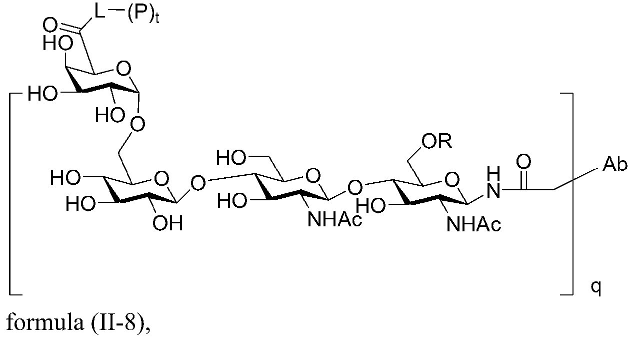 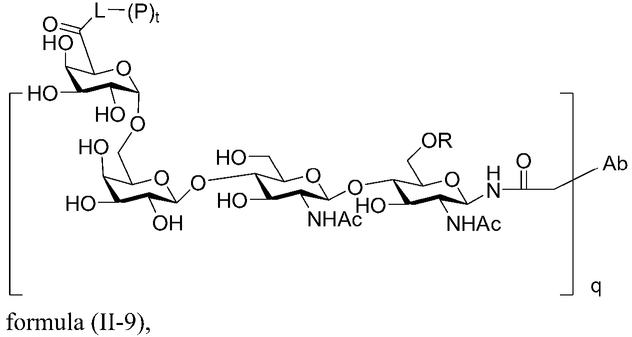 wherein
R is hydrogen or α-L-fucosyl;
q is 1 or 2;
Ab is an antibody or an antigen-binding fragment;
-L-(P)ₜ is as defined in any one of claims 1-19.

21. The antibody-conjugated drug according to claim 20, wherein
Ab is an anti-HER2 antibody.

22. A pharmaceutical composition, comprising the antibody-conjugated drug according to claim 20 or 21.

23. Use of the antibody-conjugated drug according to claims 20 or 21 in preparing a medicament for treating or preventing a tumor or an autoimmune disease.

24. A method for treating or preventing a tumor or an autoimmune disease, comprising administering to a subject in need thereof a therapeutically effective amount of the antibody-conjugated drug according to claim 20 or 21.

25. A trisaccharide linker having the structure of formula (IV) below: 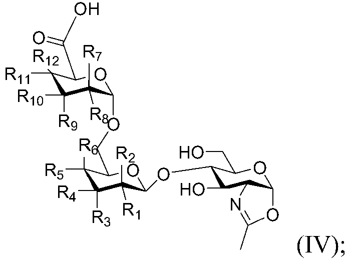
wherein, R₁-R₁₂ in formula (IV) are selected from -OH and H;
preferably, two substituents on the same carbon atom are different;
more preferably, two substituents on the same carbon atom are different, R₃ is H, and R₄ is - OH;
even more preferably, two substituents on the same carbon atom are different, R₃ is H, R₄ is - OH, R₉ is H, and R₁₀ is -OH;
still more preferably, two substituents on the same carbon atom are different, R₃ is H, R₄ is - OH, R₉ is H, R₁₀ is -OH, R₈ is H, R₇ is -OH, R₁₂ is H, and R₁₁ is -OH.

26. The trisaccharide linker according to claim 25, wherein the specific structure of the trisaccharide linker is as follows:

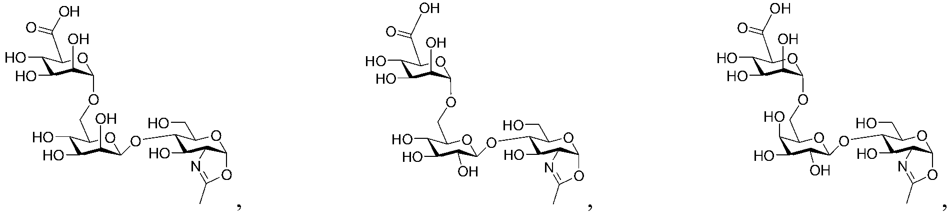

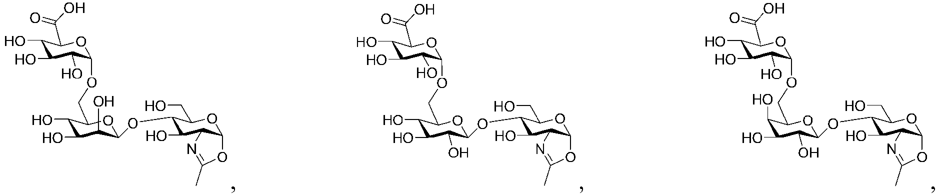

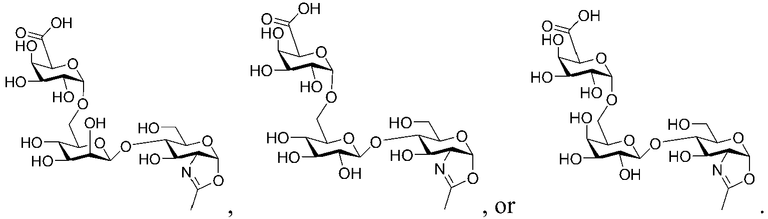

27. Use of the trisaccharide linker according to claim 25 or 26 in preparing a conjugated drug.
